(19) Europäisches Patentamt
European Patent Office
Office européen des brevets

(11) Publication number: **0 083 864**
**B1**

(12) # EUROPEAN PATENT SPECIFICATION

(45) Date of publication of patent specification: **13.05.87**

(51) Int. Cl.⁴: **C 07 K 7/00,** A 61 K 37/02

(21) Application number: **82306862.2**

(22) Date of filing: **22.12.82**

(54) **Synthetic peptides having pituitary growth hormone releasing activity.**

(30) Priority: **28.12.81 US 335000**
**28.12.81 US 335011**
**28.12.81 US 334488**

(43) Date of publication of application:
**20.07.83 Bulletin 83/29**

(45) Publication of the grant of the patent:
**13.05.87 Bulletin 87/20**

(84) Designated Contracting States:
**BE CH DE FR GB IT LI NL SE**

(56) References cited:
**EP-A-0 000 559**
**EP-A-0 018 072**
**EP-A-0 030 846**
**FR-A-2 338 925**

**UNLISTED DRUGS, vol. 33, no. 3, March 1981, page 39K, Edit. Spec. Library Association "DADL"**

(73) Proprietor: **BECKMAN INSTRUMENTS, INC.**
**Executive Office 2500 Harbor Boulevard**
**Box 3100**
**Fullerton California 92634 (US)**

(72) Inventor: **Momany, Frank A.**
**51 East Yates Road**
**N. Memphis Tennessee 38117 (US)**
Inventor: **Bowers, Cyril Y.**
**484 Audubon Street**
**New Orleans Louisiana 70118 (US)**

(74) Representative: **Wotherspoon, Graham et al**
**FITZPATRICKS 4 West Regent Street**
**Glasgow G2 1RS Scotland (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

Background of the Invention

1. Field of the Invention.

This invention relates to peptides which possess pituitary growth hormone releasing activity and to combinations comprising at least one peptide and at least one growth promoting agent, which combination possesses synergistic pituitary growth hormone releasing activity.

2. Description of the Prior Art

Growth hormone, which is secreted from the pituitary, causes growth of all tissues of the body that are capable of growing. In addition, growth hormone is known to have the following basic effects on the metabolic process of the body:

1. Increased rate of protein synthesis in all cells of the body;
2. Decreased rate of carbohydrate utilization in cells of the body;
3. Increased mobilization of free fatty acids and use of fatty acids for energy.

A deficiency in growth hormone secretion can result in various medical disorders, such as some instances of dwarfism.

Various ways are known to release growth hormone. For example, chemicals such as arginine, L-3,4-dihydroxyphenylalanine (L—DOPA), glucagon, vasopressin, and insulin induced hypoglycemia, as well as activities such as sleep and exercise, indirectly cause growth hormone to be released from the pituitary by acting in some fashion on the hypothalamus perhaps either to decrease somatostatin secretion or to increase an unknown endogenous growth hormone-releasing hormone or both.

Compounds which directly act on the pituitary to release growth hormone include prostaglandin $E_1$ and $E_2$, theophylline, and cyclic nucleotides. However, these compounds neither specifically release growth hormone nor are they believed to act at the putative growth hormone-releasing hormone receptors in the peripheral membrane of the pituitary cell to initiate growth hormone release.

In addition, under special conditions certain chemically defined peptides, e.g., vasopressin, thyrotropin-releasing hormone (TRH), luteinizing hormone-releasing hormone (LH—RH), α-melanocyte-stimulating hormone (α-MSH), glucagon, substance P, neurotensin; Met-enkephalin, β-endorphin, chlorea-enterotoxin, and basic myelin protein, act to release growth hormone from the pituitary. However, only TRH acts directly on the pituitary to elicit this response. Furthermore, the above listed peptides release other pituitary hormones and under most experimental conditions do not release growth hormone. For example, TRH does not release growth hormone in normal rats or in normal humans or from pituitaries of normal rats or monkeys. *In vitro,* TRH releases growth hormone, prolactin, and thyroid stimulating hormone (TSH) in certain species, and, *in vivo,* TRH releases these hormones from bovine pituitary.

Vasopressin's induced release of growth hormone is considered to be due to a non-specific response to stress caused by administration of high dosages of vasopressin.

Accordingly it would be highly desirable to have a compound or combination of compounds which directly acts on the pituitary under normal experimental conditions to effect the release of growth hormone therefrom. Such compound or combination of compounds would be useful *in vitro,* e.g., as unique research tools for understanding how growth hormone secretin is regulated at the pituitary level and would also be useful *in vivo,* e.g., to treat symptoms releated to growth hormone deficiencies, to increase the rate and extent of growth in commercial animals, to increase milk yield in commercial animals, and to reduce the number of mucosal erosions induced by hypoxemia.

Synthetic peptides and salts thereof having pituitary growth hormone releasing activity are disclosed in EP—A—0018072 which peptides have the formulae listed below.

$$\begin{array}{c} X \\ | \\ X—Y_1—Z_1—E_1—G_1—J_1—Q \end{array} \qquad (I)$$

$$\begin{array}{c} X \\ | \\ X—Y_2—Z_2—E_2—G_2—J_2—Q \end{array} \qquad (II)$$

$$\begin{array}{c} X \\ | \\ X—Y_3—Z_3—E_3—G_3—J_3—Q \end{array} \qquad (III)$$

$$\begin{array}{c} X \\ | \\ X—Y_4—Z_4—E_4—Q \end{array} \qquad (IV)$$

$$X-\underset{|}{\overset{X}{Y_5}}-Z_5-E_5-J_5-Q \quad (V)$$

$$X-\underset{|}{\overset{X}{Y_6}}-Z_6-E_6-J_6-Q \quad (VI)$$

$$X-\underset{|}{\overset{X}{Y_7}}-Z_7-E_7-G_7-J_7-Q \quad (VII)$$

$$X-\underset{|}{\overset{X}{Y_8}}-Z_8-E_8-G_8-Q \quad (VIII)$$

$$X-\underset{|}{\overset{X}{Y_9}}-Z_9-E_9-G_9-J_9-Q \quad (IX)$$

and

$$X'-\underset{X'}{\underset{|}{Y_{10}}}-Z_{10}-E_{10}-G_{10}-J_{10}-L_{10}-Q \quad (X)$$

wherein each X is selected from —H and —$CH_3$;

$Y_1$, $G_1$, $G_2$, $E_4$, $Z_5$, $J_5$, $Y_6$, $G_6$, $Z_7$, $G_7$, $Y_8$, $G_8$, $Y_9$, $G_9$, $Y_{10}$, and $G_{10}$ are selected from tyrosyl, tryptophyl, phenylalanyl and, with respect to $E_4$, $J_5$, and $G_8$, the descarboxy forms thereof;

$Z_1$, $J_1$, $Z_2$, $Z_3$, $E_3$, $Y_4$, $Z_4$, $E_5$, $G_5$, $E_6$, $J_6$, $Y_7$, $E_7$, $Z_8$, $E_8$, $Z_9$, $E_9$, $Z_{10}$, and $J_{10}$ are selected from D-tyrosyl, D-tryptophyl, D-phenylalanyl, and, with respect to $J_1$ and $J_6$, the descarboxy forms thereof;

$J_3$ and $Z_6$ are selected from glycyl, alanyl, valyl, leucyl, isoleucyl, prolyl, hydroxyprolyl, seryl, threonyl, cysteinyl, methionyl, and, with respect to $J_3$, the descarboxy forms thereof;

$E_1$ is selected from glycyl, alanyl, valyl, leucyl, isoleucyl, prolyl, hydroxyprolyl, seryl, threonyl, cysteinyl, methionyl, aspartyl, glutamyl, asparagyl, glutaminyl, and histidyl;

$Y_2$ is selected from tryptophyl and phenylalanyl;

$E_2$ is selected from glycyl, alanyl, valyl, leucyl, methionyl, and isoleucyl;

$J_2$ is selected from glycyl, alanyl, D-alanyl, valyl, D-valyl, leucyl, D-leucyl, isoleucyl, D-isoleucyl, prolyl, D-prolyl, hydroxyprolyl, D-hydroxyprolyl, seryl, D-seryl, threonyl, D-threonyl, cysteinyl, D-cysteinyl, methionyl, D-methionyl, and the descarboxy forms thereof;

$Y_3$ is selected from tyrosyl, D-tyrosyl, tryptophyl, D-tryptophyl, penylalanyl, and D-phenylalanyl;

$G_3$ is selected from lysyl and arginyl;

$Y_5$ is selected from D-lysyl and D-arginyl;

$J_7$ is selected from glycycl, alanyl, valyl, leucyl, isoleucyl, prolyl, hydroxyprolyl, seryl, threonyl, cysteinyl, methionyl, aspartyl, glutamyl, asparagyl, glutaminyl, arginyl, lysyl, and the descarboxy forms thereof;

$J_9$ is selected from (a) natural amino acids, (b) the D-configuration thereof, and the descarboxy forms (a) and (b);

each X' is selected from —H, —$CH_3$ and —$CHOCH_3$;

$E_{10}$ is selected from glycyl, alanyl, valyl, leucyl, isoleucyl, seryl, threonyl, methionyl, asparagyl, and glutamyl;

$L_{10}$ is selected from asparaglyl, glutamyl, glutaminyl, arginyl, lysyl, seryl, threonyl, and the descarboxy forms thereof; and

Q is a C-terminal function group selected from $NH_2$, —NHR, —$NR_1R_2$, —$CH_2OR$, —$CH_2OH$, —OH and —OR;

wherein each R, $R_1$ and $R_2$ is selected from straight and branched alkyl groups containing 1—6 carbon atoms.

## Summary of the Invention

In accordance with the present invention there is provided peptides which act directly on the pituitary under normal experimental conditions *in vitro* to release growth hormone therefrom. In addition, there is provided a combination of compounds which act directly on the pituitary under normal experimental conditions *in vitro* to synergistically release growth hormone therefrom.

These growth hormone releasing compounds and combinations can be utilized *in vitro* as unique research tools for understanding, inter alia, how growth hormone secretion is regulated at the pituitary level.

Also, the growth hormone releasing peptides of the instant invention can also be administered *in vivo* to increase growth hormone release.

More particularly, this invention encompasses novel peptides having the formulas I and II

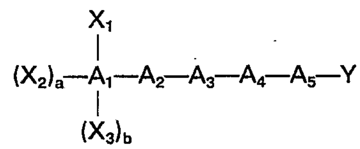

(I)

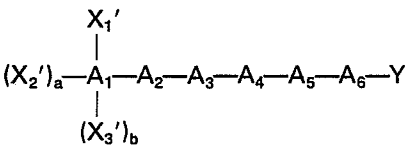

(II)

wherein $X_1$, $X_2$, $X_3$ and $X_1'$, $X_2'$, and $X_3'$ are selected from a group consisting of N-terminal and desamino alpha-carbon substitutions; a and b are 0 or 1, provided that a and b are always 0 when $A_1$ is a desamino residue; $A_1$ and $A_4$ are selected from a group consisting of histidyl, arginyl, lysyl, α-naphthylalanyl, β-naphthylalanyl, isoquinolyl, tyrosyl, tryptophyl, phenylalanyl, homologues and analogues thereof, and, with respect to $A_1$ only, the desamino forms thereof; $A_2$ and $A_5$ are selected from a group consisting of D-histidyl, D-arginyl, D-lysyl, D-α-naphthylalanyl, D-β-naphthylalanyl, D-isoquinolyl, D-tyrosyl, D-tryptophyl, D-phenylalanyl, homologues and analogues thereof, and, with respect to $A_5$ only, the descarboxy forms thereof; $A_3$ is selected from a group consisting of glycyl, alanyl, valyl, leucyl, isoleucyl, prolyl, seryl, threonyl, methionyl, aspartyl, glutamyl, asparaginyl, glutaminyl, histidyl, D-alanyl, D-valyl, D-leucyl, D-isoleucyl, D-prolyl, D-seryl, D-threonyl, D-methionyl, D-aspartyl, D-glutamyl, D-asparaginyl, D-glutaminyl, D-histidyl, and homologues and analogues thereof; $A_6$ is selected from a group consisting of amino acid residues of the L- and D-configuration, homologues and analogues thereof, and the descarboxy forms thereof; and Y is selected from a group consisting of C-terminal and descarboxy alpha-carbon substitutions; and the pharmaceutically acceptable salts thereof; provided that (a) when (1) a is 1 and b is 0 and $X_1$ and $X_2$ are —H or —$CH_3$; (2) $A_1$ and $A_4$ are selected from the group consisting of tyrosyl, tryptophyl, and phenylalanyl (3) $A_3$ is selected from the group consisting of glycyl, alanyl, valyl, leucyl, isoleucyl, prolyl, seryl, threonyl, methionyl, aspartyl, glutamyl, least one of $A_2$ and $A_5$ is selected such that it is not from a group consisting of D-tyrosyl, D-tryptophyl, and D-phenylalanyl; and (d) when (1) a is 1 and b is 0 and $X_1'$ nd $X_2'$ are selected from the group consisting of —H, —$CH_3$, and —$CHOCH_3$; (2) $A_2$ and $A_5$ are selected from the group consisting of D-tyrosyl, D-tryptophyl, and D-phenylalanyl; (3) $A_3$ is selected from the group consisting of glycyl, alanyl, valyl, leucyl, isoleucyl, seryl, threonyl, methionyl, asparaginyl, and glutaminyl; (4) $A_6$ is selected from the group consisting of asparaginyl, glutaminyl, glutamyl, arginyl, lysyl, seryl, threonyl, and the descarboxy forms thereof; and (5) Y is selected from the group consisting of —$NR_1R_2$, —OR, and —$CH_2OR$, wherein R, $R_1$, and $R_2$ are selected from a group consisting of hydrogen and straight and branched chain alkyl groups containing 1—6 carbon atoms; then at least one of $A_1$ and $A_4$ is selected such that it is not from a group consisting of tyrosyl, tryptophyl, and phenylalanyl.

In addition, this invention encompasses a combination of compounds comprising (a) at least one peptide having the formula III

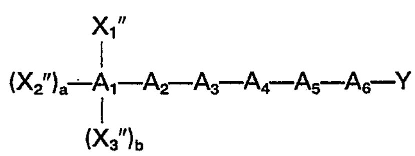

(III)

wherein $X_1''$, $X_2''$, and $X_3''$ are selected from a group consisting of N-terminal and desamino alpha-carbon substitutions; a and b are 0 or 1, provided that a and b are always 0 when $A_1$ is a desamino residue; $A_1$ and $A_4$ are selected from a group consisting of histidyl, arginyl, lysyl, α-naphthylalanyl, β-naphthylalanyl, isoquinolyl, tryosyl, tryptophyl, phenylalanyl, homologues and analogues thereof, and, with respect to $A_1$ only, the desamino forms thereof; $A_2$ and $A_5$ are selected from a group consisting of D-histidyl, D-arginyl, D-lysyl, D-α-naphthylalanyl, D-β-naphthylalanyl, D-isoquinolyl, D-tyrosyl, D-tryptophyl, D-phenylalanyl, homologues and analogues thereof, and, with respect to $A_5$ only, the descarboxy forms thereof; $A_3$ is selected from a group consisting of glycyl, alanyl, valyl, leucyl, isoleucyl, prolyl, seryl, threonyl, methionyl, aspartyl, glutamyl, asparaginyl, glutaminyl, histidyl, D-alanyl, D-valyl, D-leucyl, D-isoleucyl, D-prolyl, D-seryl, D-threonyl, D-methionyl, D-aspartyl, D-glutamyl, D-asparaginyl, D-glutaminyl, D-histidyl, and homologues and analogues thereof; $A_6$ is selected from a group consisting of amino acid residues of the L- and D-configuration, homologues and analogues thereof, and the descarboxy forms thereof; and Y is selected from a group consisting of C-terminal and descarboxy alpha-carbon substitutions; and the pharmaceutically acceptable salts thereof; and (b) at least one growth promoting agent.

## Detailed Description of the Preferred Embodiments

The peptides of this invention have the amino acid residue sequence represented by formulas I—III, supra.

All amino acid residues identified herein are in the natural or L-configuration unless otherwise specified.

Abbreviations for amino acid residues are used in accordance with the following standard peptide nomenclature:

| | | | |
|---|---|---|---|
| Tyr | L-tyrosyl | Ile | L-isoleucyl |
| D-Tyr | D-tyrosyl | D-Ile | D-isoleucyl |
| Gly | glycyl | Leu | L-leucyl |
| Phe | L-phenylalanyl | D-Leu | D-leucyl |
| D-Phe | D-phenylalanyl | Thr | L-threonyl |
| Met | L-methionyl | D-Thr | D-threonyl |
| D-Met | D-methionyl | Val | L-valyl |
| Ala | L-alanyl | D-Val | D-valyl |
| D-Ala | D-alanyl | Pro | L-prolyl |
| Ser | L-seryl | D-Pro | D-prolyl |
| D-Ser | D-seryl | Gln | L-glutaminyl |
| Lys | L-lysyl | D-Gln | D-glutaminyl |
| D-Lys | D-lysyl | Glu | L-glutamyl |
| Asn | L-asparaginyl | D-Glu | D-glutamyl |
| D-Asn | D-asparaginyl | Trp | L-tryptophyl |
| His | L-histidyl | D-Trp | D-tryptophyl |
| | | L-Asp | L-aspartyl |
| D-His | D-histidyl | D-Asp | D-aspartyl |
| Cys | L-cysteinyl | Arg | L-arginyl |
| D-Cys | D-cysteinyl | D-Arg | D-arginyl |
| Hypro | L-hydroxypropyl | | |
| D-Hypro | D-hydroxypropyl | | |
| Dopa | L-3,4-dihydroxyphenylalanyl | L-<Glu | L-pyroglutamyl |
| D-Dopa | D-3,4-dihydroxyphenylalanyl | D-<Glu | D-pyroglutamyl |
| Hylys | L-δ-hydroxylysyl | Sar | N-methylglycyl (sarcosyl) |
| D-Hylys | D-δ-methylalanyl | | |
| Aib | L-α-methylalanyl (L-aminoisobutyryl) | α-Naphth | L-α-naphthylalanyl |
| | | D-α-Naphth | D-α-naphthylalanyl |
| | | β-Naphth | L-β-naphthylalanyl |
| Iql | L-isoquinolyl | D-β-Naphth | D-β-naphthylalanyl |
| D-Iql | D-isoquinolyl | | |

Virtually any suitable N-terminal and desamino alpha-carbon substitution can be used in the instant invention as represented by the various structual formulas set forth herein. Typical N-terminal and desamino alpha-carbon substitutions include, but are not limited to, those set forth in Table I.

TABLE I

N-Terminus and Desamino Alpha-Carbon Substitutions[1]

| $X_1$ | $X_2$ | $X_3$ | a | b |
|---|---|---|---|---|
| | N-Terminus Substitutions | | | |
| $R_1$— | $R_2$— | — | 1 | 0 |
| $R_1$— | $R_2$— | $R_3$— | 1 | 1 |
| 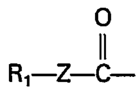 | $R_2$— | — | 1 | 0 |
| 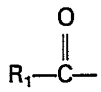 | $R_2$— | — | 1 | 0 |
| $R_1$— | HO— | — | 1 | 0 |
| 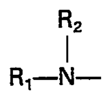 | $R_3$— | — | 1 | 0 |
| 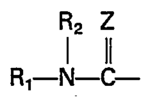 | $R_3$— | — | 1 | 0 |
| | Desamino Alpha-Carbon Substitutions | | | |
| R— | — | — | 0 | 0 |
| RZ— | — | — | 0 | 0 |

1. LEGEND: R, $R_1$, $R_2$, and $R_3$ are selected from a group consisting of hydrogen; straight and branched chain alkyl groups having from 1 to 6 carbon atoms; cycloalkyl groups having from 3 to 6 carbon atoms; benzyl; benzhydryl; trityl; aryl; alkoxybenzyl; alkoxybenzhydryl; alkoxytrityl; lower haloalkyl groups having from 1 to 6 carbon atoms; halobenzyl; halobenzhydryl; halotrityl; haloaryl; and cyclohaloalkyl groups having from 3 to 6 carbon atoms. Preferably, R, $R_1$, $R_2$, and $R_3$ are selected from the group consisting of hydrogen and alkyl groups having from 1 to 6 carbon atoms. More preferably, R, $R_1$, $R_2$, and $R_3$ are selected from the group consisting of hydrogen and alkyl groups having 1 to 2 carbon atoms.

Z is selected from a group consisting of oxygen and sulfur. Z is preferably oxygen.

Virtually any suitable C-terminal and descarboxy alpha-carbon substitution can be used in the instant invention as represented by the various structural formulas set forth herein. Typical C-terminal and descarboxy alpha-carbon substitutions include, but are not limited to, those also set forth in Table II.

TABLE II

C-Terminus and Descarboxy Alpha-Carbon Substitutions[1]

C-Terminus Substitutions

—OR

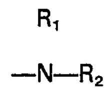

—R

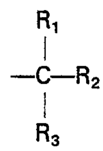

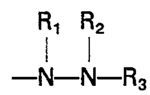

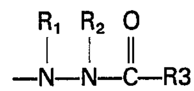

Descarboxy Alpha-Carbon Substitutions

—R

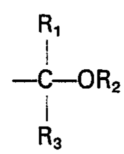

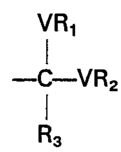

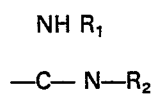

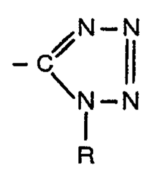

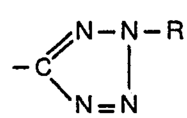

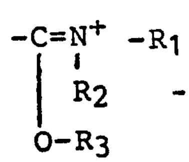

1. LEGEND: R, $R_1$, $R_2$, and $R_3$ are as defined in Table I, supra.
V is selected from a group consisting of oxygen, sulfur, and nitrogen. V is preferably oxygen.

The structure of amino acid residues employed in the peptides of this invention are set forth in Table III. Typical homologues and analogues of these amino acid residues which can also be employed in the peptides of this invention include, but are not limited to, those listed in Table III.

TABLE III

L or D Amino Acid Residue

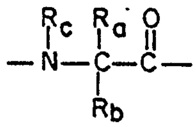

| NAME | NATURAL SUBSTITUENTS $R_a$ | $R_b$ | $R_c$ | SUBSTITUENTS OF HOMOLOGUES & ANALOGUES[1] $R_a$ | $R_b$ | $R_c$ |
|---|---|---|---|---|---|---|
| Gly | $-H$ | $-H$ | $-H$ | $-H$ | U | $U_1$ |
| Ala | $-CH_3$ | $-H$ | $-H$ | $-CH_3$ | U | $U_1$ |
| | | | | $=CH_2$ | - | U |
| Val | $-CH(CH_3)_2$ | $-H$ | $-H$ | $-CH(CH_3)_2$ | U | $U_1$ |
| | | | | $-C(CH_3)_3$ | U | $U_1$ |
| | | | | $=C(CH_3)_2$ | - | U |
| Leu | $-CH_2CH(CH_3)_2$ | $-H$ | $-H$ | $-(CH_2)_{n+1}CH(CH_3)_2$ | U | $U_1$ |
| | | | | $-(CH_2)_{n+1}C(CH_3)_3$ | U | $U_1$ |
| | | | | $=CHCH(CH_3)_2$ | - | U |
| | | | | $=CH(CH_2)_nCH(CH_3)_2$ | - | U |
| Ile | $-CH(CH_3)-C_2H_5$ | $-H$ | $-H$ | $-CH(CH_3)-C_2H_5$ | U | $U_1$ |
| NIle | $-CH_2CH_2CH_2CH_3$ | $-H$ | $-H$ | $-(CH_2)_{n+1}CH_3$ | U | $U_1$ |
| | | | | $=CH(CH_2)_nCH_3$ | - | U |
| Pro | $CH_2$—$CH_2$— | $-H$ | —$CH_2$ | $CH_2$—$(CH_2)_n$—$CH_2$ | U | |
| | | | | $CH_2$—$CH$=$CH$ | U | |
| | | | | $CH_2$—$CHOU$—$CH_2$ | $U_1$ | |
| | | | | $(CH_2)_n$—B—$(CH_2)$ | U | |
| Ser | $-CH_2OH$ | $-H$ | $-H$ | $-(CH_2)_{n+1}OU$ | $U_1$ | $U_2$ |
| Thr | $-CHOH-CH_3$ | $-H$ | $-H$ | $-(CH_2)_{n+1}CHOU-CH_3$ | $U_1$ | $U_2$ |
| Cys | $-CH_2SH$ | $-H$ | $-H$ | $-(CH_2)_{n+1}SU$ | $U_1$ | $U_2$ |
| | | | | $-(CH_2)_{n+1}SO_3H$ | U | $U_1$ |

TABLE III (contd.)

| NAME | NATURAL SUBSTITUENTS | | | SUBSTITUENTS OF HOMOLOGUES & ANALOGUES[1] | | |
|---|---|---|---|---|---|---|
| | $R_a$ | $R_b$ | $R_c$ | $R_a$ | $R_b$ | $R_c$ |
| Met | $-CH_2CH_2SCH_3$ | -H | -H | $-(CH_2)_{n+1}SCH_3$ | U | $U_1$ |
| | | | | $=CH(CH_2)_{n+1}SCH_3$ | - | $U_1$ |
| | | | | $-(CH_2)_{n+1}SOCH_3$ | U | $U_1$ |
| | | | | $=CH(CH_2)_{n+1}SOCH_3$ | - | $U_1$ |
| | | | | $-(CH_2)_{n+1}SO_2CH_3$ | U | $U_1$ |
| | | | | $=CH(CH_2)_{n+1}SO_2CH_3$ | - | U |
| Asp | $-CH_2CO_2H$ | -H | -H | $-(CH_2)_{n+1}CO_2U$ | $U_1$ | $U_2$ |
| | | | | $=CH(CH_2)_nCO_2U$ | - | $U_1$ |
| Glu | $-(CH_2)_2CO_2H$ | -H | -H | $-(CH_2)_{n+1}CO_2U$ | $U_1$ | $U_2$ |
| Asn | $-CH_2CONH_2$ | -H | -H | $-(CH_2)_{n+1}CONR_1R_2$ | U | $U_1$ |
| | | | | $=CH(CH_2)_nCONR_1R_2$ | - | U |
| Gln | $-(CH_2)_2CONH_2$ | -H | -H | $-(CH_2)_{n+1}CONR_1R_2$ | U | $U_1$ |
| Arg | 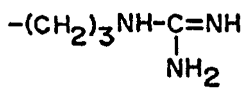 | -H | -H | 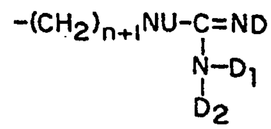 | $U_1$ | $U_2$ |
| | | | | 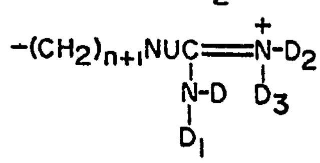 | $U_1$ | $U_2$ |
| Lys | $-(CH_2)_4NH_2$ | -H | -H | 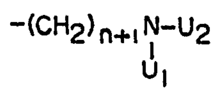 | U | $U_3$ |
| | | | | 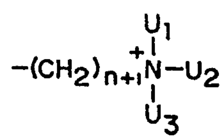 | U | $U_4$ |
| His | 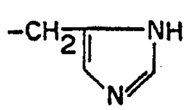 | -H | -H | 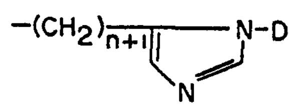 | U | $U_1$ |
| | | | | 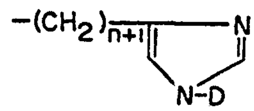 | U | $U_1$ |
| | | | | 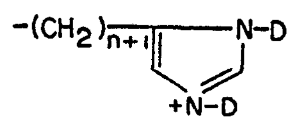 | U | $U_1$ |
| | | | | 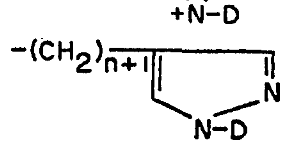 | U | $U_1$ |

TABLE III (contd.)

| NAME | NATURAL SUBSTITUENTS $R_a$ | $R_b$ | $R_c$ | SUBSTITUENTS OF HOMOLOGUES & ANALOGUES[1] $R_a$ | $R_b$ | $R_c$ |
|---|---|---|---|---|---|---|
| | | | | 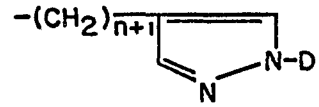 | U | $U_1$ |
| | | | | 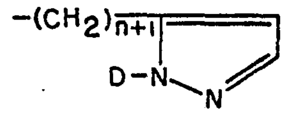 | U | $U_1$ |
| | | | | 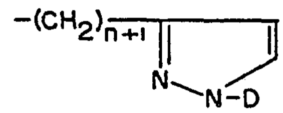 | U | $U_1$ |
| | | | | 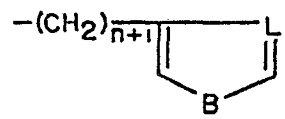 | U | $U_1$ |
| | | | | 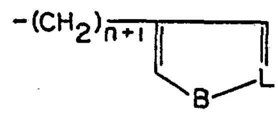 | U | $U_1$ |
| | | | | 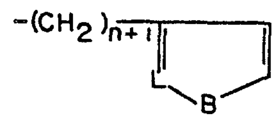 | U | $U_1$ |
| | | | | 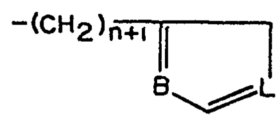 | U | $U_1$ |
| | | | | 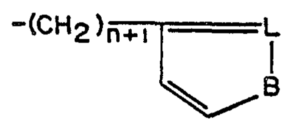 | U | $U_1$ |
| | | | | 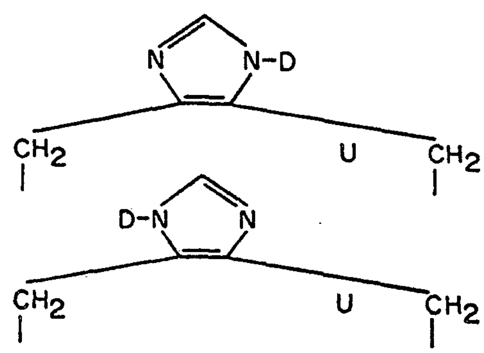 | U | |
| | | | | | U | |

TABLE III (contd.)

| NAME | NATURAL SUBSTITUENTS | | | SUBSTITUENTS OF HOMOLOGUES & ANALOGUES[1] | | |
|---|---|---|---|---|---|---|
| | $R_a$ | $R_b$ | $R_c$ | $R_a$ | $R_b$ | $R_c$ |
| Phe | 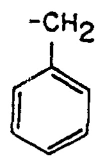 | -H | -H | 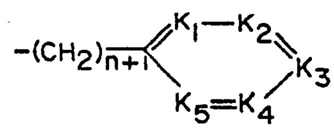 | U | $U_1$ |
| | | | | 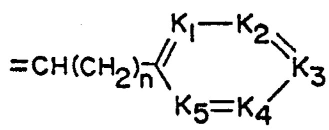 | - | $U_1$ |
| | | | | 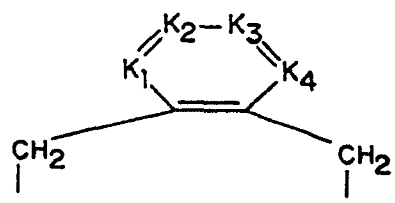 | | $U_1$ |
| Tyr | 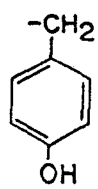 | -H | -H | See Phe above | | |
| α-Naphth | 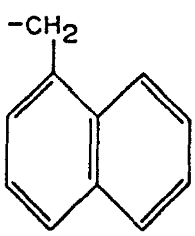 | -H | -H | 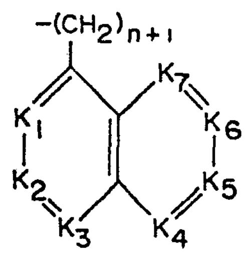 | U | $U_1$ |
| | | | | 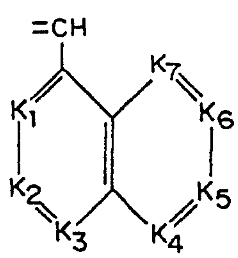 | - | U |

TABLE III (contd.)

| NAME | NATURAL SUBSTITUENTS | | | SUBSTITUENTS OF HOMOLOGUES & ANALOGUES[1] | | |
|---|---|---|---|---|---|---|
| | $R_a$ | $R_b$ | $R_c$ | $R_a$ | $R_b$ | $R_c$ |
| β-Naphth | $-CH_2$ 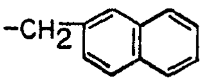 | –H | –H | $-(CH_2)$ 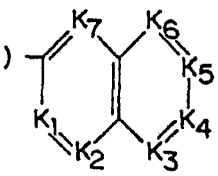 | U | $U_1$ |
| | | | | =CH 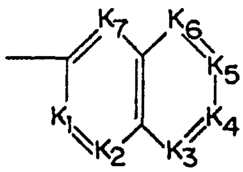 | - | U |
| Trp | $-CH_2$ 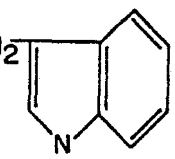 | –H | –H | $-(CH_2)_{n+1}$ 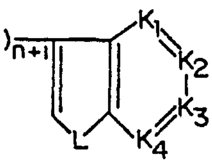 | U | $U_1$ |
| Sar | –H | –H | $-CH_3$ | U | $U_1$ | $-CH_3$ |
| Iql | $-CH_2-$ 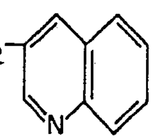 | –H | –H | $-(CH_2)_{n+1}$ 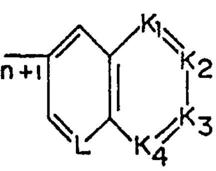 | U | $U_1$ |

1. LEGEND: U, $U_1$, $U_2$, $U_3$ and $U_4$ are selected from a group consisting of hydrogen, alkyl groups having from 1—10 carbon atoms, and benzyl.

B, $B_1$, and $B_2$ are selected from a group consisting of —N—D, O, S.

D, $D_1$, $D_2$, and $D_3$ are selected from a group consisting of hydrogen, methyl, ethyl, propyl, benzyl, formyl, and tosyl.

$K_1$, $K_2$, $K_3$, $K_4$, $K_5$, $K_6$, and $K_7$ are N or —C—G, provided that adjacent positions are not both N.

G is selected from a group consisting of hydrogen, halogen, —OU, $—OR_X$, $—SR_x$, $—R_x$, $—SO_3R_x$, $—(OH)_2$, $—RN_XSOR_Y$, $—NR_XR_Y$, —C≡N, $—N(R_X)COR_Y$, wherein $R_X$ and $R_Y$ are selected from a group consisting of hydrogen and straight and branched alkyl groups containing 1—6 carbon atoms, and substituted straight and branched alkyl groups containing 1—6 carbon atoms, wherein the substituents include, but are not limited to, one or more halo, hydroxy, amino, and mercapto groups.

L is —N or $—N^+—D$.

$R_1$ and $R_2$ are as defined in Table I.

n is an integer from 0 to 4.

The term "pharmaceutically acceptable salts", as used herein, refers to the non-toxic alkali metal, alkaline earth metal and ammonium salts commonly used in the pharmaceutical industry including, but not limited to, the sodium, potassium, lithium, calcium, magnesium, barium, ammonium and protamine salts which are prepared by methods well known in the art. The term also includes non-toxic acid addition salts which are generally prepared by reacting the compounds of this invention with a suitable organic or inorganic acid. Representative salt include, but are not limited to, the hydrochloride, hydrobromide, sulfate, bisulfate, acetate, oxalate, valerate, oleate, laurate, borate, benzoate, lactate, phosphate, tosylate, citrate, maleate, fumarate, succinate, tartrate, napsylate, and the like.

Preferably, the peptides of formula I of this invention have the amino acid sequence wherein a is 0 or 1, b is 0 and $X_1$ and $X_2$ are selected from a group consisting of —R, —OR, and RC(O)—, wherein R is selected from a group consisting of hydrogen and straight and branched chain alkyl groups containing 1—6 carbon

atoms; $A_1$ and $A_4$ are selected from the group consisting of histidyl, tryptophyl, phenylalanyl, tyrosyl, homologues and analogues thereof, and, with respect to $A_1$, the desamino forms thereof; $A_2$ and $A_5$ are selected from the group consisting of D-histidyl, D-tryptophyl, D-phenylalanyl, D-tyrosyl, homologues and analogue thereof, and, with respect to $A_5$, the descarboxy forms thereof; $A_3$ is selected from the group consisting of glycyl, alanyl, seryl, asparaginyl, prolyl, D-alanyl, D-seryl, D-asparaginyl, D-prolyl, and homologues and analogues thereof; Y is selected from a group consisting of —$CH_2OH$, —OR, and —$NR_1R_2$, wherein R, $R_1$, and $R_2$ are selected from a group consisting of hydrogen and straight and branched chain alkyl groups containing 1—6 carbon atoms; and the pharmaceutically acceptable salts thereof.

Preferably, the peptides of formulas II and III of this invention have the amino acid sequence wherein a is 0 or 1, b is 0 and $X_1'$, $X_2'$, $X_1''$, and $X_2''$ are selected from a group consisting of —R, —OR, and RC(O)—, wherein R is selected from a group consisting of hydrogen and straight and branched chain alkyl groups containing 1—6 carbon atoms; $A_1$ and $A_4$ are selected from the group consisting of histidyl, tryptophyl, phenylalanyl, tyrosyl, homologues and analogues thereof, and, with respect to $A_1$, the desamino forms thereof; $A_2$ and $A_5$ are selected from the group consisting of D-histidyl, D-tryptophyl, D-phenylalanyl, D-tyrosyl, homologues and analogues thereof; $A_3$ is selected from the group consisting of glycyl, alanyl, seryl, asparaginyl, prolyl, D-alanyl, D-seryl, D-asparaginyl, D-prolyl, and homologues and analogues thereof; $A_6$ is selected from the group consisting of arginine, lysine, ornithine, histidine, aspartic acid, glutamic acid, asparagine, glutamine, D-arginine, D-lysine, D-orthithine, D-histidine, D-aspartic acid, D-glutamic acid, D-asparagine, D-glutamine, D-arginine, homologues and analogues thereof, and the descarboxy forms thereof; Y is selected from a group consisting of —$CH_2OH$, —OR, and —$NR_1R_2$, wherein R, $R_1$ and $R_2$ are selected from a group consisting of hydrogen and straight and branched chain alkyl groups containing 1—6 carbon atoms; and the pharmaceutically acceptable salts thereof.

More preferably, the peptides of (a) formula I and (b) formulas II and III of this invention have the amino acid sequence represented by formulas IV and V, respectively:

$$(X_2)_a\text{—}\overset{\overset{\displaystyle H}{|}}{A_1}\text{—D-Trp—}A_3\text{—}A_4\text{—}A_5\text{—Y} \qquad \text{(IV)}$$

$$(X_2')\text{—}\overset{\overset{\displaystyle H}{|}}{A_1}\text{—D-Trp—}A_3\text{—}A_4\text{—}A_5\text{—}A_6\text{—Y} \qquad \text{(V)}$$

wherein a is 0 or 1; $X_2$ is selected from the group consisting of R— and RC(O)—; wherein R is selected from the group consisting of hydrogen and alkyl groups containing 1—2 carbon atoms; $A_1$ is selected from the group consisting of tyrosyl, O-methyltyrosyl, histidyl, 3-N-methylhistidyl, p-chlorophenylalanyl, and the desamino forms thereof; $A_3$ is selected from the group consisting of alanyl, seryl, and D-alanyl; $A_4$ is selected from the group consisting of tryptophyl and tyrosyl; $A_5$ is selected from the group consisting of D-phenylalanyl, D-histidyl, D-tyrosyl, and D-p-chlorophenylalanyl; $A_6$ is selected from the group consisting of arginine, homoarginine, lysine, ornithine, aspartic acid, glutamic acid, asparagine, glutamine, and D-lysine; and Y is selected from the group consisting of —OR and —NHR, wherein R is selected from the group consisting of hydrogen and alkyl groups containing 1—2 carbon atoms; and the pharmaceutically acceptable salts thereof.

Peptides within the scope of the instant invention include, but are not limited to, those set forth in Table IV and the desamino and/or descarboxy forms thereof, wherein the respective positions of (a) $X_1$, $X_2$, $X_3$, (b), $X_1'$, $X_2'$, $X_3'$, and (c) $X_1''$, $X_2''$, $X_3''$ are set forth in formulas I—III, respectively.

TABLE IV

($X_1$-, $(X_2\text{-})_a$, $(X_3\text{-})_b$)-His-D-Trp-Ala-Trp-D-Phe-Y
($X_1$-, $(X_2\text{-})_a$, $(X_3\text{-})_b$)-His-D-5-Br-Trp-Ala-Trp-D-Phe-Y
($X_1$-, $(X_2\text{-})_a$, $(X_3\text{-})_b$)-His-D-Trp-Ala-5-Br-Trp-D-Phe-Y
($X_1$-, $(X_2\text{-})_a$, $(X_3\text{-})_b$)-1-N-Me-His-D-Trp-Ala-Trp-D-Phe-Y
($X_1$-, $(X_2\text{-})_a$, $(X_3\text{-})_b$)-3-N-Me-His-D-Trp-Ala-Trp-d-Phe-Y
($X_1$-, $(X_2\text{-})_a$, $(X_3\text{-})_b$)-Arg-D-Trp-Ala-Iql-D-Phe-Y
($X_1$-, $(X_2\text{-})_a$, $(X_3\text{-})_b$)-Lys-D-Trp-Ala-Trp-D-Phe-Y
($X_1$-, $(X_2\text{-})_a$, $(X_3\text{-})_b$)-His-D-Trp-Ser-Trp-D-Phe-Y
($X_1$-, $(X_2\text{-})_a$, $(X_3\text{-})_b$)-Tyr-D-Trp-D-Ala-Trp-D-His-Y
($X_1$-, $(X_2\text{-})_a$, $(X_3\text{-})_b$)-Tyr-D-Trp-Ala-Trp-D-1-N-Me-His-Y
($X_1$-, $(X_2\text{-})_a$, $(X_3\text{-})_b$)-Tyr-D-Trp-Ala-Trp-D-3-N-Me-His-Y
($X_1$-, $(X_2\text{-})_a$, $(X_3\text{-})_b$)-Tyr-D-Trp-Ala-Trp-D-Arg-Y
($X_1$-, $(X_2\text{-})_a$, $(X_3\text{-})_b$)-Tyr-D-Trp-Ala-Trp-D-Lys-Y
($X_1$-, $(X_2\text{-})_a$, $(X_3\text{-})_b$)-Tyr-D-Trp-D-Ser-Trp-D-Lys-Y
($X_1$-, $(X_2\text{-})_a$, $(X_3\text{-})_b$)-His-D-Trp-Ala-Trp-D-His-Y
($X_1$-, $(X_2\text{-})_a$, $(X_3\text{-})_b$)-Arg-D-Phe-Val-Tyr-D-Lys-Y
($X_1$-, $(X_2\text{-})_a$, $(X_3\text{-})_b$)-Tyr-D-Tyr-Met-Phe-D-Arg-Y

($X_1$-, $(X_2\text{-})_a$, $(X_3\text{-})_b$)-Phe-D-Phe-Gln-Phe-D-1-N-Me-His-Y
($X_1$-, $(X_2\text{-})_a$, $(X_3\text{-})_b$)-His-D-Trp-Ile-Tyr-D-Trp-Y
($X_1$-, $(X_2\text{-})_a$, $(X_3\text{-})_b$)-α-Naphth-D-Trp-D-Ala-β-Naphth-D-Phe-Y
($X_1$-, $(X_2\text{-})_a$, $(X_3\text{-})_b$)-β-Naphth-D-Lys-D-His-His-D-Arg-Y
($X_1'$-, $(X_2'\text{-})_a$, $(X_3'\text{-})_b$)-His-D-Trp-Ala-Trp-D-Phe-Lys-Y
($X_1'$-, $(X_2'\text{-})_a$, $(X_3'\text{-})_b$)-His-D-5-Br-Trp-Ala-Trp-D-Phe-Lys-Y
($X_1'$-, $(X_2'\text{-})_a$, $(X_3'\text{-})_b$)-His-D-Trp-Als-5-Br-Trp-D-Phe-Lys-Y
($X_1'$-, $(X_2'\text{-})_a$, $(X_3'\text{-})_b$)-1-N-Me-His-D-Trp-Ala-Trp-D-Phe-Asn-Y
($X_1'$-, $(X_2'\text{-})_a$, $(X_3'\text{-})_b$)-3-N-Me-His-D-Trp-Ala-Trp-D-Phe-Lys-Y
($X_1'$-, $(X_2'\text{-})_a$, $(X_3'\text{-})_b$)-Arg-D-Trp-Ala-Iql-D-Phe-Arg-Y
($X_1'$-, $(X_2'\text{-})_a$, $(X_3'\text{-})_b$)-Lys-D-Trp-Ala-Trp-D-Phe-Glu-Y
($X_1'$-, $(X_2'\text{-})_a$, $(X_3'\text{-})_b$)-His-D-Trp-Ser-Trp-D-Phe-Lys-Y
($X_1'$-, $(X_2'\text{-})_a$, $(X_3'\text{-})_b$)-Tyr-D-Trp-D-Ala-Trp-D-His-Gln-Y
($X_1'$-, $(X_2'\text{-})_a$, $(X_3'\text{-})_b$)-Tyr-D-Trp-Ala-Trp-D-1-N-Me-His-Met-Y
($X_1'$-, $(X_2'\text{-})_a$, $(X_3'\text{-})_b$)-Tyr-D-Trp-Ala-Trp-D-3-N-Me-His-Pro-Y
($X_1'$-, $(X_2'\text{-})_a$, $(X_3'\text{-})_b$)-Tyr-D-Trp-Ala-Trp-D-Arg-His-Y
($X_1'$-, $(X_2'\text{-})_a$, $(X_3'\text{-})_b$)-Tyr-D-Trp-Ala-Trp-D-Lys-Ser-Y
($X_1'$-, $(X_2'\text{-})_a$, $(X_3'\text{-})_b$)-Tyr-D-Trp-D-Ser-Trp-D-Lys-Phe-Y
($X_1'$-, $(X_2'\text{-})_a$, $(X_3'\text{-})_b$)-His-D-Trp-Ala-Trp-D-His-Trp-Y
($X_1'$-, $(X_2'\text{-})_a$, $(X_3'\text{-})_b$)-Arg-D-Phe-Val-Tyr-D-Lys-D-Lys-Y
($X_1'$-, $(X_2'\text{-})_a$, $(X_3'\text{-})_b$)-Tyr-D-Tyr-Met-Phe-D-Arg-D-Orn-Y
($X_1'$-, $(X_2'\text{-})_a$, $(X_3'\text{-})_b$)-Phe-D-Phe-Gln-Phe-D-1-N-Me-His-D-Asp-Y
($X_1'$-, $(X_2'\text{-})_a$, $(X_3'\text{-})_b$)-His-D-Trp-Ile-Tyr-D-Trp-D-Glu-Y
($X_1'$-, $(X_2'\text{-})_a$, $(X_3'\text{-})_b$)-α-Naphth-D-Trp-D-Ala-β-Naphth-D-Phe-Val-Y
($X_1'$-, $(X_2'\text{-})_a$, $(X_3'\text{-})_b$)-β-Naphth-D-Lys-D-His-His-D-Arg-Thr-Y

The peptides of the instant invention can be prepared by solution methods known in the art or by using standard solid-phase techniques. The solid-phase synthesis, for example, can be commenced from the C-terminal end of the peptide using an α-amino protected amino acid. A suitable starting material can be prepared, for instance, by attaching the required α-amino acid to a chloromethyl resin, a hydroxymethyl resin, a benzhydrylamine (BHA) resin, or a p-methylbenzylhydrylamine (p-Me-BHA) resin. One such chloromethyl resin is sold under the tradename BIO-BEADS X—1 by Bio Rad Laboratories, Richmond, California. The preparation of the hydroxymethyl resin is described by Bodansky et al., *Chem. Ind.* (*London*) 38, 1597 (1966). The BHA resin has been described by Pietta and Marshall, *Chem. Commn.* 650 (1970) and is commercially available from Beckman Instruments, Inc., Palo Alto, California in the hydrochloride form thereof (BHA·HCl).

In the solid-phase preparation of the compounds of this invention, a protected amino acid can be coupled to a resin with the aid of a coupling agent. After the initial coupling, the α-amino protecting group can be removed by a choice of reagents including trifluoroacetic acid (TFA) or hydrochloric acid (HCl) solution in organic solvents at room temperature. After removal of the α-amino protecting group, the remaining protected amino acids can be coupled stepwise in the desired order. Each protected amino acid can be generally reacted in about a 3-fold excess using an appropriate carboxyl group activator such as dicyclohexylcarbodiimide (DCC) in solution, for example, in methylene chloride ($CH_2Cl_2$)-dimethylformamide (DMF) mixtures.

After the desired amino acid sequence has been completed, the desired peptide can be cleaved from the resin support by treatment with a reagent such as hydrogen fluoride (HF) which not only cleaves the peptide from the resin, but also cleaves all remaining side-chain protecting groups. When a chloromethyl resin or hydroxymethyl resin is used, HF treatment results in the formation of the free peptide acids of formulas I—III (Y = —COOH). When the BHA or p-Me-BHA resin is used, HF treatment results directly in the free peptide amides of formulas I—III (Y = —$CONH_2$). Alternatively, when the chloromethylated or hydroxymethyl resin is employed, the side-chain protected peptide can be cleaved from the resin by treatment of the peptide-resin with ammonia to give the desired side-chain protected amide or with an alkylamine to give a side-chain protected alkylamide or dialkylamide. Side-chain protection can then be removed in the usual fashion by treatment with HF to give the free peptide amides, alkylamides, or dialkylamides.

In preparing the esters of this invention, the resins used to prepare the acids of formulas I—III (Y = —COOH) can be employed and the side-chain protected peptide can be cleaved with a base and an appropriate alcohol, i.e., methanol. Side-chain protecting groups can then be removed in the usual fashion by treatment with HF to obtain the desired ester.

The solid-phase procedure discussed above is well known in the art and has been essentially described by Stewart and Young, *Solid Phase Peptide Synthesis,* Freeman and Co., San Francisco (1969).

Some of the well known solution methods which can be employed to synthesize the peptides of the instant invention are set forth in Bodansky et al., *Peptide Synthesis,* 2nd Edition, John Wiley & Sons, New York, N.Y. 1976).

Accordingly, also within the scope of the instant invention are intermediate compositions prepared during the synthesis of the novel peptides of formulas I—II. Intermediate compositions prepared via solid-phase techniques are the peptide-resin compounds of formulas VI—VII and intermediate compositions prepared via solution techniques are the protected peptide-compounds of formulas VIII—XIII:

$$\begin{array}{rcl} & (Pr_1)_q & \\ & | & \\ & (X_1^{iv})_m & \\ & | & \\ (Pr_1)_r—(X_1^{iv})_a— & B_1 & —B_2—B_3—B_4—B_5—\textcircled{r} \\ & | & \\ & (X_3^{iv})_b & \\ & | & \\ & (Pr_1)_s & \end{array} \qquad (VI)$$

$$\begin{array}{rcl} & (Pr_1)_q & \\ & | & \\ & (X_1^{v})_m & \\ & | & \\ (Pr_1)_r—(X_2^{v})v_a— & B_1 & —B_2—B_3—B_4—B_5—\textcircled{r} \\ & | & \\ & (X_3^{v})_b & \\ & | & \\ & (Pr_1)_s & \end{array} \qquad (VII)$$

$$\begin{array}{rcl} & (Pr_1)_q & \\ & | & \\ & (X_1^{iv})_m & \\ & | & \\ (Pr_1)_r—(X_2^{iv})_a— & B_1 & —B_2—B_3—B_4—B_5'—Y \\ & | & \\ & (X_3^{iv})_b & \\ & | & \\ & (Pr_1)_s & \end{array} \qquad (VIII)$$

$$\begin{array}{rcl} & (Pr_1)_q & \\ & | & \\ & (X_1^{iv})_m & \\ & | & \\ (Pr_1)_r—(X_2^{iv})_a— & B_1 & —B_2—B_3—B_4—B_5—O—Pr_2 \\ & | & \\ & (X_3^{iv})_m & \\ & | & \\ & (Pr_1)_s & \end{array} \qquad (IX)$$

$$B_1—B_2—B_3—B_4—B_5'—Y \qquad (X)$$

$$\begin{array}{rcl} & (Pr_1)_q & \\ & | & \\ & (X_1^{v})_m & \\ & | & \\ (Pr_1)_r—(X_2^{v})_a— & B_1 & —B_2—B_3—B_4—B_5—B'_6—Y \\ & | & \\ & (X_3^{v})_m & \\ & | & \\ & (Pr_1)_s & \end{array} \qquad (XI)$$

$$\begin{array}{rcl} & (Pr_1)_q & \\ & | & \\ & (X_2^{v})_a & \\ & | & \\ (Pr_1)_r—(X_2^{v})_a— & B_1 & —B_2—B_3—B_4—B_5—B_6—O—Pr_2 \\ & | & \\ & (X_3^{v})_b & \\ & | & \\ & (Pr_1)_s & \end{array} \qquad (XII)$$

$$B_1—B_2—B_3—B_4—B_5—B'_6—Y \quad (XIII)$$

wherein $Pr_1$ is an α-amino protecting group; q, r, and s are each either 0 or 1; a and b are as defined above; m is either 0 or 1; $X_1^{iv}$, $X_2^{iv}$, $X_3^{iv}$, and $X_1^{v}$, $X_2^{v}$, and $X_3^{v}$ are selected from a group consisting of N-terminal and desamino alpha-carbon substitutions and radicals; $B_1$ and $B_4$ are selected from a group consisting of histidyl, arginyl, lysyl, α-naphthylalanyl, β-naphthylalanyl, isoquinolyl, tyrosyl, tryptophyl, phenylalanyl, homologues and analogues thereof, the side-chain protected forms thereof, and, with respect to $B_1$, the desamino forms thereof; $B_2$, $B_5$, and $B'_5$ are selected from a group consisting of D-histidyl, D-arginyl, D-lysyl, D-α-naphthylalanyl, D-β-naphthylalanyl, D-isoquinolyl, D-tyrosyl, D-tryptophyl, D-phenylalanyl, homologues and analogues thereof, the side-chain protected forms thereof, and, with respect to $B'_5$, the descarboxy forms thereof; $B_3$ is selected from a group consisting of glycyl, alanyl, valyl, leucyl, isoleucy, prolyl, seryl, threonyl, methionyl, aspartyl, glutamyl, asparaginyl, glutaminyl, histidyl, D-alanyl, D-valyl, D-leucyl, D-isoleucyl, D-prolyl, D-seryl, D-threonyl, D-methionyl, D-aspartyl, D-glutamyl, D-asparaginyl, D-glutaminyl, D-histidyl, homologues and analogues thereof, and the side-chain protected forms thereof; $B_6$ and $B'_6$ are selected from a group consisting of amino acid residues of the L- and D-configuration, homologues and analogues thereof, the side-chain protection forms thereof; Ⓡ is a resin; Y is as defined above; and $Pr_2$ is a carboxyl protecting group; provided that (a) when (1) a is 1 and b and m are 0 and $X_2^{iv}$ is selected from the group consisting of —H and —$CH_3$; (2) $B_1$ and $B_4$ are selected from the group consisting of tyrosyl, tryptophyl, phenylalanyl, and the side-chain protected forms thereof; (3) $B_3$ is selected from the group consisting of glycyl, alanyl, valyl, leucyl, isoleucyl, prolyl, seryl, threonyl, methionyl, aspartyl, glutamyl, asparaginyl, glutaminyl, histidyl and the side-chain protected forms thereof; and, with respect to formulas (VIII) and (X), (4) Y is selected from the group consisting of —$NR_1R_2$, —OR, and —$CH_2OR$, wherein each R, $R_1$, and $R_2$ is selected from a group consisting of hydrogen and straight and branched chain alkyl groups containing 1—6 carbon atoms; then at least one of $B_2$, $B_5$, and $B'_5$ is selected such that it is not from a group consisting of D-tyrosyl, D-tryptophyl, D-phenylalanyl, and, with respect to $B'_5$, the descarboxy forms thereof, and the side-chain protected forms thereof; (b) when (1) a is 1 and b and m are 0 and $X_2^{iv}$ is selected from the group consisting of —H and —$CH_3$; (2) $B_2$ and $B_5$ or $B'_5$ are selected from the group consisting of D-tyrosyl, D-tryptophyl, D-phenylalanyl, and, with respect to $B'_5$, the descarboxy forms thereof, and the side-chain protected forms thereof; (3) $B_3$ is selected from the group consisting of glycyl, alanyl, valyl, leucyl, isoleucyl, prolyl, seryl, threonyl, methionyl, aspartyl, glutamyl, asparaginyl, glutaminyl, histidyl, and the side-chain protected forms thereof; and, with respect to formulas (VIII) and (X), (4) Y is selected from the group consisting of —$NR_1R_2$, —OR, and —$CH_2OR$, wherein each R, $R_1$, and $R_2$ is selected from a group consisting of hydrogen and straight and branched chain alkyl groups containing 1—6 carbon atoms; then at least one of $B_1$ and $B_4$ is selected such that it is not from a group consisting of tyrosyl, tryptophyl, phenylalanyl, and the side-chain protected forms thereof; (c) when (1) a is 1 and b and m are 0 and $X_2^{v}$ is selected from the group consisting of —H, —$CH_3$, and —$CHOCH_3$; (2) $B_1$ and $B_4$ are selected from the group consisting of tyrosyl, tryptophyl, phenylalanyl, and the side-chain protected forms thereof; (3) $B_3$ is selected from the group consisting of glycyl, alanyl, valyl, leucyl, isoleucyl, seryl, threonyl, methionyl, asparaginyl, glutaminyl, and the side-chain protected forms thereof; (4) $B_6$ is selected from the group consisting of asparaginyl, glutaminyl, glutamyl, arginyl, lysyl, seryl, threonyl, and the side-chain protected forms thereof; and, with respect to formulas (XI) and (XIII), (5) Y is selected from the group consisting of —$NR_1R_2$, —OR, and —$CH_2OR$, wherein each R, $R_1$, and $R_2$ is selected from a group consisting of hydrogen and straight and branched chain alkyl groups containing 1—6 carbon atoms; then at least one of $B_2$ and $B_5$ is selected such that it is not from a group consisting of D-tyrosyl, D-tryptophyl, D-phenylalanyl, and the side-chain protected forms thereof; and (d) when (1) a is 1 and b and m are 0 and $X_2^{v}$ is selected from the group consisting of —H, —$CH_3$, and —$CHOCH_3$; (2) $B_2$ and $B_5$ are selected from the group consisting of D-tyrosyl, D-tryptophyl, D-phenylalanyl, and the side-chain protected forms thereof; (3) $B_3$ is selected from the group consisting of glycyl, alanyl, valyl, leucyl, isoleucyl, prolyl, seryl, threonyl, methionyl, aspartyl, glutamyl, asparaginyl, glutaminyl, histidyl, and the side-chain protected forms thereof; (4) $B_6$ is selected from the group consisting of asparaginyl, glutaminyl, glutamyl, arginyl, lysyl, seryl, threonyl, and the side-chain protected forms thereof; and, with respect to formulas (XI) and (XIII), (5) Y is selected from the group consisting of —$NR_1R_2$, —OR, and —$CH_2OR$, wherein each R, $R_1$, and $R_2$ is selected from a group consisting of hydrogen and straight and branched chain alkyl groups containing 1—6 carbon atoms; then at least one of $B_1$ and $B_4$ is selected such that it is not from a group consisting of tyrosyl, tryptophyl, phenylalanyl, and the side-chain protected forms thereof.

Preferably, the peptide resins of formula VI, and the protected peptide-compounds of formulas VIII—X, have the amino acid sequence wherein $B_1$ and $B_4$ are selected from the group consisting of histidyl, tryptophyl, phenylalanyl, tyrosyl, homologues and analogues thereof, the side-chain protected forms thereof, and, with respect to $B_1$, the desamino forms thereof; $B_2$, $B_5$ and $B'_5$ are selected from the group consisting of D-histidyl, D-tryptophyl, D-phenylalanyl, D-tyrosyl, homologues and analogues thereof, and, with respect to $B'_5$, the descarboxy forms thereof, and the side-chain protected forms thereof; and $B_3$ is selected from the group consisting of glycyl, alanyl, seryl, asparaginyl, prolyl, D-alanyl, D-seryl, D-asparaginyl, D-prolyl, homologues and analogues thereof, and the side-chain protected forms thereof.

Preferably, the peptide resins of formula VII, and the protected peptide-compounds of formulas XI—XIII, have the amino acid sequence wherein $B_1$ and $B_4$ are selected from the group consisting of

histidyl, tryptophyl, phenylalanyl, tyrosyl, homologues and analogues thereof, the side-chain protected forms thereof, and, with respect to $B_1$, the desamino forms thereof; $B_2$ and $B_5$ are selected from the group consisting of D-histidyl, D-tryptophyl, D-phenylalanyl, D-tyrosyl, homologues and analogues thereof, and the side-chain protected forms thereof; $B_3$ is selected from the group consisting of glycyl, alanyl, seryl, asparaginyl, prolyl, D-alanyl, D-seryl, D-asparaginyl, D-prolyl, homologues and analogues thereof, and the side-chain protected forms thereof, and $B_6$ and $B'_6$ are selected from the group consisting of arginine, lysine, ornithine, histidine, aspartic acid, glutamic acid, asparagine, glutamine, D-arginine, D-lysine, D-ornithine, D-histidine, D-aspartic acid, D-glutamic acid, D-asparagine, D-glutamine, D-arginine, homologues and analogues thereof, and the descarboxy forms thereof; and, with respect to $B'_6$, the descarboxy forms thereof, and the side-chain protection forms thereof.

More preferably, the peptide-resins of this invention are represented by formulas XIV and XV and the protected peptide-compounds are represented by formulas XVI—XXI:

$(Pr_1)_q$
|
$(X^{iv}{}_1)_m$
|
$(Pr_1)_r—(X^{iv}{}_2)_a—B_1—D\text{-}Trp—B_3—B_4—B_5—Ⓡ$ (XIV)
|
$(X^{iv}{}_3)_b$
|
$(Pr_1)_s$

$(Pr_1)_q$
|
$(X_1{}^{v})_m$
|
$(Pr_1)_r—(X_2{}^{v})_a—B_1—D\text{-}Trp—B_3—B_4—B_5—B_6—Ⓡ$ (XV)
|
$(X^{v}{}_3)_b$
|
$(Pr_1)_s$

$(Pr_1)_q$
|
$(X_1{}^{iv})_m$
|
$(Pr_1)_r—(X_2{}^{iv})_a—B_1—D\text{-}Trp—B_3—B_4—B'_5—Y$ (XVI)
|
$(X_3{}^{iv})_b$
|
$(Pr_1)_s$

$(Pr_1)_q$
|
$(X_1{}^{iv})_m$
|
$(Pr_1)_r—(X_2{}^{iv})_a—B_1—D\text{-}Trp—B_3—B_4—B_5—O—Y$ (XVII)
|
$(X_3{}^{iv})_b$
|
$(Pr_1)_s$

$B_1—D\text{-}Trp—B_3—B_4—B'_5—Y$ (XVIII)

$(Pr_1)_q$
|
$(X_1{}^{v})_m$
|
$(Pr_1)_r—(X_2{}^{v})_a—B_1—D\text{-}Trp—B_3—B_4—B_5—B'_6—Y$ (XIX)
|
$(X_3{}^{v})_b$
|
$(Pr_1)_s$

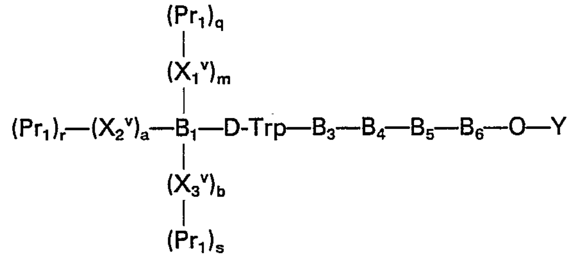

(XX)

$B_1$—D-Trp—$B_3$—$B_4$—$B_5$—$B_6$—O—Y (XII)

wherein $B_1$ is selected from the group consisting of tyrosyl, O-methyltyrosyl, histidyl, 3-N-methylhistidyl, p-chlorophenylalanyl, the desamino forms thereof, and the side-chain protected forms thereof; $B_3$ is selected from the group consisting of alanyl, seryl, D-alanyl and the side-chain protected forms thereof; $B_4$ is selected from the group consisting of tryptophyl, tyrosyl and the side-chain protected forms thereof; $B_5$ and $B'_5$ are selected from the group consisting of D-phenylalanyl, D-histidyl, D-tyrosyl, D-p-chlorophenylalanyl, and, with respect to $B'_5$ the descarboxy forms thereof, and the side-chain protected forms thereof and $B_6$ and $B'_6$ are selected from the group consisting of arginine, homoarginine, lysine, ornithine, aspartic acid, glutamic acid, asparagine, glutamine, and D-lysine.

Suitable α-amino acid protecting groups $Pr_1$, include, but are not limited to, tertiary-butyloxycarbonyl (BOC), isoamyloxycarbonyl (AOC), o-nitrophenylsulfenyl (NPS), fluoroenylmethyloxycarbonyl (FMOC), o-nitropyridinylsulfenyl (NPYS), and biphenylproploxycarbonyl (BPOC).

Suitable carboxyl protecting groups, $Pr_2$, include, but are not limited to, salts (e.g., $Li^+$, $Na^+$, $CS^+$, etc.), methyl, ethyl, benzyl, benzhydryl, substituted benzyl, phthalimidomethyl, tertiary butyl, phenacyl, phenyl, 4-picolyl, 2-methylthioethyl, 2(p-toluenesulfonyl)ethyl, 2(p-nitrothiophenyl)ethyl, p-methylthiophenyl, and hydrazides.

In addition to the resins, ⓡ, noted above, other resins include, but are not limited to, phenylacetamidomethyl (PAM), chloromethyl, and poly-N-acrylpyrolidine resins.

Virtually any suitable N-terminal and desamino alpha-carbon substitution and radical can be used in the instant invention. Typical N-terminal and desamino alpha-carbon substitutions and radicals include, but are not limited to, those set forth in Table V.

TABLE V

N-Terminus and Desamino Substitutions and Radicals[1]

| $X'_1$ | $X'_2$ | $X'_3$ | a | b | m | q | r | s |
|---|---|---|---|---|---|---|---|---|
| N-Terminus Substitutions and Radicals | | | | | | | | |
| $R_1$—($R_1$≠H) | $R_2$—($R_2$≠H) | — | 1 | 0 | 1 | 0 | 0 | 0 |
| — | $R_2$— | — | 1 | 0 | 0 | 1 | 0 | 0 |
| $R_1$—($R_1$≠H) | $R_2$—($R_2$≠H) | $R_3$—($R_3$≠H) | 1 | 1 | 1 | 0 | 0 | 0 |
| $R_1$— | $R_2$— | $R_3$— | 1 | 1 | 1 | 0 | 0 | 0 |
| O<br>‖<br>$R_1$—Z—C— | $R_2$— | — | 1 | 0 | 1 | 0 | 0 | 0 |
| O<br>‖<br>$R_1$—C— | $R_2$— | — | 1 | 0 | 1 | 0 | 0 | 0 |
| $R_1$— | —O— | — | 1 | 0 | 1 | 0 | 1 | 0 |
| $R_2$<br>\|<br>$R_1$—N—($R_1$&$R_2$≠H) | $R_3$—($R_3$≠H) | — | 1 | 0 | 1 | 0 | 0 | 0 |
| $R_2$<br>\|<br>$R_1$—N—($R_1$&$R_2$≠H) | — | — | 0 | 0 | 1 | 0 | 1 | 0 |

TABLE V (continued)

| $X_1'$ | $X_2'$ | $X_3'$ | a | b | m | q | r | s |
|---|---|---|---|---|---|---|---|---|
| 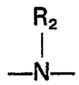 | — | — | O | O | I | I | I | O |
| 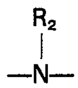 | $R_3$—($R_3$≠H) | — | I | O | I | I | O | O |
| 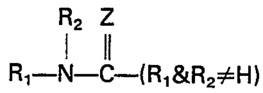 | $R_3$—($R_3$≠H) | — | I | O | I | O | O | O |
| 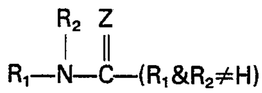 | — | — | O | O | I | O | I | O |
| 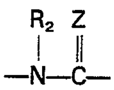 | $R_3$—($R_3$≠H) | — | I | O | I | I | O | O |
| Desamino Substituents and Radicals | | | | | | | | |
| R— | — | — | O | O | I | O | O | O |
| RZ—(R≠H) | — | — | O | O | I | O | O | O |
| —Z— | — | — | O | O | I | I | O | O |

1. LEGEND:, R, $R_1$, $R_2$, $R_3$, and Z are as defined in Table I, supra.

The growth hormone releasing peptides of formulas I and II and combinations thereof (including combinations of formula III) with at least one growth promoting agent are useful *in vitro* as unique tools for understanding how growth hormone secretion is regulated at the pituitary level. This includes use in the evaluation of many factors thought or known to influence growth hormone secretin such as age, sex, nutritional factors, glucose, amino acids. In addition, the peptides of this invention can be used in the evaluation of how other hormones modify growth hormone releasing activity. For example, it has already been established that somatostatin inhibits growth hormone release. Other hormones that are important and in need of study as to their effect on growth hormone release include the gonadal hormones, e.g., testosterone, estradiol, and progesterone; the adrenal hormones, e.g., cortisol and other corticoids, epinephrine and norepinephrine; the pancreatic and gastrointestinal hormones, e.g., insulin, glucagon, gastrin, secretin; the vasoactive intestinal peptides, e.g., bombesin; and the thyroid hormones, e.g., thyroxine and thriiodothyronine. These peptides and combinations of this invention can also be employed to investigate the possible negative or positive feedback effects of some of the pituitary hormones, e.g., growth hormone and endorphin peptides, on the pituitary to modify growth hormone release. Of particular scientific importance is the use of these peptides to elucidate the subcellular mechanisms mediating the release of growth hormone.

The peptides and combinations of this invention can also be administered to animals, including man, to release growth hormone *in vivo*. For example, the peptides can be administered to commercially important animals such as swine, cattle, sheep and the like to accelerate and increase their rate and extent of growth, and to increase milk production in such animals. In addition, these peptides can be administered to humans *in vivo* as a diagnostic tool to directly determine whether the pituitary is capable of releasing growth hormone. For example, the peptides and combinations can be administered *in vivo* to children. Serum samples taken before and after such administration can be assayed for growth hormone. Comparison of the amounts of growth hormone in each of these samples would be a means for directly determining the ability of the patient's pituitary to release growth hormone.

Accordingly, the present invention includes within its scope pharmaceutical compositions comprising, as an active ingredient, at least one of the peptides or combinations of this invention in association with a pharmaceutical carrier or diluent. Optionally, the active ingredient of the pharmaceutical compositions can comprise a growth promoting agent in addition to at least one of the peptides of formulas I or II or another composition which exhibits a different activity, e.g., antibiotic or other pharmaceutically active material.

Growth promoting agents include, but are not limited to, TRH, diethylstilbesterol, theophylline,

enkephalins, E series prostaglandins, compounds disclosed in U.S. Patent 3,239,345, e.g., zeranol, and compounds disclosed in U.S. Patent 4,036,979, e.g., sulbenox.

The peptides of this invention can be administered by oral, parenteral (e.g., intramuscular, intraperitoneal, intravenous or subcutaneous injection, or implant), nasal, vaginal, rectal, sublingual, or topical routes of administration and can be formulated in dosage forms appropriate for each route of administration.

Solid dosage forms for oral administration include capsules, tablets, pills, powders and granules. In such solid dosage forms, the active compound is admixed with at least one inert pharmaceutically acceptable carrier such as sucrose, lactose, or starch. Such dosage forms can also comprise, as is normal practice, additional substances other than inert diluents, e.g., lubricating agents such as magnesium stearate. In the case of capsules, tablets and pills, the dosage forms may also comprise buffering agents. Tablets and pills can additionally be prepared with enteric coatings.

Liquid dosage forms for oral administration include pharmaceutically acceptable emulsions, solutions, suspensions, syrups, the elixirs containing inert diluents commonly used in the art, such as water. Besides such inert diluents, compositions can also include adjuvants, such as wetting agents, emulsifying and suspending agents, and sweetening, flavoring, and perfuming agents.

Preparations according to this invention for parenteral administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. They may be sterilized by, for example, by filtration through a bacteria-retaining filter, by incorporating sterilizing agents into the compositions, by irradiating the compositions, or by heating the compositions. They can also be manufactured in the form of sterile solid compositions which can be dissolved in sterile water, or some other sterile injectable medium immediately before use.

Compositions for rectal or vaginal administration are preferably suppositories which may contain, in addition to the active substance, excipients such as cocoa butter or a suppository wax.

Compositions for nasal or sublingual administration are also prepared with standard excipients well known in the art.

The dosage of each active ingredient in the compositions of this invention may be varied; however, it is necessary that the amount of the active ingredient be such that a suitable dosage form is obtained. The selected dosage depends upon the desired therapeutic effect, on the route of administration, and on the duration of the treatment. Generally, dosage levels per active ingredient of between 0.001 to 10 mg/kg. of body weight daily are administered to animals, e.g., mammals, to obtain effective release of growth hormone.

The following examples are provided for the purpose of further illustration only and are not intended to be limitations on the disclosed invention.

## Example 1

### Synthesis of $H_2$-His-D-Trp-Ala-Trp-D-Phe $NH_2$

Para-methylbenzhydrylamine hydrochloride (p-Me-BHA.HCl) resin was placed in a reaction vessel. The following procedure, starting at step 6, was then employed in conjunction with a Beckman brand Peptide Synthesizer Model No. 990 in preparing the peptide $H_2$-His-D-Trp-Ala-Trp-D-Phe-$NH_2$. The synthesis was started at step 6 because there was no amino acid present in the resin and one need only neutralize the resin which was initially in the HCl form.

1. Was with methylene chloride ($CH_2Cl_2$) for 1.5 minutes, three times.
2. Treat with trifluoroacetic acid — methylene chloride (40% TFA/$CH_2Cl_2$, V/V) containing 0.1% indole for 1.5 minutes.
3. Repeat Step 2 for 20 minutes.
4. Wash with chloroform ($CHCl_3$) for 1.5 minutes, three times.
5. Wash with 30% ethanol-methylene chloride (30% EtOH/$CH_2Cl_2$, V/V) for 1.5 minutes, two times.
6. Wash with $CH_2Cl_2$ for 1.5 minutes, three times.
7. Treat with 10% triethylamine in $CH_2Cl_2$ (10% TEA/$CH_2Cl_2$, V/V) for 1.5 minutes.
8. Repeat Step 7 for 10 minutes.
9. Wash with $CH_2Cl_2$ for 1.5 minutes, three times.
10. Add to the washed resin 2.5 equivalents of the appropriate protected amino acid in dimethyl formamidemethylene chloride (DMF—$CH_2Cl_2$).
11. Add 0.5N dicyclohexylcarbodiimide in $CH_2Cl_2$ (DCC/$CH_2Cl_2$); more than 2.5 equivalents.
12. Rinse addition funnel with $CH_2Cl_2$ and add rinse to the reaction vessel.
13. Stir the reagents in Steps 10—12 for 2 hours or more.
14. Wash with $CH_2Cl_2$ for 1.5 minutes, three times.
15. Wash with DMF for 1.5 minutes.
16. Wash with $CH_2Cl_2$ for 1.5 minutes, two times.
17. Test by ninhydrin reaction according to the procedure of Kaiser et al., *Annal. Biochem.*, *34*:595 (1970).

18. If Step 17 shows complete reaction, repeat the above procedures starting from Step 1 employing the next protected amino acid. If Step 17 shows incomplete reaction, repeat Steps 7—17.

The above procedure was employed using the following sequence of amino acids:

c-D-Phe
Boc-Trp
Boc-Ala
Boc-D-Trp
Boc-His(Tos*)

*Tos denotes p-toluenesulfonyl.

After completion of the synthesis of the desired peptide resin, the reaction vessel containing the peptide resin was then placed in a dessicator and dried overnight under a vacuum. The dried peptide resin was removed from the reaction vessel and placed in another vessel suitable for HF cleavage. This latter vessel also contained a magnetic stirring bar. A quantity of anisole sufficient to wet the peptide resin was added to this vessel. The vessel was next connected to an HF line and placed under a vacuum to remove any air therein. The vessel was then cooled to about −78°C. with a dry ice-acetone bath. Doubly distilled HF (about 10 ml/gm of peptide resin) was added to the vessel and replaced by an ice-water bath. The vessel's contents were vigorously stirred for about 45 minutes while the vessel remained immersed in the ice-water bath. Most of the HF in the vessel was then removed by water aspiration. After the majority of HF was removed by water aspiration, the remaining HF and anisole were removed via a vacuum pump.

The vessel's contents were washed with about 100 ml of ether to further remove any residual anisole.

The peptide was removed from the resin by extraction with aqueous acetic acid (aq·HOAc). The aq·HOAc was lyophilized off to yield a fluffy peptide powder.

The peptide was then purified by partition chromatography or counter current distribution (CCD) employing a butanol:HOAc:water (4:1:5) system. When further purification was necessary, a Pharmacia LH—20 brand chromatography column was also employed.

Example 2

Synthesis of $H_2$-Tyr-D-Trp-Ala-Trp-D-His-$NH_2$

The procedure set forth in Example 1 can be employed to synthesize the peptide $H_2$-Tyr-D-Trp-Ala-Trp-D-His-$NH_2$ employing the following sequence of amino acids:

Boc-D-His(Tos)
Boc-Trp
Boc-Ala
Boc-D-Trp
Boc-Tyr(BrZ*)

*BrZ denotes o-bromobenzyloxycarbonyl

Example 3

Synthesis of $H_2$-His-D-Trp-Ala-Trp-D-Tyr-$NH_2$

The procedure set forth in Example 1 can be employed to synthesize the peptide $H_2$-His-D-Trp-Ala-Trp-D-Tyr-$NH_2$ employing the following sequence of amino acids:

Boc-D-Tyr (BrZ)
Boc-Trp
Boc-Ala
Boc-D-Trp
Boc-His (Tos)

Example 4

Synthesis of $H_2$-His-D-Trp-Ala-Trp-D-His-$NH_2$

The procedure set forth in Example 1 can be employed to synthesize the peptide $H_2$-His-D-Trp-Ala-Trp-D-His-$NH_2$ employing the following sequence of amino acids:

Boc-D-His (Tos)
Boc-Trp
Boc-Ala
Boc-D-Trp
Boc-His (Tos)

Example 5

Synthesis of $H_2$-Tyr-D-Trp-Ala-Trp-D-p-Cl-Phe-$NH_2$

The procedure set forth in Example 1 can be employed to synthesize the peptide $H_2$-Tyr-D-Trp-Ala-Trp-D-p-Cl-Phe-$NH_2$ employing the following sequence of amino acids:

Boc-D-p-Cl-Phe
Boc-Trp
Boc-Ala
Boc-D-Trp
Boc-Tyr(BrZ)

Example 6

Synthesis of $H_2$-Tyr-D-Trp-D-Ala-Trp-D-Phe-$NH_2$

The procedure set forth in Example 1 can be employed to synthesize the peptide $H_2$-Tyr-D-Trp-D-Ala-Trp-D-Phe-$NH_2$ employing the following sequence of amino acids:

Boc-D-Phe
Boc-Trp
Boc-D-Ala
Boc-D-Trp
Boc-Tyr(BrZ)

Example 7

Synthesis of $H_2$-p-Cl-Phe-D-Trp-Ala-Trp-D-Phe-$NH_2$

The procedure set forth in Example 1 can be employed to synthesize the peptide $H_2$-p-Cl-Phe-D-Trp-Ala-Trp-D-Phe-$NH_2$ employing the following sequence of amino acids:

Boc-D-Phe
Boc-Trp
Boc-Ala
Boc-D-Trp
Boc-p-Cl-Phe

Example 8

Synthesis of H-desamino Tyr-D-Trp-Ala-Trp-D-Phe-$NH_2$

The procedure set forth in Example 1 can be employed to synthesize the peptide H-desamino Tyr-D-Trp-Ala-Trp-D-Phe-$NH_2$ employing the following sequence of amino acids:

Boc-D-Phe
Boc-Trp
Boc-Ala
Boc-D-Trp
3(p-OH-phenyl)propanoic acid

Example 9

H
|
Synthesis of $CH_3CO$-Tyr-D-Trp-Ala-Trp-D-Phe-$NH_2$

The procedure set forth in Example 1 can be employed with several modifications to synthesize the peptide

H
|
$CH_3CO$-Tyr-D-Trp-Ala-Trp-D-Phe-$NH_2$

employing the following sequence of amino acids:

Boc-D-Phe
Boc-Trp
Boc-Ala
Boc-D-Trp
Boc-Tyr(BrZ)

The modifications consisted of the following additional steps after the last protected amino acid, Boc-Tyr(BrZ) was added to the peptide resin:

19. The Boc group was removed from the peptide resin by TFA.
20. The resulting peptide resin was washed with $CH_2Cl_2$ for 1.5 minutes, two times.
21. Acetic anhydride (2.5 molar excess) and 2.5 molar excess of pyridine were added and stirred for about 10 minutes.
22. Repeat Step 20.

The same drying and purification steps as used in Example 1 were then employed to obtain the desired peptide.

Example 10

Synthesis of $H_2$-O-Me-Tyr-D-Trp-Ala-Trp-D-Phe-$NH_2$

The procedure set forth in Example 1 can be employed to synthesize the peptide $H_2$-O-Me-Tyr-D-Trp-Ala-Trp-D-Phe-$NH_2$ employing the following sequence of amino acids:

Boc-D-Phe
Boc-Trp
Boc-Ala
Boc-D-Trp
Boc-O-Me-Tyr(BrZ)

Example 11

Synthesis of $H_2$-Tyr-D-Trp-Ala-Trp-D-Phe-Met-$NH_2$

BHA·HCl resin was placed in a reaction vessel. The following procedure was then employed in conjunction with a Beckman brand Peptide Synthesizer Model No. 990 in preparing the hexapeptide $H_2$-Tyr-D-Trp-Ala-Trp-D-Phe-Met-$NH_2$:

1. Methylene chloride ($CH_2Cl_2$; about 10 ml/gm of BHA·HCl resin) was added to the reaction vessel. The BHA·HCl resin was washed with vigorous stirring for about 1.5 minutes. The $CH_2Cl_2$ solution was then drained from the reaction vessel. This washing step was repeated once.

2. A triethylamine solution (($Et_3N$)/$CH_2Cl_2$ (10:90); about 10 ml/gm BHA·HCl resin) was added to the washed BHA·HCl resin in the reaction vessel. The resulting mixture was vigorously stirred for about 1.5 minutes. The solution was then drained from the reaction vessel.

3. Another $Et_3N$/$CH_2Cl_2$ (10:90) solution (about 10 ml/gm BHA·HCl was added to the reaction vessel. The BHA·HCl resin was neutralized by vigorous stirring for about 20 minutes. The solution was then drained from the reaction vessel.

4. $CH_2Cl_2$ (about 10 ml/gm of BHA·HCl resin) was added to the reaction vessel. The resulting mixture was vigorously stirred for about 1.5 minutes. The solution was then drained from the reaction vessel. This procedure was repeated an additional two times.

5. Tertiarybutyloxycarbonyl-methionine (Boc-Met; about 2.5 times the theoretical amount of the total binding capacity of the BHA·HCl resin originally placed in the reaction vessel) in about 50 ml of dimethylformamidemethylene chloride solution (DMF—$CH_2Cl_2$ (1:9)) was added to the reaction vessel. The resulting mixture was vigorously stirred for about 1.5 minutes.

6. A 0.5 molar (M) dicyclohexylcarbodiimide (DCC) in $CH_2Cl_2$ solution (about 2.5 times the theoretical amount of total binding capacity of the BHA·HCl resin originally placed in the reaction vessel) was added to the reaction vessel. The resulting mixture was vigorously stirred until a negative ninhydrin test was obtained (about 120 minutes). The solution was then drained from the reaction vessel.

7. $CH_2Cl_2$ (about 10 ml/gm of BHA·HCl resin) was added to the reaction vessel. The resulting solution was vigorously stirred for about 1.5 minutes. The solution was then drained from the reaction vessel. This washing procedure was repeated once.

8. DMF (about 10 ml/gm of BHA·HCl resin) was added to the reaction vessel. The resulting mixture was stirred for about 1.5 minutes. The solution was then drained from the reaction vessel.

9. $CH_2Cl_2$ (about 10 ml/cm of BHA·HCl resin) was added to the reaction vessel. The resulting mixture was vigorously stirred for about 1.5 minutes. The solution was then drained from the reaction vessel. This washing procedure was repeated an additional two times.

10. A trifluoroacetic acid/methylene chloride solution (TFA/$CH_2Cl_2$ (40:60); about 10 ml/gm of BHA·HCl resin) was added to the reaction vessel. The resulting mixture was vigorously stirred for about 1.5 minutes. The solution was then drained from the reaction vessel.

11. Another TFA/$CH_2Cl_2$ (40:60) solution (about 10 ml/gm of BHA·HCl resin) was added to the reaction vessel. The resulting mixture was vigorously stirred for about 20 minutes. The solution was then drained from the reaction vessel.

12. $CH_2Cl_2$ (about 10 ml/gm of BHA·HCl resin) was added to the reaction vessel. The resulting solution was vigorously stirred for about 1.5 minutes. The solution was then drained from the reaction vessel. This washing procedure was repeated once.

13. A triethylamine solution (($Et_3N$)/$CH_2Cl_2$ (10:90); about 10 ml/gm BHA HCl resin) was added to the washed BHA·HCl resin in the reaction vessel. The resulting mixture was vigorously stirred for about 1.5 minutes. The solution was then drained from the reaction vessel.

14. Another $Et_3N$/$CH_2Cl_2$ (10:90) solution (about 10 ml/gm BHA·HCl) was added to the reaction vessel. The BHA·HCl resin was neutralized by vigorously stirring for about 20 minutes. The solution was then drained from the reaction vessel.

15. Chloroform ($CHCl_3$; about 10 ml/gm of BHA·HCl resin) was added to the reaction vessel. The resulting mixture was vigorously stirred for about 1.5 minutes. The solution was then drained from the reaction vessel.

16. An ethanol/methylene chloride solution (EtOH/$CH_2Cl_2$ (30:70); about 10 ml/gm of BHA·HCl resin) was added to the reaction vessel. The resulting mixture was vigorously stirred for about 1.5 minutes. The solution was then drained from the reaction vessel. This washing step was repeated once.

Steps 4 through 16 were then repeated employing the following sequence of amino acids:

Boc-D-Phe
Boc-Trp
Boc-Ala
Boc-D-Trp
Boc-Tyr (BrZ*)

*Brz denotes o-bromobenzyloxycarbonyl

After completion of the synthesis of the desired peptide resin, the reaction vessel containing the peptide resin was then placed in a dessicator and dried overnight under a vacuum. The dried peptide resin was removed from the reaction vessel and placed in another vessel suitable for HF cleavage. This latter vessel also contained a magnetic stirring bar. A quantity of anisole sufficient to wet the peptide resin was

added to this vessel. The vessel was next connected to an HF-line and placed under a vacuum to remove any air therein. The vessel was then cooled to about −78°C. with a dry ice-acetone. Doubly distilled HF (about 10 ml/gm of peptide resin) was added to the vessel. The dry ice-acetone bath was then removed from the vessel and replaced by an ice-water bath. The vessel's contents were vigorously stirred for about 45 minutes while the vessel remained immersed in the ice-water bath. Most of the HF in the vessel was then removed by water aspiration, the remaining HF and anisole were removed via a vacuum pump.

The vessel's contents were washed with about 100 ml of ether to further remove any residual anisole.

The peptide was removed from the resin by extraction with 30% aqueous acetic acid (aq.HOAc). The aq.HOAc was lyophilized off to yield a fluffy peptide powder.

The peptide was then purified by partition chromatography or counter current distribution (CCD) employing a butanol: HOAc: water (4:1:5) system. When further purification was necessary, a Pharmacia LH—20 brand chromatography column was also employed.

Example 12

Synthesis of $H_2$-Tyr-D-Trp-Ala-Trp-D-Phe-Thr-$NH_2$

The procedure set forth in Example 11 was employed to synthesize the hexapeptide $H_2$-Tyr-D-Trp-Ala-Trp-D-Phe-Thr-$NH_2$ employing the following sequence of amino acids:

Boc-Thr(Bzl*)
Boc-D-Phe
Boc-Trp
Boc-Ala
Boc-D-Trp
Boc-Tyr(BrZ)

*Bzl denotes benzyl.

Example 13

Synthesis of $H_2$-Tyr-D-Trp-Ala-Trp-D-Phe-Gln-OH

The procedure set forth in Example 11 was employed to synthesize the hexapeptide $H_2$-Tyr-D-Trp-Ala-Trp-D-Phe-Gln-OH employing the following sequence of amino acids:

Boc-Glu-α-benzyl ester
Boc-D-Phe
Boc-Trp
Boc-Ala
Boc-D-Trp
Boc-Tyr(BrZ)

Example 14

Synthesis of $H_2$-Tyr-D-Trp-Ala-Trp-D-Phe-Gln-$NH_2$

The procedure set forth in Example 11 was employed with several modifications to synthesize the hexapeptide $H_2$-Tyr-D-Trp-Ala-Trp-D-Phe-Gln-$NH_2$. The modifications consisted of:

(1) Omitting steps 2—4 of Example 11 and replacing them with a single step which entailed dissolving Boc-Gln (about 5 times the theoretical amount of the total binding of the BHA·HCl resin originally placed in the reaction vessel) in 10 ml of $CH_3Cl_2$-DMF solution (3:2) present in a round bottom flask. The flask and its contents was cooled in ice and then DCC (about 1.25 times the theoretical amount of the total binding capacity of the BHA·HCl resin originally placed in the reaction vessel) was added to the flask. The resulting mixture was stirred in an ice bath for about 25—30 minutes. The DCC urea precipitate formed by the reaction between Boc-Gln and DCC was separated from the supernatant via filtration. The supernature comprising the $CH_2Cl_2$-DMF solution having a symmetrical anhydride (also formed by the reaction between Boc-Gln and DCC) dissolved therein was added to the reaction vessel. The resulting mixture was stirred until a negative ninhydrin test was obtained. The solution was then drained from the reaction vessel.

(2) After completing steps 7 through 16 of Example 11, steps 4 through 16 of Example 11 were then repeated, without modifications, employing the following sequence of amino acids:

Boc-D-Phe
Boc-Trp
Boc-Ala
Boc-D-Trp
Boc-Tyr(BrZ)

Example 15

Synthesis of $H_2$-Tyr-D-Trp-Ala-Trp-D-Phe-Asn-$NH_2$

The procedure set forth in Example 11 was employed with several modifications to synthesize the hexapeptide $H_2$-Tyr-D-Trp-Ala-Trp-D-Phe-Asn-$NH_2$. The modifications consisted of:

(1) Omitting steps 2—4 of Example 11 and replacing them with a simple step which entailed dissolving Boc-Asn (about 5 times the theoretical amount of the total binding capacity of the BHA·HCl resin originally placed in the reaction vessel) in about 50 ml of DMF in a suitable flask. The resulting solution was added to

the reaction vessel to form a mixture. The mixture was vigorously stirred until a negative ninhydrin test was obtained. The solution was then drained from the reaction vessel.

(2) After completing steps 7 through 16 of Example 11, steps 4 through 16 of Example 11 were then repeated, without modification, employing the following sequence of amino acids:

Boc-D-Phe
Boc-Trp
Boc-Ala
Boc-Tyr(BrZ)

Example 16

Synthesis of $H_2$-His-D-Trp-D-Phe-Lys-$NH_2$

Para-methylbenzhydrylamino hydrochloride (p-Me-BHA·HCl) resin was placed in a reaction vessel. The following procedure, starting at step 6, was then employed in conjunction with a Beckman brand Peptide Synthesizer Model 990 in preparing the peptide $H_2$-His-D-Trp-Ala-Trp-D-Phe-Lys-$NH_2$. The synthesis was started at step 6 because there was no amino acid present in the resin and one need only neutralize the resin which was initially in the HCl form.

1. Wash with methylene chloride ($CH_2Cl_2$) for 1.5 minutes, three times.
2. Treat with trifluoroacetic acid — methylene chloride (40% TFA/$CH_2Cl_2$, V/V) containing 0.1% indole for 1.5 minutes.
3. Repeat Step 2 for 20 minutes.
4. Wash with chloroform ($CHCl_3$) for 1.5 minutes, three times.
5. Wash with 30% ethanol-methylene chloride (30% EtOH/$CH_2Cl_2$, V/V) for 1.5 minutes, two times.
6. Wash with $CH_2Cl_2$ for 1.5 minutes, three times.
7. Treat with 10% triethylamine in $CH_2Cl_2$ (10% TEA/$CH_2Cl_2$, V/V) for 1.5 minutes.
8. Repeat Step 7 for 10 minutes.
9. Wash with $CH_2Cl_2$ for 1.5 minutes, three times.
10. Add to the washed basin 2.5 equivalents of the appropriate protected amino acid in dimethyl formamidemethylene chloride (DMF—$CH_2Cl_2$).
11. Add 0.5N dicyclohexylcarbodiimide in $CH_2Cl_2$ (DCC/$CH_2Cl_2$); more than 2.5 equivalents.
12. Rinse addition funnel with $CH_2Cl_2$ and add rinse to the reaction vessel.
13. Stir the reagents in Steps 10—12 for 2 hours or more.
14. Wash with $CH_2Cl_2$ for 1.5 minutes, three times.
15. Wash with DMF for 1.5 minutes.
16. Wash with $CH_2Cl_2$ for 1.5 minutes, two times.
17. Test by ninhydrin reaction according to the procedure of Kaiser et al., *Annal. Biochem., 34*:595 (1970).
18. If Step 17 shows complete reaction, repeat the above procedures starting from Step 1 employing the next protected amino acid. If Step 17 shows incomplete reaction, repeat Steps 7—17.

The above procedure was employed using the following sequence of amino acids:

Boc-Lys(ClZ⁺)
Boc-D-Phe
Boc-Trp
Boc-Ala
Boc-D-Trp
Boc-His(Tos*)

⁺ClZ denotes o-chloro-benzyloxycarbonyl.
*Tos denotes p-toluenesulfonyl.

After completion of the synthesis of the desired peptide resin, the reaction vessel containing the peptide resin was then placed in a dessicator and dried overnight under a vacuum. The dried peptide resin was removed from the reaction vessel and placed in another vessel suitable for HF cleavage. This latter vessel also contained a magnetic stirring bar. A quantity of anisole sufficient to wet the peptide resin was added to this vessel. The vessel was next connected to an HF line and placed under a vacuum to remove any air therein. The vessel was then cooled to about −78°C. with a dry ice-acetone bath. Doubly distilled HF (about 10 ml/gm of peptide resin) was added to the vessel. The dry ice-acetone bath was then removed from the vessel and replaced by an ice-water bath. The vessel's contents were vigorously stirred for about 45 minutes while the vessel remained immersed in the ice-water bath. Most of the HF in the vessel was then removed by water aspiration. After the majority of HF was removed by water aspiration, the remaining HF and anisole were removed via a vacuum pump.

The vessel's contents were washed with about 100 ml of ether to further remove any residual anisole.

The peptide was removed from the resin by extraction with aqueous acetic acid (aq.HOAc). The aq. HOAc was lyophilized off to yield a fluffy peptide powder.

The peptide was then purified by partition chromatography or counter current distribution (CCD) employing a butanol:HOAc:water (4:1:5) system. When further purification was necessary, a Pharmacia LH—20 brand chromatography column was also employed.

Example 17

Synthesis of $H_2$-Tyr-D-Trp-Ala-Trp-D-Phe-Lys-$NH_2$

The procedure set forth in Example 16 can be employed to synthesize the peptide $H_2$-Tyr-D-Trp-Ala-Trp-D-Phe-Lys-$NH_2$ employing the following sequence of amino acids:

Boc-Lys(ClZ)
Boc-D-Phe
Boc-Trp
Boc-Ala
Boc-D-Trp
Boc-Tyr-BrZ*)

*Brz Denotes o-bromobenzyloxycarbonyl

Example 18

Synthesis of $H_2$-Tyr-D-Trp-Ala-Trp-D-Phe-Glu-$NH_2$

The procedure set forth in Example 16 can be employed to synthesize the peptide $H_2$-Tyr-D-Trp-Ala-Trp-D-Phe-Glu-$NH_2$ employing the following sequence of amino acids:

Boc-Glu-γ-Bzl*
Boc-D-Phe
Boc-Trp
Boc-Ala
Boc-D-Trp
Boc-Tyr(BrZ)

*γ-Bzl denotes γ-benzyl ester

Example 19

Synthesis of $H_2$-Tyr-D-Trp-Ala-Trp-D-Phe-Phe-$NH_2$

The procedure set forth in Example 16 can be employed to synthesize the peptide $H_2$-Tyr-D-Trp-Ala-Trp-D-Phe-Phe-$NH_2$ employing the following sequence of amino acids:

Boc-Phe
Boc-D-Phe
Boc-Trp
Boc-Ala
Boc-D-Trp
Boc-Tyr(BrZ)

Example 20

Synthesis of $H_2$-Tyr-D-Trp-Gly-Trp-D-Phe-Gln-$NH_2$

The procedure set forth in Example 16 can be employed with one modification to synthesize the peptide $H_2$-Tyr-D-Trp-Gly-Trp-D-Phe-Gln-$NH_2$ employing the following sequence of amino acids:

Boc-Gln-ONP*
Boc-D-Phe
Boc-Trp
Boc-Gly
Boc-D-Trp
Boc-Tyr(BrZ)

*ONP denote p-nitrophenyl ester.

The sole modification was made just in the procedure for coupling Boc-Gln-ONP to the resin. This modification consisted of the omission of step 11 of Example 16 in this one coupling procedure.

Example 21

Synthesis of $H_2$-His-D-Trp-Ala-Trp-D-Phe-Lys-OH

The procedure set forth in Example 16 can be employed with several modifications to synthesize the peptide $H_2$-His-D-Trp-Ala-Trp-D-Phe-Lys-OH employing the following sequence of amino acids:

Boc-Lys(ClZ)
Boc-D-Phe
Boc-Trp
Boc-Ala
Boc-D-Trp
Boc-D-Trp
Boc-His(Tos)

The modifications were as follows:

1. A hydroxymethyl resin was employed instead of the p-Me-BHA·HCl resin.

2. Steps 6—18 were modified as follows just in the procedure for coupling Boc-Lys(ClZ) to the hydroxymethyl resin:

a. Step 6 was the same;

b. Steps 7 through 9 were omitted;
c. Steps 10 and 11 were the same;
d. Step 12 was omitted and the following procedure was substituted therefor:
Add 2.5 equivalents of N,N-dimethylaminopyridine (DMAP).
e. Steps 13 through 16 were the same;
f. Steps 17 and 18 were omitted and the following procedure was substituted therefor:

Dry the amino acid-resin under vacuum until a constant weight is obtained. If the final constant weight is equal to the sum of the weights of the resin and Boc-amino acid added to the reaction vessel, then add 10 equivalents of benzoyl chloride and 10 equivalents of pyridine to deactivate any unused hydroxymethyl resin. Wash as set forth in steps 15 and 16. Then employ steps 1—18 of Example 16 for remaining protected amino acids.

If the final constant weight is not equal to the sum of the weights of the resin and Boc-amino acid added to the reaction vessel, then repeat steps 10 to end as modified above in this example.

Example 22

Synthesis of $H_2$-His-D-Trp-Ala-Trp-D-Phe-Arg-$NH_2$

The procedure set forth in Example 16 can be employed to synthesize the peptide $H_2$-His-D-Trp-Ala-Trp-D-Phe-Arg-$NH_2$ employing the following sequence of amino acids:

Aoc*-Arg(Tos)
Boc-D-Phe
Boc-Trp
Boc-Ala
Boc-D-Trp
Boc-His(Tos)

*Aoc denotes isoamyloxycarbonyl

Example 23

Synthesis of $H_2$-His-D-Trp-Ala-Trp-D-Phe-Gln-$NH_2$

The procedure set forth in Example 16 can be employed with one modification to synthesize the peptide $H_2$-His-D-Trp-Ala-Trp-D-Phe-Gln-$NH_2$ employing the following sequence of amino acids:

Boc-Gln-ONP
Boc-D-Phe
Boc-Trp
Boc-Ala
Boc-D-Trp
Boc-His(Tos)

The sole modification was made first in the procedure for coupling Boc-Gln-ONP to the resin. This modification consisted of the omission of step 11 of Example 16 in this one coupling procedure.

Example 24

Synthesis of $H_2$-His-D-Trp-Ala-Trp-D-Phe-Glu-$NH_2$

The procedure set forth in Example 16 can be employed to synthesize the peptide $H_2$-His-D-Trp-Ala-Trp-D-Phe-Glu-$NH_2$ employing the following sequence of amino acids:

Boc-Glu-γ-Bzl
Boc-D-Phe
Boc-Trp
Boc-Ala
Boc-D-Trp
Boc-His(Tos)

Example 25

Synthesis of $H_2$-His-D-Trp-Ala-Trp-D-Phe-HomoArg-$NH_2$

The procedure set forth in Example 16 can be employed to synthesize the peptide $H_2$-His-D-Trp-Ala-Trp-D-Phe-HomoArg-$NH_2$ employing the following sequence of amino acids:

Boc-HomoArg(Tos)
Boc-D-Phe
Boc-Trp
Boc-Ala
Boc-D-Trp
Boc-His(Tos)

Example 26

Synthesis of $H_2$-3-N-Me-His-D-Trp-Ala-Trp-D-Phe-Lys-$NH_2$

The procedure set forth in Example 16 can be employed to synthesize the peptide $H_2$-3-N-Me-His-D-Trp-Ala-Trp-D-Phe-Lys-$NH_2$ employing the following sequence of amino acids:

Boc-Lys(ClZ)
Boc-D-Phe
Boc-Trp
Boc-Ala
Boc-D-Trp
Boc-3-N-Me-His

Example 27

Synthesis of $H_2$-His-D-Trp-Ala-Trp-D-Phe-Lys-$NHCH_2CH_3$

The procedure set forth in Example 16 can be employed with several modifications to synthesize the peptide $H_2$-His-D-Trp-D-Ala-Trp-D-Phe-Lys-$NHCH_2CH_3$ employing the following sequence of amino acids:

Boc-Lys(ClZ)
Boc-D-Phe
Boc-Trp
Boc-D-Ala
Boc-D-Trp
Boc-His(Tos)

The following ethylamine ($CH_3CH_2NH_2$) cleavage procedure was employed in place of the HF cleavage procedure of Example 16:

After completion of the synthesis of the desired peptide resin, the reaction vessel containing the peptide resin was then placed in a dessicator and dried overnight under a vacuum. The dried peptide resin was removed from the reaction vessel and placed in another vessel suitable for ethylamine cleavage. This latter vessel also contained a magnetic stirring bar. The vessel was placed in an ice bath and gaseous ethylamine was condensed into the vessel. The contents of the vessel was stirred overnight.

This cleavage procedure was then followed by the extraction and purifications procedures as set forth in Example 16.

Example 28

Synthesis of $H_2$-His-D-Trp-Ala-Trp-D-Phe-Orn-$NH_2$

The procedure set forth in Example 16 can be employed to synthesize the peptide $H_2$-His-D-Trp-Ala-Trp-D-Phe-Orn-$NH_2$ employing the following sequence of amino acids:

Boc-Orn(Z*)
Boc-D-Phe
Boc-Trp
Boc-Ala
Boc-D-Trp
Boc-His(Tos)

*Z denotes benzyloxycarbonyl

Example 29

Synthesis of $H_2$-His-D-Trp-Val-Trp-D-Phe-Lys-$NH_2$

The procedure set forth in Example 16 can be employed to synthesize the peptide $H_2$-His-D-Trp-Val-Trp-D-Phe-Lys-$NH_2$ employing the following sequence of amino acids:

Boc-Lys(ClZ)
Boc-D-Phe
Boc-Trp
Boc-Val
Boc-D-Trp
Boc-His(Tos)

Example 30

Synthesis of $H_2$-His-D-Trp-Ser-Trp-D-Phe-Lys-$NH_2$

The procedure set forth in Example 16 can be employed to synthesize the peptide $H_2$-His-D-Trp-Ser-Trp-D-Phe-$NH_2$ employing the following sequence of amino acids:

Boc-Lys(ClZ)
Boc-D-Phe
Boc-Trp
Boc-Ser(Bzl)
Boc-D-Trp
Boc-His(Tos)

Example 31

In Vitro Growth Hormone Release Study

Female rats of the CD—1 strain were housed in a constant temperature room at 24°C. with 14 hours light and 10 hours darkness. The rats were fed Purina brand rat chow ab libitum. All studies were started between 0800 and 1000 hours.

Pituitaries were removed from 20 day old female rats. In each polytetrafluoroethylene beaker (10 ml) was incubated two pituitaries at 36°C. in 1 ml of lactated Ringer's solution in a Dubnoff Shaker (90 cycles/min.). Three beakers were employed for each dosage shown in Table VI. All medium in each beaker was removed each hour (e.g., $P_1$, $P_2$, $I_3$, $I_4$, $I_5$) and then fresh medium was added back to each beaker. Each medium removed was assayed for GH, in duplicate, by a standard radioimmunoassay (RIA).

The growth hormone agonist of Example 1 was not added to the incubation mediums employed during the first hour of the incubation period ($P_1$) or to the incubation mediums employed during the second hour of the incubation period ($P_2$). The growth hormone agonist of Example 1 was dissolved in dimethylsulfoxide (DMSO; 10:1, agonist:DMSO), added to each incubation medium employed during the third hour of the incubation period ($I_3$), to each medium employed during the third hour of the incubation period ($I_3$), to each medium employed during the fourth hour of the incubation period ($I_4$) and, when performed, to each medium employed during the fifth hour of the incubation period ($I_5$). The release of growth hormone was recorded as ΔGH values obtained from the three beakers per dosage level measured at $I_3$, $I_4$, and, when performed, $I_5$ are set forth in Table VI.

Example 32
In Vitro Growth Hormone Release Study

The procedure set forth in Example 31 was employed as an *in vitro* growth hormone release study of the peptide of Example 2 and the results therefrom are set forth in Table VII.

Example 33
In Vitro Growth Hormone Release Study

The procedure set forth in Example 31 was employed in an *in vitro* growth hormone release study of the peptide of Example 3 and the results therefrom are set forth in Table VIII.

Example 34
In Vitro Growth Hormone Study

The procedure set forth in Example 31 was employed as an *in vitro* growth hormone release study of the peptide of Example 4 and the results therefrom are set forth in Table IX.

Example 35
In Vitro Growth Hormone Release Study

The procedure set forth in Example 31 was employed as an *in vitro* growth hormone release study of the peptide of Example 5 and the results therefrom from are set forth in Table X.

Example 36
In Vitro Growth Hormone Release Study

The procedure set forth in Example 31 was employed in an *in vitro* growth hormone release study of the peptide of Example 6 and the results therefrom are set forth in Table XI.

Example 37
In Vitro Growth Hormone Release Study

The procedure set forth in Example 31 was employed as an *in vitro* growth hormone release study of the peptide of Example 7 and the results therefrom are set forth in Table XII.

Example 38
In Vitro Growth Hormone Release Study

The procedure set forth in Example 31 was employed in an *in vitro* growth hormone release study of the peptide of Example 8 and the results therefrom are set forth in Table XIII.

Example 39
In Vitro Growth Hormone Release Study

The procedure set forth in Example 31 was employed in an *in vitro* growth hormone release study of the peptide of Example 9 and the results therefrom are set forth in Table XIV.

Example 40
In Vitro Growth Hormone Release Study

The procedure set forth in Example 31 was employed in an *in vitro* growth hormone release study of the peptide of Example 10 and the results therefrom are set forth in Table XV.

Example 41
In Vitro Growth Hormone Release Study

The procedure set forth in Example 31 was employed in an *in vitro* growth hormone release study of the peptide of Example 11 and the results therefrom are set forth in Table XVI.

Example 42
In Vitro Growth Hormone Release Study
The procedure set forth in Example 31 was employed in an *in vitro* growth hormone release study of the peptide of Example 12 and the results therefrom are set forth in Table XVII.

Example 43
In Vitro Growth Hormone Release Study
The procedure set forth in Example 31 was employed in an *in vitro* growth hormone release study of the peptide of Example 13 and the results therefrom are set forth in Table XVIII.

Example 44
In Vitro Growth Hormone Release Study
The procedure set forth in Example 31 was employed in an *in vitro* growth hormone release study of the peptide of Example 14 and the results therefrom are set forth in Table XIX.

Example 45
In Vitro Growth Hormone Release Study
The procedure set forth in Example 31 was employed in an *in vitro* growth hormone release study of the peptide of Example 15 and the results therefrom are set forth in Table XX.

Example 46
In Vitro Growth Hormone Release Study
The procedure set forth in Example 31 was employed in an *in vitro* growth hormone release study of the peptide of Example 16 and the results therefrom are set forth in Table XXI.

Example 47
In Vitro Growth Hormone Release Study
The procedure set forth in Example 31 was employed in an *in vitro* growth hormone release study of the peptide of Example 17 and the results therefrom are set forth in Table XXII.

Example 48
In Vitro Growth Hormone Release Study
The procedure set forth in Example 31 was employed in an *in vitro* growth hormone release study of the peptide of Example 18 and the results therefrom are set forth in Table XXIII.

Example 49
In Vitro Growth Hormone Release Study
The procedure set forth in Example 31 was employed in an *in vitro* growth hormone release study of the peptide of Example 19 and the results therefrom are set forth in Table XXIV.

Example 50
In Vitro Growth Hormone Release Study
The procedures set forth in Example 31 was employed in an *in vitro* growth hormone release study of the peptide of Example 20 and the results therefrom are set forth in Table XXV.

Example 51
In Vitro Growth Hormone Release Study
The procedure set forth in Example 31 was employed in an *in vitro* growth hormone release study of the peptide of Example 21 and the results therefrom are set forth in Table XXVI.

Example 52
In Vitro Growth Hormone Release Study
The procedure set forth in Example 31 was employed in an *in vitro* growth hormone release study of the peptide of Example 22 and the results therefrom are set forth in Table XXVII.

Example 53
In Vitro Growth Hormone Release Study
The procedure set forth in Example 31 was employed in an *in vitro* growth hormone release study of the peptide of Example 23 and the results therefrom are set forth in Table XXVIII.

Example 54
In Vitro Growth Hormone Release Study
The procedure set forth in Example 31 was employed in an *in vitro* growth hormone release study of the peptide of Example 24 and the results therefrom are set forth in Table XXIX.

Example 55

In Vitro Growth Hormone Release Study

The procedure set forth in Example 31 was employed in an *in vitro* growth hormone release study of the peptide of Example 25 and the results therefrom are set forth in Table XXX.

Example 56

In Vitro Growth Hormone Release Study

The procedure set forth in Example 31 was employed in an *in vitro* growth hormone release study of the peptide of Example 26 and the results therefrom are set forth in Table XXXI.

Example 57

In Vitro Growth Hormone Release Study

The procedure set forth in Example 31 was employed in an *in vitro* growth hormone release study of the peptide of Example 27 and the results therefrom are set forth in Table XXXII.

Example 58

In Vitro Growth Hormone Release Study

The procedure set forth in Example 31 was employed in an *in vitro* growth hormone release study of the peptide of Example 28 and the results therefrom are set forth in Table XXXIII.

Example 59

In Vitro Growth Hormone Release Study

The procedure set forth in Example 31 was employed in an *in vitro* growth hormone release study of the peptide of Example 29 and the results therefrom are set forth in Table XXXIV.

Example 60

In Vitro Growth Hormone Release Study

The procedure set forth in Example 31 was employed in an *in vitro* growth hormone release study of the peptide of Example 30 and the results therefrom are set forth in Table XXXV.

TABLE VI

In Vitro Growth Hormone Release

| Dosage[2] $H_2$-His-D-Trp-Ala-Trp-D-Phe-$NH_2$ | $\Delta GH$[1] | p Value[4] |
|---|---|---|
| — | $-167 \pm 114$ | — |
| 1 | $206 \pm 168$ | NS[3] |
| 3 | $288 \pm 79$ | $<0.01$ |
| 10 | $2{,}005 \pm 203$ | $<0.001$ |
| 30 | $3{,}046 \pm 93$ | $<0.001$ |

1. The mean of 9 assays given in terms of ng/ml incubation medium ± standard error of the mean (SEM)
2. Given in terms of ng/ml incubation medium
3. NS denotes not significant
4. Comparison of the GH levels in medium containing growth hormone agonist analog to the GH levels in medium without the agonist

TABLE VII

In Vitro Growth Hormone Release

| Dosage[2] $H_2$-Tyr-D-Trp-Ala-Trp-D-His-$NH_2$ | ΔGH[1] | p Value[4] |
|---|---|---|
| — | −167 ± 114 | — |
| 1 | −179 ± 323 | NR[3] |
| 3 | −192 ± 120 | NS |
| 10 | −802 ± 302 | NS |
| 30 | 611 ± 103 | <0.001 |

1. The mean of 9 assays given in terms of ng/ml incubation medium ± standard error of the mean (SEM)
2. Given in terms of ng/ml incubation medium
3. NS denotes not significant
4. Comparison of the GH levels in medium containing growth hormone agonist analog to the GH levels in medium without the agonist

TABLE VIII

In Vitro Growth Hormone Release

| Dosage[2] $H_2$-His-D-Trp-Ala-Trp-D-Tyr-$NH_2$ | ΔGH[1] | p Value[4] |
|---|---|---|
| — | −236 ± 125 | — |
| 1 | −126 ± 253 | NS[3] |
| 10 | −99 ± 230 | NS |
| 100 | −238 ± 133 | NS |
| 1,000 | 2,598 ± 284 | <0.001 |

1. The mean of 9 assays given in terms of ng/ml incubation medium ± standard error of the mean (SEM)
2. Given in terms of ng/ml incubation medium
3. NS denotes not significant
4. Comparison of the GH levels in medium containing growth hormone agonist analog to the GH levels in medium without the agonist

TABLE IX

In Vitro Growth Hormone Release

| Dosage[2] $H_2$-His-D-Trp-Ala-Trp-D-His-$NH_2$ | ΔGH[1] | p Value[4] |
|---|---|---|
| — | −236 ± 125 | — |
| 1 | −166 ± 277 | NS[3] |
| 10 | −369 ± 152 | NS |
| 100 | 43 ± 185 | NS |
| 1,000 | 1,501 ± 222 | <0.001 |

1. The mean of 9 assays given in terms of ng/ml incubation medium ± standard error of the mean (SEM)
2. Given in terms of ng/ml incubation medium
3. NS denotes not significant
4. Comparison of the GH levels in medium containing growth hormone agonist analog to the GH levels in medium without the agonist

TABLE X

In Vitro Growth Hormone Release

| Dosage[2] $H_2$-Tyr-D-Trp-Ala-Trp-D-p-Cl-Phe-$NH_2$ | ΔGH[1] | p Value[4] |
|---|---|---|
| — | 28 ± 54 | — |
| 10 | −87 ± 81 | NS[3] |
| 30 | 124 ± 123 | NS |
| 300 | 103 ± 77 | NS |
| 3,000 | 531 ± 42 | <0.001 |
| 30,000 | 489 ± 138 | <0.01 |

1. The mean of 9 assays given in terms of ng/ml incubation medium ± standard error of the mean (SEM)
2. Given in terms of ng/ml incubation medium
3. NS denotes not significant
4. Comparison of the GH levels in medium containing growth hormone agonist analog to the GH levels in medium without the agonist

TABLE XI

In Vitro Growth Hormone Release

| Dosage[2] $H_2$-Tyr-D-Trp-D-Ala-Trp-D-Phe-$NH_2$ | ΔGH[1] | p Value[4] |
|---|---|---|
| — | −257 ± 97 | — |
| 300 | 648 ± 210 | <0.02 |
| 3,000 | 435 ± 143 | <0.02 |
| 30,000 | 1,136 ± 190 | <0.001 |

1. The mean of 6 assays given in terms of ng/ml incubation medium ± standard error of the mean (SEM)
2. Given in terms of ng/ml incubation medium
3. Comparison of the GH levels in medium containing growth hormone agonist analog to the GH levels in medium without the agonist

TABLE XII

In Vitro Growth Hormone Release

| Dosage[2] $H_2$-p-Cl-Phe-D-Trp-Ala-Trp-D-Phe-$NH_2$ | ΔGH[1] | p Value[4] |
|---|---|---|
| — | −218 ± 161 | — |
| 30 | −260 ± 271 | NS[3] |
| 300 | 367 ± 159 | <0.05 |
| 1,000 | 714 ± 110 | <0.01 |
| 10,000 | 1,326 ± 143 | <0.001 |

1. The mean of 6 assays given in terms of ng/ml incubation medium ± standard error of the mean (SEM)
2. Given in terms of ng/ml incubation medium
3. NS denotes not significant
4. Comparison of the GH levels in medium containing growth hormone agonist analog to the GH levels in medium without the agonist

TABLE XIII

In Vitro Growth Hormone Release

| Dosage[2] $H_2$-desamino-Tyr-D-Trp-Ala-Trp-D-Phe-$NH_2$ | ΔGH[1] | p Value[3] |
|---|---|---|
| — | 28 ± 54 | — |
| 30 | 231 ± 65 | ~0.02 |
| 300 | 432 ± 109 | <0.01 |
| 3,000 | 700 ± 201 | <0.01 |
| 20,000 | −861 ± 13 | <0.001 |

1. The mean of 9 assays given in terms of ng/ml incubation medium ± standard error of the mean (SEM)
2. Given in terms of ng/ml incubation medium
3. Comparison of the GH levels in medium containing growth hormone agonist analog to the GH levels in medium without the agonist

TABLE XIV

In Vitro Growth Hormone Release

| Dosage[2] $CH_3CO$-Tyr-D-Trp-Ala-Trp-D-Phe-$NH_2$ | ΔGH[1] | p Value[4] |
|---|---|---|
| — | −175 ± 58 | — |
| 10 | −297 ± 79 | NS[3] |
| 30 | −118 ± 97 | NS |
| 300 | 5 ± 29 | ~0.02 |
| 3,000 | 1,594 ± 385 | <0.001 |
| 30,000 | 1,607 ± 250 | <0.001 |

1. The mean of 9 assays given in terms of ng/ml incubation medium ± standard error of the mean (SEM)
2. Given in terms of ng/ml incubation medium
3. NS denotes not significant
4. Comparison of the GH levels in medium containing growth hormone agonist analog to the GH levels in medium without the agonist

TABLE XV

In Vitro Growth Hormone Release

| Dosage[2] | ΔGH[1] | p Value[4] |
|---|---|---|
| $H_2$-O-Me-Tyr-D-Trp-Ala-Trp-D-Phe-$NH_2$ | | |
| — | −9 ± 66 | — |
| 300 | 177 ± 166 | NS[3] |
| 3,000 | 1,138 ± 266 | <0.001 |
| 30,000 | 892 ± 388 | <0.05 |

1. The mean of 9 assays given in terms of ng/ml incubation medium ± standard error of the mean (SEM)
2. Given in terms of ng/ml incubation medium
3. NS denotes not significant
4. Comparison of the GH levels in medium containing growth hormone agonist analog to the GH levels in medium without the agonist

TABLE XVI

In Vitro Growth Hormone Release

| Dosage[2] | ΔGH[1] | p Value[4] |
|---|---|---|
| $H_2$-Tyr-D-Trp-Ala-Trp-D-Phe-Met-$NH_2$ | | |
| — | −148 ± 137 | — |
| 30 | 456 ± 151 | NS[3] |
| 300 | 1,260 ± 245 | <0.01 |
| 1,000 | 1,832 ± 441 | <0.01 |

1. The mean of 6 assays given in terms of ng/ml incubation medium ± standard error of the mean (SEM)
2. Given in terms of ng/ml incubation medium
3. NS denotes not significant
4. Comparison of the GH levels in medium containing growth hormone agonist analog to the GH levels in medium without the agonist

TABLE XVII

In Vitro Growth Hormone Release

| Dosage[2] $H_2$-Tyr-D-Trp-Ala-Trp-D-Phe-Thr-$NH_2$ | $\Delta$GH[1] | p Value[4] |
|---|---|---|
| — | −40 ± 35 | — |
| 10 | 94 ± 69 | NS[3] |
| 100 | 650 ± 320 | NS |
| 1,000 | 2,167 ± 591 | <0.01 |
| 10,000 | 2,957 ± 834 | <0.01 |

1. The mean of 6 assays given in terms of ng/ml incubation medium ± standard error of the mean (SEM)
2. Given in terms of ng/ml incubation medium
3. NS denotes not significant
4. Comparison of the GH levels in medium containing growth hormone agonist analog to the GH levels in medium without the agonist

TABLE XVIII

In Vitro Growth Hormone Release

| Dosage[2] $H_2$-Tyr-D-Trp-Ala-Trp-D-Phe-Gln-OH | $\Delta$GH[1] | p Value[4] |
|---|---|---|
| — | −40 ± 35 | — |
| 100 | 753 ± 559 | NS[3] |
| 1,000 | 2,759 ± 515 | <0.001 |
| 10,000 | 3,994 ± 1,214 | <0.01 |

1. The mean of 6 assays given in terms of ng/ml incubation medium ± standard error of the mean (SEM)
2. Given in terms of ng/ml incubation medium
3. NS denotes not significant
4. Comparison of the GH levels in medium containing growth hormone agonist analog to the GH levels in medium without the agonist

TABLE XIX

In Vitro Growth Hormone Release

| Dosage[2] $H_2$-Tyr-D-Trp-Ala-Trp-D-Phe-Gln-$NH_2$ | ΔGH[1] | p Value[4] |
|---|---|---|
| — | −40 ± 35 | — |
| 10 | 240 ± 96 | NS[3] |
| 100 | 2,000 ± 523 | <0.01 |
| 1,000 | 4,235 ± 785 | <0.001 |
| 10,000 | 4,141 ± 576 | <0.001 |

1. The mean of 6 assays given in terms of ng/ml incubation medium ± standard error of the mean (SEM)
2. Given in terms of ng/ml incubation medium
3. NS denotes not significant
4. Comparison of the GH levels in medium containing growth hormone agonist analog to the GH levels in medium without the agonist

TABLE XX

In Vitro Growth Hormone Release

| Dosage[2] $H_2$-Tyr-D-Trp-Ala-Trp-D-Phe-Asn-$NH_2$ | ΔGH[1] | p Value[3] |
|---|---|---|
| — | −89 ± 51 | — |
| 30 | 244 ± 58 | <0.01 |
| 100 | 528 ± 184 | <0.01 |
| 1,000 | 1,670 ± 126 | <0.001 |

1. The mean of 6 assays given in terms of ng/ml incubation medium ± standard error of the mean (SEM)
2. Given in terms of ng/ml incubation medium
3. Comparison of the GH levels in medium containing growth hormone agonist analog to the GH levels in medium without the agonist

TABLE XXI

In Vitro Growth Hormone Release

| Dosage[2] $H_2$-His-D-Trp-Ala-Trp-D-Phe-Lys-$NH_2$ | ΔGH[1] | p Value[3] |
|---|---|---|
| — | −167 ± 114 | — |
| 1 | 955 ± 272 | <0.01 |
| 3 | 1,603 ± 305 | <0.001 |
| 10 | 2,244 ± 173 | <0.001 |
| 30 | 2,198 ± 358 | <0.001 |

1. The mean of 9 assays given in terms of ng/ml incubation medium ± standard error of the mean (SEM)
2. Given in terms of ng/ml incubation medium
3. Comparison of the GH levels in medium containing growth hormone agonist analog to the GH levels in medium without the agonist

TABLE XXII

In Vitro Growth Hormone Release

| Dosage[2] $H_2$-Tyr-D-Trp-Ala-Trp-D-Phe-Lys-$NH_2$ | ΔGH[1] | p Value[3] |
|---|---|---|
| — | −29 ± 59 | — |
| 30 | 1,346 ± 328 | <0.001 |
| 300 | 2,160 ± 166 | <0.001 |
| 3,000 | 1,630 ± 135 | <0.001 |
| 30,000 | 1,318 ± 247 | <0.001 |

1. The mean of 9 assays given in terms of ng/ml incubation medium ± standard error of the mean (SEM)
2. Given in terms of ng/ml incubation medium
3. Comparison of the GH levels in medium containing growth hormone agonist analog to the GH levels in medium without the agonist

TABLE XXIII

In Vitro Growth Hormone Release

| Dosage[2] $H_2$-Tyr-D-Trp-Ala-Trp-D-Phe-Glu-$NH_2$ | ΔGH[1] | p Value[3] |
|---|---|---|
| — | −29 ± 59 | — |
| 30 | 224 ± 98 | ~0.05 |
| 300 | 472 ± 104 | ~0.05 |
| 3,000 | 1,404 ± 179 | <0.001 |
| 30,000 | 1,909 ± 270 | <0.001 |

1. The mean of 9 assays given in terms of ng/ml incubation medium ± standard error of the mean (SEM)
2. Given in terms of ng/ml incubation medium
3. Comparison of the GH levels in medium containing growth hormone agonist analog to the GH levels in medium without the agonist

TABLE XXIV

In Vitro Growth Hormone Release

| Dosage[2] $H_2$-Tyr-D-Trp-Ala-Trp-D-Phe-Phe-$NH_2$ | ΔGH[1] | p Value[3] |
|---|---|---|
| — | 29 ± 164 | — |
| 30 | 10 ± 57 | NS[3] |
| 300 | 330 ± 43 | NS |
| 3,000 | 710 ± 67 | ~0.01 |
| 30,000 | 1,484 ± 135 | <0.001 |

1. The mean of 9 assays given in terms of ng/ml incubation medium ± standard error of the mean (SEM)
2. Given in terms of ng/ml incubation medium
3. NS denotes not significant
4. Comparison of the GH levels in medium containing growth hormone agonist analog to the GH levels in medium without the agonist

TABLE XXV

In Vitro Growth Hormone Release

| Dosage[2] | ΔGH[1] | p Value[4] |
|---|---|---|
| $H_2$-Tyr-D-Trp-Ala-Trp-D-Phe-Gln-$NH_2$ | | |
| — | −89 ± 50 | — |
| 30 | 103 ± 248 | NS[3] |
| 100 | 167 ± 65 | <0.02 |
| 1,000 | 1,092 ± 42 | <0.001 |

1. The mean of 6 assays given in terms of ng/ml incubation medium ± standard error of the mean (SEM)
2. Given in terms of ng/ml incubation medium
3. NS denotes not significant
4. Comparison of the GH levels in medium containing growth hormone agonist analog to the GH levels in medium without the agonist

TABLE XXVI

In Vitro Growth Hormone Release

| Dosage[2] | ΔGH[1] | p Value[3] |
|---|---|---|
| $H_2$-His-D-Trp-Ala-Trp-D-Phe-Lys-OH | | |
| — | −146 ± 67 | — |
| 1 | 467 ± 178 | <0.01 |
| 10 | 330 ± 167 | <0.02 |
| 100 | 2,441 ± 353 | <0.001 |

1. The mean of 9 assays given in terms of ng/ml incubation medium ± standard error of the mean (SEM)
2. Given in terms of ng/ml incubation medium
3. Comparison of the GH levels in medium containing growth hormone agonist analog to the GH levels in medium without the agonist

TABLE XXVII

In Vitro Growth Hormone Release

| Dosage[2] | ΔGH[1] | p Value[3] |
|---|---|---|
| $H_2$-His-D-Trp-Ala-Trp-D-Phe-Arg-$NH_2$ | | |
| — | −88 ± 56 | — |
| 1 | 805 ± 152 | <0.001 |
| 3 | 997 ± 249 | <0.001 |
| 10 | 2,064 ± 346 | <0.001 |

1. The mean of 9 assays given in terms of ng/ml incubation medium ± standard error of the mean (SEM)
2. Given in terms of ng/ml incubation medium
3. Comparison of the GH levels in medium containing growth hormone agonist analog to the GH levels in medium without the agonist

TABLE XXVIII

In Vitro Growth Hormone Release

| Dosage[2] | ΔGH[1] | p Value[4] |
|---|---|---|
| $H_2$-His-D-Trp-Ala-Trp-D-Phe-Gln-$NH_2$ | | |
| — | −288 ± 182 | — |
| 1 | 5 ± 136 | NS[3] |
| 3 | 140 ± 168 | NS |
| 10 | 275 ± 66 | ~0.02 |

1. The mean of 6 assays given in terms of ng/ml incubation medium ± standard error of the mean (SEM)
2. Given in terms of ng/ml incubation medium
3. NS denotes not significant
4. Comparison of the GH levels in medium containing growth hormone agonist analog to the GH levels in medium without the agonist

TABLE XXIX

In Vitro Growth Hormone Release

| Dosage[2] | ΔGH[1] | p Value[4] |
|---|---|---|
| $H_2$-His-D-Trp-Ala-Trp-D-Phe-Glu-$NH_2$ | | |
| — | −251 ± 105 | — |
| 1 | −78 ± 72 | NS[3] |
| 3 | −294 ± 75 | NS |
| 10 | −243 ± 71 | NS |
| 30 | 117 ± 41 | <0.01 |

1. The mean of 9 assays given in terms of ng/ml incubation medium ± standard error of the mean (SEM)
2. Given in terms of ng/ml incubation medium
3. NS denotes not significant
4. Comparison of the GH levels in medium containing growth hormone agonist analog to the GH levels in medium without the agonist

TABLE XXX

In Vitro Growth Hormone Release

| Dosage[2] | ΔGH[1] | p Value[3] |
|---|---|---|
| $H_2$-His-D-Trp-Ala-Trp-D-Phe-HomoArg-$NH_2$ | | |
| — | −553 ± 105 | — |
| 1 | −150 ± 124 | ~0.02 |
| 10 | 1,096 ± 341 | <0.001 |

1. The mean of 6 assays given in terms of ng/ml incubation medium ± standard error of the mean (SEM)
2. Given in terms of ng/ml incubation medium
3. Comparison of the GH levels in medium containing growth hormone agonist analog to the GH levels in medium without the agonist

TABLE XXXI

In Vitro Growth Hormone Release

| Dosage[2] $H_2$-3-N-Me-D-Trp-Ala-Trp-D-Phe-Lys-$NH_2$ | ΔGH[1] | p Value[4] |
|---|---|---|
| — | 435 ± 176 | — |
| 1 | 165 ± 136 | NS[3] |
| 10 | 1,857 ± 254 | <0.01 |

1. The mean of 9 assays given in terms of ng/ml incubation medium ± standard error of the mean (SEM)
2. Given in terms of ng/ml incubation medium
3. NS denotes not significant
4. Comparison of the GH levels in medium containing growth hormone agonist analog to the GH levels in medium without the agonist

TABLE XXXII

In Vitro Growth Hormone Release

| Dosage[2] $H_2$-His-D-Trp-Ala-Trp-D-Phe-Lys-$NHCH_2CH_3$ | ΔGH[1] | p Value[4] |
|---|---|---|
| — | 435 ± 176 | — |
| 1 | 726 ± 115 | NS[3] |
| 10 | 1,688 ± 300 | <0.01 |

1. The mean of 9 assays given in terms of ng/ml incubation medium ± standard error of the mean (SEM)
2. Given in terms of ng/ml incubation medium
3. NS denotes not significant
4. Comparison of the GH levels in medium containing growth hormone agonist analog to the GH levels in medium without the agonist

TABLE XXXIII

In Vitro Growth Hormone Release

| Dosage[2] $H_2$-His-D-Trp-Ala-Trp-D-Phe-Orn-$NH_2$ | ΔGH[1] | p Value[4] |
|---|---|---|
| — | −553 ± 105 | — |
| 1 | −295 ± 93 | NS[3] |
| 10 | 356 ± 92 | <0.001 |

1. The mean of 6 assays given in terms of ng/ml incubation medium ± standard error of the mean (SEM)
2. Given in terms of ng/ml incubation medium
3. NS denotes not significant
4. Comparison of the GH levels in medium containing growth hormone agonist analog to the GH levels in medium without the agonist

TABLE XXXIV

In Vitro Growth Hormone Release

| Dosage[2] | ΔGH[1] | p Value[3] |
|---|---|---|
| $H_2$-His-D-Trp-Val-Trp-D-Phe-Lys-$NH_2$ | | |
| — | −553 ± 105 | — |
| 30 | −118 ± 65 | <0.01 |

1. The mean of 6 assays given in terms of ng/ml incubation medium ± standard error of the mean (SEM)
2. Given in terms of ng/ml incubation medium
3. Comparison of the GH levels in medium containing growth hormone agonist analog to the GH levels in medium without the agonist

TABLE XXXV

In Vitro Growth Hormone Release

| Dosage[2] | ΔGH[1] | p Value[4] |
|---|---|---|
| $H_2$-His-D-Trp-Ser-Trp-D-Phe-Lys-$NH_2$ | | |
| — | −125 ± 132 | — |
| 10 | 161 ± 157 | NS[3] |
| 30 | 705 ± 149 | <0.01 |

1. The mean of 6 assays given in terms of ng/ml incubation medium ± standard error of the mean (SEM)
2. Given in terms of ng/ml incubation medium
3. NS denotes not significant
4. Comparison of the GH levels in medium containing growth hormone agonist analog to the GH levels in medium without the agonist

The results set forth in Tables VI—XXXV demonstrate that peptides within the scope of the instant invention can induce a significant *in vitro* release of growth hormone from the pituitary.

By introducing various other hormones, e.g., somatostatin, testosterone, cortisol, insulin, etc., into the incubation medium of Examples 11—30, one can study what effect these latter hormones have on the regulation of growth hormone secretion.

Example 61

In Vivo Growth Hormone Release Study

Female rats of the CD—1 strain were housed in a constant temperature room at 24°C. with 14 hours light and 10 hours darkness. The rats were fed Purina brand rat chow ab libitum. All studies were started between 0800 and 1000 hours.

Each female rat (21 days old; eight rats per dosage level shown in Table XVI) was intraperitoneally injected with a desired dosage of the peptide of Example 1. Approximately 15 minutes after injection, the rat was guillotined. A blood sample was collected from the guillotined rat. The blood sample was centrifuged and a serum sample was collected therefrom. Each serum sample was assayed for GH, in duplicate, by a standard radio-immunoassay (RIA). The mean of the GH values obtained per dosage level are set forth in Table XXXVI.

Example 62

In Vivo Growth Hormone Release Study

The procedure set forth in Example 61 was employed in an *in vivo* growth hormone release study of the peptide of Example 21 and the results therefrom are set forth in Table XXXVII.

Example 63

In Vivo Growth Hormone Release Study

The procedure set forth in Example 61 was employed in an *in vivo* growth hormone release study of the peptide of Example 22 and the results therefrom are set forth in Table XXXVIII.

Example 64

In Vivo Growth Hormone Release Study

The procedure set forth in Example 61 was employed in an *in vivo* growth hormone release study of the peptide of Example 23 and the results therefrom are set forth in Table XXXIX.

Example 65

In Vivo Growth Hormone Release Study

The procedure set forth in Example 61 was employed in an *in vivo* growth hormone release study of the peptide of Example 24 and the results therefrom are set forth in Table XXXX.

Example 66

In Vivo Growth Hormone Release Study

The procedure set forth in Example 61 was employed in an *in vivo* growth hormone release study of the peptide of Example 25 and the results therefrom are set forth in Table XXXXI.

Example 67

In Vivo Growth Hormone Release Study

The procedure set forth in Example 61 was employed in an *in vivo* growth hormone release study of the peptide of Example 26 and the results therefrom are set forth in Table XXXXII.

Example 68

In Vivo Growth Hormone Release Study

The procedure set forth in Example 61 was employed in an *in vivo* growth hormone release study of the peptide of Example 27 and the results therefrom are set forth in Table XXXXIII.

Example 69

In Vivo Growth Hormone Release Study

The procedure set forth in Example 61 was employed in an *in vivo* growth hormone release study of the peptide of Example 16 and the results therefrom are set forth in Table XXXXIV.

TABLE XXXVI

In Vivo Growth Hormone Release

| Dosage[2] | ΔGH[1] | p Value[4] |
|---|---|---|
| $H_2$-His-D-Trp-Ala-Trp-D-Phe-$NH_2$ | | |
| Control | 4 ± 1 | — |
| 0.1 | 1 ± 1 | <0.05 |
| 1.0 | 2 ± 2 | NS[3] |
| 10.0 | 2 ± 1 | NS |
| 100.0 | 82 ± 18 | <0.001 |

1. The mean of 8 assays given in terms of ng/ml intraperitoneal ± standard error of the mean (SEM)
2. Given in terms of μg/ml serum
3. NS denotes not significant
4. GH levels in serum of rats intraperitonally injected with peptide compared to the GH levels in serum of control rats

TABLE XXXVII

In Vivo Growth Hormone Release

| Dosage[2] | ΔGH[1] | p Value[3] |
|---|---|---|
| $H_2$-His-D-Trp-Ala-Trp-D-Phe-Lys-OH | | |
| Control | 1.7 ± 1 | — |
| 3.0 | 76 ± 24 | <0.01 |
| 10.0 | 119 ± 25 | <0.001 |
| 30.0 | 226 ± 43 | <0.001 |

1. The mean of 8 assays given in terms of ng/ml subcutaneous medium ± standard error of the mean (SEM)
2. Given in terms of μg/ml serum
3. GH levels in serum of rats subcutaneously injected with peptide compared to the GH levels in serum of control rats

TABLE XXXVIII

In Vivo Growth Hormone Release

| Dosage[2] | ΔGH[1] | p Value[4] |
|---|---|---|
| $H_2$-His-D-Trp-Ala-Trp-D-Phe-Arg-$NH_2$ | | |
| Control | 5.7 ± 2.2 | — |
| 1.0 | 9.2 ± 3.4 | NS[3] |
| 3.0 | 32.2 ± 5.5 | <0.001 |
| 10.0 | 87.5 ± 31 | 0.02 |
| 30.0 | 31.1 ± 3.4 | <0.001 |

1. The mean of 8 assays given in terms of ng/ml subcutaneous ± standard error of the mean (SEM)
2. Given in terms of μg/ml serum
3. NS denotes not significant
4. GH levels in serum of rats subcutaneously injected with peptide compared to the GH levels in serum of control rats

TABLE XXXIX

In Vivo Growth Hormone Release

| Dosage[2] | ΔGH[1] | p Value[4] |
|---|---|---|
| $H_2$-His-D-Trp-Ala-Trp-D-Phe-Gln-$NH_2$ | | |
| Control | 4.9 ± 0.9 | — |
| 1.0 | 1.4 ± 0.9 | NS[3] |
| 3.0 | 3.8 ± 2.1 | NS |
| 10.0 | 32.1 ± 11.4 | <0.05 |

1. The mean of 9 assays given in terms of ng/ml subcutaneous ± standard error of the mean (SEM)
2. Given in terms of µg/ml serum
3. NS denotes not significant
4. GH levels in serum of rats subcutaneously injected with peptide compared to the GH levels in serum of control rats

TABLE XXXX

In Vivo Growth Hormone Release

| Dosage[2] | ΔGH[1] | p Value[4] |
|---|---|---|
| $H_2$-His-D-Trp-Ala-Trp-D-Phe-Glu-$NH_2$ | | |
| Control | 0.34 ± 0.19 | — |
| 1.0 | 0.10 ± 0 | NS[3] |
| 3.0 | 1.50 ± 0.56 | NS |
| 10.0 | 1.4 ± 0.78 | NS |
| 30.0 | 8.06 ± 2.68 | ~0.01 |

1. The assays given in terms of ng/ml subcutaneous ± standard error of the mean (SEM)
2. Given in terms of µg/ml serum
3. NS denotes not significant
4. GH levels in serum of rats subcutaneously injected with peptide compared to the GH levels in serum of control rats

TABLE XXXXI

In Vivo Growth Hormone Release

| Dosage[2] | ΔGH[1] | p Value[4] |
|---|---|---|
| $H_2$-His-D-Trp-Ala-Trp-D-Phe-HomoArg-$NH_2$ | | |
| Control | 0 ± 0 | — |
| 0.3 | 3 ± 2 | NS[3] |
| 10.0 | 62 ± 8 | <0.001 |

1. The mean of 6 assays given in terms of ng/ml subcutaneous ± standard error of the mean (SEM)
2. Given in terms of µg/ml serum
3. NS denotes not significant
4. GH levels in serum of rats subcutaneously injected with peptide compared to the GH levels in serum of control rats

TABLE XXXXII
In Vivo Growth Hormone Release

| Dosage[2] | ΔGH[1] | p Value[3] |
|---|---|---|
| $H_2$-3-N-Me-His-D-Trp-Ala-Trp-D-Phe-Lys-$NH_2$ | | |
| Control | 1.1 ± 0.6 | — |
| 0.3 | 45.0 ± 10 | ~0.001 |
| 10.0 | 128 ± 16 | <0.001 |

1. The mean of 6 assays given in terms of ng/ml subcutaneous ± standard error of the mean (SEM)
2. Given in terms of µg/ml serum
3. GH levels in serum of rats subcutaneously injected with peptide compared to the GH levels in serum of control rats

TABLE XXXXIII
In Vivo Growth Hormone Release

| Dosage[2] | ΔGH[1] | p Value[3] |
|---|---|---|
| $H_2$-His-D-Trp-Ala-Trp-D-Phe-Lys-$NHCH_2CH_3$ | | |
| Control | 1.1 ± 0.6 | — |
| 0.3 | 13.0 ± 5 | <0.05 |
| 10.0 | 119 ± 12 | <0.001 |

1. The mean of 6 assays given in terms of ng/ml subcutaneous ± standard error of the mean (SEM)
2. Given in terms of µg/ml serum
3. GH levels in serum of rats subcutaneously injected with peptide compared to the GH levels in serum of control rats

TABLE XXXXIV
In Vivo Growth Hormone Release

| Dosage[2] | ΔGH[1] | p Value[4] |
|---|---|---|
| $H_2$-His-D-Trp-Ala-Trp-D-Phe-Lys-$NH_2$ | | |
| Control | 0 ± 0 | — |
| 0.3 | 6 ± 4 | NS[3] |
| 1.0 | 36 ± 15 | <0.04 |
| 3.0 | 66 ± 15 | ~0.001 |
| 10.0 | 100 ± 19 | <0.001 |
| 30.0 | 122 ± 4 | <0.001 |

1. The mean of 6 assays given in terms of ng/ml subcutaneous ± standard error of the mean (SEM)
2. Given in terms of µg/ml serum
3. NS denotes not significant
4. GH levels in serum of rats subcutaneously injected with peptide compared to the GH levels in serum of control rats

The results set forth in Tables XXVI—XXXXIV demonstrate that some peptides within the scope of this invention can induce a significant *in vivo* release of growth hormone.

Example 70

In Vitro Growth Hormone Release Study

The procedure set forth in Example 31 was employed in an *in vitro* growth hormone release study of the growth promoting agent zeranol, $C_{18}H_{26}O_5$, and the results therefrom are set forth in Table XXXXV.

Example 71

In Vitro Growth Hormone Release Study

The procedure set forth in Example 31 was employed in an *in vitro* growth hormone release study of the peptide of Example 21 in combination with the growth promoting agent zeranol, $C_{18}H_{26}O_5$, and the results therefrom are set forth in Table XXXXVI. The results set forth in Table XXXXVI, when compared to the results set forth in Tables XXVI and XXXXV, demonstrate that a combination within the scope of the instant invention can induce a synergistic *in vitro* release of growth hormone from the pituitary.

TABLE XXXXV
In Vitro Growth Hormone Release

| Dosage[2] Zeranol, $C_{18}H_{26}O_5$ | $\Delta GH^1$ | p Value[3] |
|---|---|---|
| — | $-115 \pm 49$ | — |
| 300 | $83 \pm 63$ | 0.02 |
| 3,000 | $87 \pm 59$ | <0.02 |
| 30,000 | $683 \pm 187$ | <0.001 |
| 100,000 | $-133 \pm 98$ | NS[3] |

1. The mean of 9 assays given in terms of ng/ml incubation medium $\pm$ standard error of the mean (SEM)
2. Given in terms of ng/ml incubation medium
3. NS denotes not significant
4. Comparison of the GH levels in medium containing growth hormone agonist analog to the GH levels in medium without the agonist

TABLE XXXXVI
In Vitro Growth Hormone Release

| Dosage[2] Zeranol, $C_{18}H_{26}O_5$ (A) + $H_2$-His-D-Trp-Ala-Trp-D-Phe-Lys-$NH_2$ | $\Delta GH^1$ | p Value[3] |
|---|---|---|
| — | $-309 \pm 39$ | — |
| 3,000 A + 1B | $2,183 \pm 399$ | <0.001 |
| 3,000 A + 3B | $2,114 \pm 336$ | <0.001 |
| 30,000 A + 1B | $2,266 \pm 327$ | <0.001 |
| 30,000 A + 3B | $2,761 \pm 356$ | <0.001 |

1. The mean of 9 assays given in terms of ng/ml incubation medium $\pm$ standard error of the mean (SEM)
2. Given in terms of ng/ml incubation medium
3. Comparison of the GH levels in medium containing growth hormone agonist analog to the GH levels in medium without the agonist

## Claims

1. A peptide having a formula selected from a group consisting of

$$(X_2)_a-\underset{\displaystyle (X_3)_b}{\underset{|}{\overset{\displaystyle X_1}{\overset{|}{A_1}}}}-A_2-A_3-A_4-A_5-Y \qquad (I)$$

$$(X_2')_a-\underset{\displaystyle (X_3')_b}{\underset{|}{\overset{\displaystyle X_1'}{\overset{|}{A_1}}}}-A_2-A_3-A_4-A_5-A_6-Y \qquad (II)$$

wherein

$X_1$, $X_2$, $X_3$, $X_1'$, $X_2'$, and $X_3'$ are selected from a group consisting of N-terminal and desamino alpha-carbon substitutions;

a and b are 0 or 1, provided that a and b are 0 when $A_1$ is a desamino residue;

$A_1$ and $A_4$ are selected from a group consisting of His, Arg, Lys, α-Naphth, β-Naphth, Iql, Tyr, Trp, Phe, homologues and analogues thereof, and, with respect to $A_1$, the desamino forms thereof;

$A_2$ and $A_5$ are selected from a group consisting of D-His, D-Arg, D-Lys, D-α-Naphth, D-β-Naphth, D-Iql, D-Tyr, D-Trp, D-Phe, homologues and analogues thereof, and, with respect to $A_5$, the descarboxy forms thereof;

$A_3$ is selected from a group consisting of Gly, Ala, Val, Leu, Ile, Pro, Ser, Thr, Met, Asp, Glu, Asn, Gln, His, D-Ala, D-Val, D-Leu, D-Ile, D-Pro, D-Ser, D-Thr, D-Met, D-Asp, D-Glu, D-Asn, D-Gln, D-His, and homologues and analogues thereof;

$A_6$ is selected from a group consisting of amino acids of the L- and D- configuration, homologues and analogues thereof, and the descarboxy forms thereof; and

Y is selected from a group consisting of C-terminal and descarboxy alpha-carbon substitutions;

and the pharmaceutically acceptable salts thereof;

provided that, (a) when (1) a is 1 and b is 0 and $X_1$ and $X_2$ are selected from the group consisting of $-H$ or $-CH_3$; (2) $A_1$ and $A_4$ are selected from the group consisting of Tyr, Trp, and Phe; (3) $A_3$ is selected from the group consisting of Gly, Ala, Val, Leu, Ile, Pro, Ser, Thr, Met, Asp, Glu, Asn, Gln, and His; and (4) Y is selected from the group consisting of $-NR_1R_2$, $-CH_2OR$, and $-OR$, wherein R, $R_1$, and $R_2$ are selected from a group consisting of hydrogen and straight and branched chain alkyl groups containing 1—6 carbon atoms; then at least one of $A_2$ and $A_5$ is selected such that it is not from the group consisting of D-Tyr, D-Trp, D-Phe, and, with respect to $A_5$, the descarboxy forms thereof; (b) when (1) a is 1 and b is 0 and $X_1$ and $X_2$ are selected from the group consisting of $-H$ and $-CH_3$; (2) $A_2$ and $A_5$ are selected from the group consisting of D-Tyr, D-Trp, D-Phe, and, with respect to $A_5$, the descarboxy forms thereof; (3) $A_3$ is selected from the group consisting of Gly, Ala, Val, Leu, Ile, Pro, Ser, Thr, Met, Asp, Glu, Asn, Gln, and His; and (4) Y is selected from the group consisting of $-NR_1R_2$, $-CH_2OR$, and $-OR$, wherein R, $R_1$, $R_2$ are selected from a group consisting of hydrogen and straight and branched chain alkyl groups containing 1—6 carbon atoms; then at least one of $A_1$ and $A_4$ is selected such that it is not from a group consisting of Tyr, Trp, and Phe; (c) (1) when a is 1 and b is 0 and $X_1'$ and $X_2'$ are selected from the group consisting of $-H$, $-CH_3$, and $-CHOCH_3$; (2) $A_1$ and $A_4$ are selected from the group consisting of Tyr, Trp, and Phe; (3) $A_3$ is selected from the group consisting of Gly, Ala, Val, Leu, Ile, Ser, Thr, Met, Asn, and Gln; (4) $A_6$ is selected from the group consisting of Asn, Gln, Glu, Arg, Lys, Ser, Thr, and the descarboxy forms thereof; and (5) Y is selected from the group consisting of $-NR_1R_2$, $-CH_2OR$, and $-OR$, wherein R, $R_1$, and $R_2$ are selected from a group consisting of hydrogen and straight and branched chain alkyl groups containing 1—6 carbon atoms; then at least one of $A_2$ and $A_5$ is selected such that it is not from the group consisting of D-Tyr, D-Trp, and D-Phe; and (d) when (1) a is 1 and b is 0 and $X_1'$ and $X_2'$ are selected from the group consisting of $-H$, $-CH_3$ and $-CHOCH_3$; (2) $A_2$ and $A_5$ are selected from the group consisting of D-Tyr, D-Trp, and D-Phe; (3) $A_3$ is selected from the group consisting of Gly, Ala, Val, Leu, Ile, Ser, Thr, Met, Asn, and Gln; (4) $A_6$ is selected from the group consisting of Asn, Gln, Glu, Arg, Lys, Ser, Thr, and the descarboxy forms thereof; and (5) Y is selected from the group consisting of $-NR_1R_2$, $-CH_2OR$, and $-OR$, wherein R, $R_1$, $R_2$ are selected from a group consisting of hydrogen and straight and branched chain alkyl groups containing 1—6 carbon atoms; then at least one of $A_1$ and $A_4$ is selected such that it is not from a group consisting of Tyr, Trp, and Phe.

2. The peptide of claim 1 wherein:

a is 0 or 1 and b is 0;

$X_1$, $X_2$, $X_1'$ and $X_2'$ are selected from a group consisting of $-R$, $-OR$, and $RC(O)-$, wherein R is selected from a group consisting of hydrogen and straight and branched chain alkyl group containing 1—6 carbon atoms;

$A_1$ and $A_4$ are selected from the group consisting of His, Tyr, Trp, Phe, homologues and analogues thereof, and, with respect to $A_1$, the desamino forms thereof;

$A_2$ and $A_5$ are selected from the group consisting of D-His, D-Tyr, D-Phe, homologues and analogues thereof, and, with respect to $A_5$, the descarboxy forms thereof;

$A_3$ is selected from the group consisting of Gly, Ala, Ser, Asn, Pro, D-Ala, D-Ser, D-Asn, D-Pro, and homologues and analogues thereof;

$A_6$ is selected from the group consisting of Arg, Lys, Orn, His, Asp, Glu, Asn, Gln, D-Arg, D-Lys, D-Orn, D-His, D-Asp, D-Glu, D-Asn, D-Gln, D-Arg, homologues and analogues thereof, and descarboxy forms thereof;

Y is selected from the group consisting of —$CH_2OH$, —OR, and —$NR_1R_2$, wherein R, $R_1$ and $R_2$ are selected from the group consisting of hydrogen and straight or branched chain alkyl group containing 1—6 carbon atoms;

and the pharmaceutically acceptable salts thereof.

3. The peptide of claim 1 having the formula selected from the group consisting of

$$\overset{\displaystyle H}{\overset{|}{(X_2)_a\text{—}A_1}}\text{—D-Trp-}A_3\text{—}A_4\text{—}A_5\text{—Y} \qquad (IV)$$

$$\overset{\displaystyle H}{\overset{|}{(X_2')_a\text{—}A_1}}\text{—D-Trp-}A_3\text{—}A_4\text{—}A_5\text{—}A_6\text{—Y} \qquad (V)$$

wherein

a is 0 or 1;

$X_2$ and $X_2'$ are selected from the group consisting of RCO and R— wherein R is selected from the group consisting of hydrogen and alkyl groups containing 1—2 carbon atoms;

$A_1$ is selected from the group consisting of Tyr, O-Me-Tyr, His, 3-N-Me-His, p-Cl-Phe, and the desamino forms thereof;

$A_3$ is selected from the group consisting of Ala, Ser, and D-Ala;

$A_4$ is selected from the group consisting of Trp and Tyr;

$A_5$ is selected from the group consisting of D-Phe, D-His, D-Tyr, and D-p-Cl-Phe;

$A_6$ is selected from the group consisting of Arg, HomoArg, Lys, Orn, Asp, Glu, Asn, Gln, and D-Lys;

Y is selected from the group consisting of —OR, and —NHR, wherein R is selected from the group consisting of hydrogen and alkyl groups containing 1—2 carbon atoms;

and the pharmaceutically acceptable salts thereof.

4. The peptide of claim 1 of the formula selected from a group consisting of

$H_2$-His-D-Trp-Ala-Trp-D-Phe-$NH_2$,
$H_2$-His-D-Trp-Ala-Trp-D-His-$NH_2$,
$H_2$-His-D-Trp-Ala-Trp-D-His-$NH_2$,
$H_2$-His-D-Trp-Ala-Trp-D-Tyr-$NH_2$,
$H_2$-Tyr-D-Trp-Ala-Trp-D-p-Cl-Phe-$NH_2$,
$H_2$-p-Cl-Phe-D-Trp-Ala-Trp-D-Phe-$NH_2$,
$H_2$-desaminoTyr-D-Trp-Ala-Trp-D-Phe-$NH_2$,

$$\overset{\displaystyle H}{\overset{|}{CH_3CO\text{—Tyr}}}\text{-D-Trp-Ala-Trp-D-Phe-}NH_2,$$

$H_2$-O-Me-Tyr-D-Trp-Ala-Trp-D-Phe-$NH_2$,
$H_2$-Tyr-D-Trp-Ala-Trp-D-Phe-Met-$NH_2$,
$H_2$-Tyr-D-Trp-Ala-Trp-D-Phe-Thr-$NH_2$,
$H_2$-Tyr-D-Trp-Ala-Trp-D-Phe-Gln-OH,
$H_2$-Tyr-D-Trp-Ala-Trp-D-Phe-Gln-$NH_2$,
$H_2$-Tyr-D-Trp-Ala-Trp-D-Phe-Asn-$NH_2$,
$H_2$-His-D-Trp-Ala-Trp-D-Phe-Lys-$NH_2$,
$H_2$-Tyr-D-Trp-Ala-Trp-D-Phe-Lys-$NH_2$,
$H_2$-Tyr-D-Trp-Ala-Trp-D-Phe-Glu-$NH_2$,
$H_2$-Tyr-D-Trp-Ala-Trp-D-Phe-Phe-$NH_2$,
$H_2$-Tyr-D-Trp-Ala-Trp-D-Phe-Gln-$NH_2$,
$H_2$-His-D-Trp-Ala-Trp-D-Phe-Lys-OH,
$H_2$-His-D-Trp-Ala-Trp-D-Phe-Arg-$NH_2$,
$H_2$-His-D-Trp-Ala-Trp-D-Phe-Gln-$NH_2$,
$H_2$-His-D-Trp-Ala-Trp-D-Phe-Glu-$NH_2$,
$H_2$-His-D-Trp-Ala-Trp-D-Phe-HomoArg-$NH_2$,
$H_2$-3-N-Me-His-D-Trp-Ala-Trp-D-Phe-Lys-$NH_2$,
$H_2$-His-D-Trp-Ala-Trp-D-Phe-Lys-$NHCH_2CH_3$,

$H_2$-His-D-Trp-Ala-Trp-D-Phe-Orn-$NH_2$,
$H_2$-His-D-Trp-Val-Trp-D-Phe-Lys-$NH_2$, and
$H_2$-His-D-Trp-Ser-Trp-D-Phe-Lys-$NH_2$.

5. A combination comprising:
(a) at least one growth promoting agent; and
(b) at least one peptide of any one of claims 1—3 or 4.

6. The combination of claim 5 wherein said peptide has the formula

$H_2$-His-D-Trp-Ala-Trp-D-Phe-Lys-$NH_2$

and wherein said growth promoting agent is zeranol, $C_{18}H_{26}O_5$.

7. A peptide according to any one of the preceding claims for use in regulating the release of growth hormone from a pituitary gland.

8. A peptide according to any one of the preceding claims for use in regulating the release of growth hormone *in vivo* from a pituitary gland.

9. An intermediate composition having a formula selected from a group consisting of

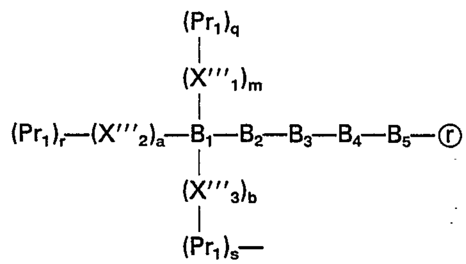

(IV)

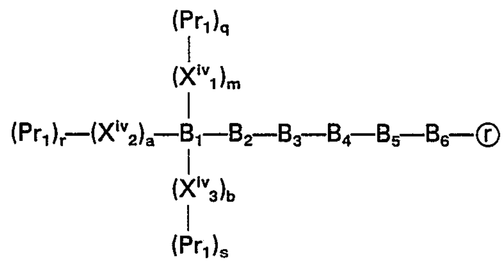

(VII)

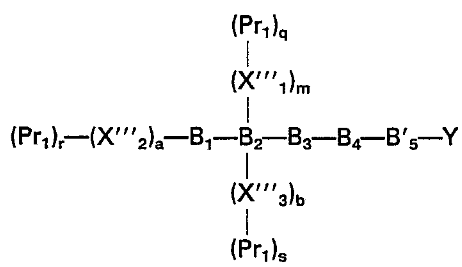

(VIII)

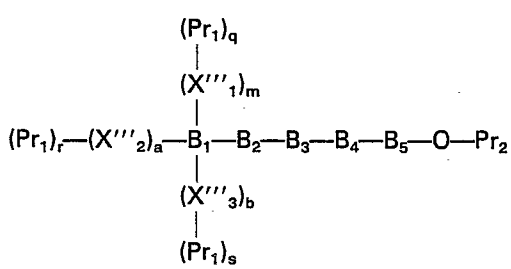

(IX)

$B_1—B_2—B_3—B_4—B'_5—Y$ (X)

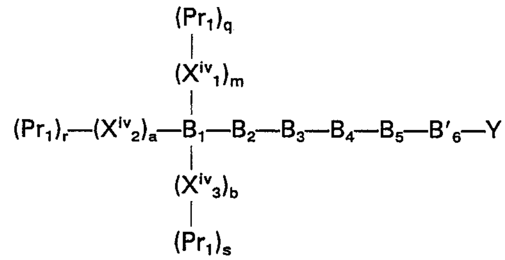

(XI)

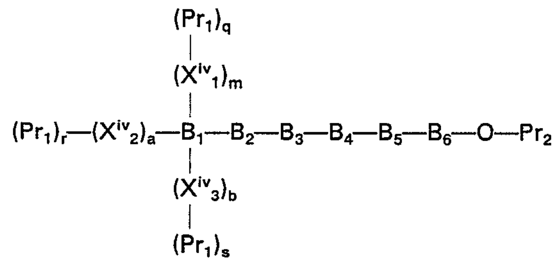

(XII)

$$B_1—B_2—B_3—B_4—B_5—B'_6—Y \quad (XIII)$$

wherein

$Pr_1$ is an α-amino acid protecting group;

a, b, m, q, r, and s are each 0 or 1;

$X'''_1$, $X'''_2$, $X'''_3$, $X^{iv}_2$ and $X^{iv}_3$ are selected from a group consisting of N-terminus and desamino alpha-carbon substitutions and radicals;

$B_1$ and $B_4$ are selected from a group consisting of His, Arg, Lys, α-Naphth, β-Naphth, Iql, Tyr, Trp, Phe, homologues and analogues thereof, the side-chain protected forms thereof, and, with respect to $B_1$, the desamino forms thereof;

$B_2$, $B_5$, and $B'_5$ are selected from a group consisting of D-His, D-Arg, D-Lys, D-α-Naphth, D-β-Naphth, D-Iql, D-Tyr, D-Trp, D-Phe, homologues and analogues thereof, the side-chain protected forms thereof, and, with respect to $B'_5$, the descarboxy forms thereof;

$B_3$ is selected from a group consisting of Gly, Ala, Val, Leu, Ile, Pro, Ser, Thr, Met, Asp, Glu, Asn, Gln, His, D-Ala, D-Val, D-Leu, D-Ile, D-Pro, D-Ser, D-Thr, D-Met, D-Asp, D-Glu, D-Asn, D-Gln, D-Arg, D-His, homologues and analogues thereof, and the side-chain protected forms thereof;

$B_6$ and $B'_6$ are selected from a group consisting of amino acids of the L- and D-configurations, homologues and analogues thereof, the side chain protected forms thereof, and, with respect to $B'_6$, the descarboxy forms thereof;

Ⓡ is a resin;

Y is selected from a group consisting of C-terminal and descarboxy alpha-carbon substitutions; and

$Pr_2$ is a carboxyl protecting group;

provided that, when (1) a is 1 and b and m are 0 and $X'''_2$ is selected from the group consisting of —H and —$CH_3$; (2) $B_1$ and $B_4$ are selected from the group consisting of Tyr, Trp, Phe, and the side-chain protected forms thereof; (3) $B_3$ is selected from the group consisting of Gly, Ala, Val, Leu, Ile, Pro, Ser, Thr, Met, Asp, Glu, Asn, Gln, His, and the side-chain protected forms thereof; and, with respect to XI and XIII, (4) Y is selected from the group consisting of —$NR_1R_2$, —OR, and —$CH_2OR$, wherein each R, $R_1$, and $R_2$ is selected from a group consisting of hydrogen and straight and branched chain alkyl groups containing 1—6 carbon atoms; then at least one of $B_2$, $B_5$, or $B'_5$ is selected such that it is not from a group consisting of D-Tyr, D-Trp, D-Phe, and, with respect to $B'_5$, the descarboxy forms thereof, and the side-chain protected forms thereof;

when (1) a is 1 and b and m are 0 and $X'''_2$ is selected from the group consisting of —H and —$CH_3$; (2) $B_2$ and $B_5$ or $B'_5$ are selected from the group consisting of D-Tyr, D-Trp, D-Phe, and, with respect to $B'_5$, the descarboxy forms thereof, and the side chain protected forms thereof; (3) $B_3$ is selected from the group consisting of Gly, Ala, Val, Leu, Ile, Pro, Ser, Thr, Met, Asp, Glu, Asn, Gln, His and the side-chain protected forms thereof; and, with respect to XI and XIII, (4) Y is selected from the group consisting of —$NR_1R_2$, —OR, and —$CH_2OR$, wherein each R, $R_1$, and $R_2$ is selected from a group consisting of hydrogen and straight and branched chain alkyl groups containing 1—6 carbon atoms; then at least one of $B_1$ and $B_4$ is selected such that it is not from a group consisting of Tyr, Trp, Phe, and the side-chain protected forms thereof;

when (1) a is 1 and b and m are 0 and $X^{iv}_2$ is selected from the group consisting of —H, —$CH_3$, and —$CHOCH_3$; (2) $B_1$ and $B_4$ are selected from the group consisting of Tyr, Trp, Phe, and the side-chain protected forms thereof; (3) $B_3$ is selected from the group consisting of Gly, Ala, Val, Leu, Ile, Ser, Thr, Met, Asn, Gln, and the side-chain protected forms thereof; (4) $B_6$ or $B'_6$ is selected from the group consisting of Asn, Gln, Glu, Arg, Lys, Ser, Thr, and, with respect to $B'_6$, the descarboxy forms thereof, and the side-chain protected forms thereof; and, with respect to XI and XIII, (5) Y is selected from the group consisting of —$NR_1R_2$, —OR, and —$CH_2OR$, wherein each R, $R_1$, and $R_2$ is selected from a group consisting of hydrogen

and straight and branched chain alkyl groups containing 1—6 carbon atoms; then at least one of $B_2$ or $B_5$ is selected such that it is not from a group consisting of D-Tyr, D-Trp, D-Phe, and the side-chain protected forms thereof; and

when (1) a is 1 and b and m are 0 and $X^{iv}_2$ is selected from the group consisting of —H, —$CH_3$, and —$CHOCH_3$; (2) $B_2$ and $B_5$ are selected from the group consisting of D-Tyr, D-Trp, D-Phe, and the side-chain protected forms thereof; (3) $B_3$ is selected from the group consisting of Gly, Ala, Val, Leu, Ile, Ser, Thr, Met, Asn, Gln, and the side-chain protected forms thereof; (4) $B_6$ or $B'_6$ is selected from the group consisting of Asn, Gln, Glu, Arg, Lys, Ser, Thr, and, with respect to $B'_6$, the descarboxy forms thereof, and the side-chain protected forms thereof; and, with respect to XI and XIII, (5) Y is selected from the group consisting of —$NR_1R_2$, —OR, and —$CH_2OR$, wherein each R, $R_1$, and $R_2$ is selected from a group consisting of hydrogen and straight and branched chain alkyl groups containing 1—6 carbon atoms; then at least one of $B_1$ and $B_4$ is selected such that it is not from a group consisting of Tyr, Trp, Phe, and the side-chain protected forms thereof.

10. The intermediate composition of claim 9 wherein:

$B_1$ and $B_4$ are selected from the group consisting of His, Tyr, Trp, Phe, homologues and analogues thereof, and, with respect to $B_1$, the desamino forms thereof, and the side-chain protected forms thereof;

$B_2$, $B_5$, and $B'_5$ are selected from the group consisting of D-His, D-Tyr, D-Trp, D-Phe, homologues and analogues thereof, and, with respect to $B'_5$, the descarboxy forms thereof, and the side-chain protected forms thereof;

$B_3$ is selected from the group consisting of Gly, Ala, Ser, Asn, Pro, D-Ala, D-Ser, D-Asn, D-Pro, homologues and analogues thereof, and the side-chain protected forms thereof; and

$B_6$ and $B'_6$ are selected from the group consisting of Arg, Lys, Orn, His, Asp, Glu, Asn, Gln, D-Arg, D-Lys, D-Orn, D-His, D-Asp, D-Glu, D-Asn, D-Gln, homologues and analogues thereof, and, with respect to $B'_6$, the descarboxy forms thereof, and the side-chain protected forms thereof.

11. The intermediate composition of claim 9 having the formula selected from the group consisting of

$$\begin{array}{rcl} & (Pr_1)_q & \\ & | & \\ & (X'''_1)_m & \\ & | & \\ (Pr_1)_r—(X'''_2)_a— & B_1 & —\text{D-Trp}—B_3—B_4—B_5—B_6—\textcircled{r} \\ & | & \\ & (X'''_3)_b & \\ & | & \\ & (Pr_1)_s & \end{array} \qquad (XIV)$$

$$\begin{array}{rcl} & (Pr_1)_q & \\ & | & \\ & (X^{iv}_1)_m & \\ & | & \\ (Pr_1)_r—(X'''_2)_a— & B_1 & —\text{D-Trp}—B_3—B_4—B_5—B_6—\textcircled{r} \\ & | & \\ & (X^{iv}_3)_b & \\ & | & \\ & (Pr_1)_s & \end{array} \qquad (XV)$$

$$\begin{array}{rcl} & (Pr_1)_q & \\ & | & \\ & (X'''_1)_m & \\ & | & \\ (Pr_1)_r—(X'''_2)_a— & B_1 & —\text{D-Trp}—B_3—B_4—B'_5—Y \\ & | & \\ & (X'''_3)_b & \\ & | & \\ & (Pr_1)_s & \end{array} \qquad (XVI)$$

$$\begin{array}{rcl} & (Pr_1)_q & \\ & | & \\ & (X'''_1)_m & \\ & | & \\ (Pr_1)_r—(X'''_2)_a— & B_1 & —\text{D-Trp}—B_3—B_4—B_5—O—Pr_2 \\ & | & \\ & (X'''_3)_b & \\ & | & \\ & (Pr_1)_s & \end{array} \qquad (XVII)$$

$B_1$—D-Trp—$B_3$—$B_4$—$B'_5$—Y (XVIII)

$(Pr_1)_q$
|
$(X^{iv}{}_1)_m$
|
$(Pr_1)_r$—$(X^{iv}{}_2)_a$—$B_1$—D-Trp—$B_3$—$B_4$—$B_5$—$B'_6$—Y (XIX)
|
$(X^{iv}{}_3)_b$
|
$(Pr_1)_s$

$(Pr_1)_q$
|
$(X^{iv}{}_1)_b$
|
$(Pr_1)_r$—$(X^{iv}{}_2)_a$—$B_1$—D-Trp—$B_3$—$B_4$—$B_5$—$B_6$—O—Y (XX)
|
$(X^{iv}{}_3)_b$
|
$(Pr_1)_s$

$B_1$—D-Trp—$B_3$—$B_4$—$B_5$—$B'_6$—Y (XXI)

wherein

$B_1$ is selected from the group consisting of Tyr, O—Me—Tyr, His, 3-N-Me-His, p-Cl-Phe, the desamino forms thereof, and the side-chain protected forms thereof;

$B_3$ is selected from the group consisting of Ala, Ser, D-Ala, and the side-chain protected forms thereof;

$B_4$ is selected from the group consisting of Trp, Tyr, and the side-chain protected forms thereof; and

$B_5$ and $B'_5$ are selected from the group consisting of D-Phe, D-His, D-Tyr, D-p-Cl-Phe, and, with respect to $B'_5$, the descarboxy forms thereof, and the side-chain protected forms thereof; and

$B_6$ and $B'_6$ are selected from the group consisting of Arg, HomoArg, Lys, Orn, Asp, Glu, Asn, Gln, D-Lys, the descarboxy forms thereof, and the side-chain protected forms thereof.

12. The intermediate composition of claim 9 of the formula selected from the group consisting of

Boc-His(Tos)-D-Trp-Ala-Trp-D-Phe-ⓡ,
Boc-Tyr(BrZ)-D-Trp-Ala-Trp-D-His(Tos)-ⓡ,
Boc-His(Tos)-D-Trp-Ala-Trp-D-His(Tos)-ⓡ,
Boc-His(Tos)-D-Trp-Ala-Trp-D-Tyr(BrZ)-ⓡ,
Boc-Tyr(BrZ)-D-Trp-Ala-Trp-D-p-Cl-Phe-ⓡ,
Boc-Tyr(BrZ)-D-Trp-Ala-Trp-D-Phe-ⓡ,
Boc-p-Cl-Phe-D-Trp-Ala-Trp-D-Phe-ⓡ,
3(p-OH-phenyl)propanoic acid-D-Trp-Ala-Trp-D-p-Cl-Phe-ⓡ,
Boc-O-Me-Tyr(BrZ)-D-Trp-Ala-Trp-D-Phe-ⓡ,
Boc-Tyr(BrZ)-D-Trp-Ala-Trp-D-Phe-Met-ⓡ,
Boc-Tyr(BrZ)-D-Trp-Ala-Trp-D-Phe-Thr(Bzl)-ⓡ,
Boc-Tyr(BrZ)-D-Trp-Ala-Trp-D-Phe-Glu-α-benzyl ester-ⓡ,
Boc-Tyr(BrZ)-D-Trp-Ala-Trp-D-Phe-Gln-ⓡ,
Boc-Tyr(BrZ)-D-Trp-Ala-Trp-D-Phe-Asn-ⓡ,
Boc-His(Tos)-D-Trp-Ala-Trp-D-Phe-Lys(ClZ)-ⓡ,
Boc-Tyr(BrZ)-D-Trp-Ala-Trp-D-His(Tos)-Lys(ClZ)-ⓡ,
Boc-Tyr(BrZ)-D-Trp-Ala-Trp-D-Phe-Glu-γ-Bzl-ⓡ,
Boc-Tyr(BrZ)-D-Trp-Ala-Trp-D-Phe-Phe-ⓡ,
Boc-Tyr(BrZ)-D-Trp-Gly-Trp-D-Phe-Gln-ONP-ⓡ,
Boc-His(Tos)-D-Trp-Ala-Trp-D-Phe-Lys(ClZ)-ⓡ',
Boc-His(Tos)-D-Trp-Ala-Trp-D-Phe-Arg(Tos)-ⓡ,
Boc-His(Tos)-D-Trp-Ala-Trp-D-Phe-Gln-ONP-ⓡ,
Boc-His(Tos)-D-Trp-Ala-Trp-D-Phe-Glu-γ-Bzl-ⓡ,
Boc-His(Tos)-D-Trp-Ala-Trp-D-Phe-HomoArg(Tos)-ⓡ,
Boc-3-N-Me-His-D-Trp-Ala-Trp-D-Phe-Lys(ClZ)-ⓡ,
Boc-His(Tos)-D-Trp-Ala-Trp-D-Phe-Lys(ClZ)-ⓡ,
Boc-His(Tos)-D-Trp-Ala-Trp-D-Phe-Orn(Z)-ⓡ,
Boc-His(Tos)-D-Trp-Val-Trp-D-Phe-Lys(ClZ)-ⓡ, and
Boc-His(Tos)-D-Trp-Ser(Bzl)-Trp-D-Phe-Lys(ClZ)-ⓡ,

wherein

Boc is t-butyloxycarbonyl;

BrZ is o-bromobenzyloxycarbonyl;
BrZ is p-bromobenzyloxycarbonyl;
Bzl is benzyl;
γ-Bzl is γ-benzylester;
ClZ is o-chloro-benzyloxycarbonyl;
ONP is p-nitrophenyl ester;
Tos is p-toluenesulfonyl;
Z is benzyloxycarbonyl;
Ⓡ is p-methylbenzhydryamine resin; and
Ⓡ' is hydroxymethyl resin.

**Patentansprüche**

1. Peptid mit einer Formel aus der Gruppe

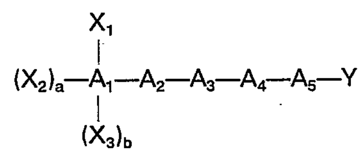

(I)

bzw.

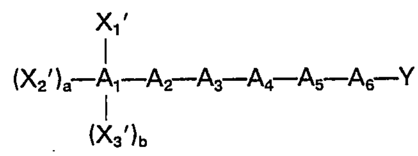

(II)

worin bedeuten:

$X_1$, $X_2$, $X_3$, $X_1'$, $X_2'$ und $X_3'$ bedeuten N-endständige und/oder Desamino-α-Kohlenstoff-Substitutionen;

a und bedeuten 0 oder 1, mit der Maßgabe, daß a und b 0 sind, wenn $A_1$ einen Desamino-Rest bedeutet;

$A_1$ und $A_4$ bedeuten His, Arg, Lys, α-Naphth, β-Naphth, Iql, Tyr, Trp, Phe und/oder deren Homologe bzw. Analoge und, bzw. im Hinblick auf $A_1$, deren Desamino-Formen;

$A_2$ und $A_5$ bedeuten D-His, D-Arg, D-Lys, D-α-Naphth, D-β-Naphth, D-Iql, D-Tyr, D-Trp, D-Phe und/oder deren Homologe und Analoge bzw., im Hinblick auf $A_5$, deren Descarboxy-Formen;

$A_3$ bedeutet Gly, Ala, Val, Leu, Ile, Pro, Ser, Thr, Met, Asp, Glu, Asn, Gln, His, D-Ala, D-Val, D-Leu, D-Ile, D-Pro, D-Ser, D-Thr, D-Met, D-Asp, D-Glu, D-Asn, D-Gln, D-His und/oder deren Homologe und Analoge;

$A_6$ bedeutet Aminodsäuren der L- und D-Konfiguration und/oder deren Homologe und Analoge bzw. deren Descarboxy-Formen; Y bedeutet C-endständige und Descarboxy-α-Kohlenstoff-Substitutionen;

sowie dessen pharmazeutisch akzeptablen Salze; mit der Maßgabe, daß

(a) wenn (1) a = 1 und b = 0 und $X_1$ und $X_2$—H und/oder —$CH_3$; (2) $A_1$ und $A_4$ Tyr, Trp und/oder Phe; (3) $A_3$ Gly, Ala, Val, Leu, Ile, Pro, Ser, Thr, Met, Asp, Glu, Asn, Gln und/oder His; und (4) Y —$NR_1R_2$, —$CH_2OR$ und/oder —OR bedeuten, worin R, $R_1$ und $R_2$ Wasserstoff und/oder geradkettige bzw. verzweigtkettige Alkylgruppen mit 1 bis 6 Kohlenstoffatomen darstellen; mindestens eines von $A_2$ und $A_5$ nicht D-Tyr, D-Trp, D-Phe und/oder im Hinblick auf $A_5$, deren Descarboxy-Formen bedeutet;

(b) wenn (1) a = 1 und b = 0 und $X_1$ und $X_2$—H und/oder —$CH_3$; (2) $A_2$l und $A_5$ D-Tyr, D-Trp, D-Phe und, im Hinblick auf $A_5$, deren Descarboxy-Formen; (3) $A_3$ Gly, Ala, Val, Leu, Ile, Pro, Ser, Thr, Asp, Glu, Asn, Gln und/oder His; und (4) Y —$NR_1R_2$, —$CH_2OR$ und/oder —OR bedeuten, worin R, $R_1$, $R_2$ Wasserstoff und/oder geradkettige und verzweigtkettige Alkylgruppen mit 1 bis 6 Kohlenstoffatomen darstellen; mindestens eines von $A_1$ und $A_4$ nicht Tyr, Trp und/oder Phe bedeutet;

(c) (1) wenn a = 1 und b = 0 und $X_1'$ und $X_2'$ —H, $CH_3$, und/oder —$CHOCH_3$; (2) $A_1$ und $A_4$ Tyr, Trp und/oder Phe; (3) $A_3$ Gly, Ala, Val, Leu, Ile, Ser, Thr, Met, Asn und/oder Gln; (4) $A_6$ Asn, Gln, Gly, Arg, Lys, Ser, Thr und/oder deren Descarboxy-Formen; und (5) Y —$NR_1R_2$, —$CH_2OR$ und/oder —OR bedeuten, worin R, $R_1$ und $R_2$ Wasserstoff und/oder geradkettige und verzweigtkettige Alkylgruppen mit 1 bis 6 Kohlenstoffatomen darstellen; mindstens eines von $A_2$ und $A_5$ nicht D-Tyr, D-Typ und D-Phe bedeutet; und

(d) wenn (1) a = 1 und b = 0 und $X_1'$ und $X_2'$ —H, $CH_3$ und/oder —$CHOCH_3$; (2) $A_2$ und $A_5$ D-Tyr, D-Trp und/oder T-Phe; (3) $A_3$ Gly, Ala, Val, Leu, Ile, Ser, Thr, Met, Asn und/oder Gln; (4) $A_6$ Asn, Gln, Gly, Arg, Lys, Ser, Thr und/oder deren Descarboxy-Formen; und (5) Y —$NR_1R_2$, —$CH_2OR$ und/oder —OR bedeuten, worin R, $R_1$, $R_2$ Wasserstoff und/oder geradkettige bzw. verzweigtkettige Alkylgruppen mit 1 bis 6 Kohlenstoffatomen darstellen; mindestens eines von $A_1$ und $A_4$ nicht Tyr, Trp und/oder Phe bedeutet.

2. Peptid nach Anspruch 1, worin bedeutet:

a = 0 oder 1 und b = 0;

$X_1$, $X_2$, $X_1'$ und $X_2'$ bedeuten —R, —OR und/oder RC(O)—, worin R Wasserstoff und/oder geradkettige bzw. verzweigtkettige Alkylgruppen mit 1 bis 6 Kohlenstoffatomen darstellt;

$A_1$ und $A_4$ bedeuten His, Trp, Phe und/oder deren Homologe bzw. Analoge und, im Hinblick auf $A_1$, deren Desaminoformen;

$A_2$ und $A_5$ bedeuten D-His, D-Tyr, D-Trp, D-Phe und/oder deren Homologe bzw. Analoge und, im Hinblick auf $A_5$, deren Descarboxyformen;

$A_3$ bedeutet Gly, Ala, Ser, Asn, Pro, D-Ala, D-Ser, D-Asn, D-Pro und/oder deren Homologe bzw. Analoge;

$A_6$ bedeutet Arg, Lys, Orn, His, Asp, Glu, Asn, Gln, D-Arg, D-Lys, D-Orn, D-His, D-Asp, D-Glu, D-Asn, D-Gln, D-Arg und/oder deren Homologe bzw, Analoage bzw. deren Descarboxy-Formen;

Y bedeutet —$CH_2OH$, —OR und/oder $NR_1O_2$, worin R, $R_1$ und $R_2$ Wasserstoff und/oder geradkettige bzw. verzweigtkettige Alkylgruppen mit 1 bis 6 Kohlenstoffatomen darstellen;

sowie dessen pharmazeutisch akzeptablen Salze.

3. Peptid nach Anspruch 1 mit der Formel

$$(X_2)_a—A_1—\overset{\overset{\displaystyle H}{|}}{D}—Trp—A_3—A_4—A_5—Y \qquad (IV)$$

bzw.

$$(X_2')_a—A_1—\overset{\overset{\displaystyle H}{|}}{D}—Trp—A_3—A_4—A_5—A_6—Y \qquad (V)$$

worin bedeuten:

a = 0 oder 1;

$X_2$ und $X_2'$ bedeuten RCO und/oder R—, worin R Wasserstoff und/oder Alkylgruppen mit 1 bis 2 Kohlenstoffatomen darstellt;

$A_1$ bedeutet Tyr, O-Me-Tyr, His, 3-N-Me-His, p-Cl-Phe und/oder deren Desamino-Formen;

$A_3$ bedeutet Ala, Ser und/oder D-Ala;

$A_4$ bedeutet Trp und/oder Tyr;

$A_5$ bedeutet D-Phe, D-His, D-Tyr und/oder D-p-Cl-Phe;

$A_6$ bedeutet Arg, HomoArg, Lys, Orn, Asp, Glu, Asn, Gln und/oder D-Lys;

Y bedeutet —OR und/oder —NHR, worin R Wasserstoff und/oder Alkylgruppen mit 1 bis 2 Kohlenstoffatomen darstellt;

sowie dessen pharmazeutisch akzeptablen Salze.

4. Peptid nach Anspruch 1, mit einer der Formeln

$H_2$-His-D-Trp-Ala-Trp-D-Phe-$NH_2$,
$H_2$-Tyr-D-Trp-Ala-Trp-D-His-$NH_2$,
$H_2$-His-D-Trp-Ala-Trp-D-His-$NH_2$,
$H_2$-His-D-Trp-Ala-Trp-D-Tyr-$NH_2$,
$H_2$-Tyr-D-Trp-Ala-Trp-D-p-Cl-Phe-$NH_2$,
$H_2$-p-Cl-Phe-D-Trp-Ala-Trp-D-Phe-$NH_2$,
H-desaminoTyr-D-Trp-Ala-Trp-D-Phe-$NH_2$,

$$CH_3CO\text{-Tyr-}\overset{\overset{\displaystyle H}{|}}{D}\text{-Trp-Ala-Trp-D-Phe-}NH_2,$$

$H_2$-O-Me-Tyr-D-Trp-Ala-Trp-D-Phe-$NH_2$,
$H_2$-Tyr-D-Trp-Ala-Trp-D-Phe-Met-$NH_2$,
$H_2$-Tyr-D-Trp-Ala-Trp-D-Phe-Thr-$NH_2$,
$H_2$-Tyr-D-Trp-Ala-Trp-D-Phe-Gln-OH,
$H_2$-Tyr-D-Trp-Ala-Trp-D-Phe-Gln-$NH_2$,
$H_2$-Tyr-D-Trp-Ala-Trp-D-Phe-Asn-$NH_2$,
$H_2$-His-D-Trp-Ala-Trp-D-Phe-Lys-$NH_2$,
$H_2$-Tyr-D-Trp-Ala-Trp-D-Phe-Lys-$NH_2$,
$H_2$-Tyr-D-Trp-Ala-Trp-D-Phe-Glu-$NH_2$,
$H_2$-Tyr-D-Trp-Ala-Trp-D-Phe-Phe-$NH_2$,
$H_2$-Tyr-D-Trp-Ala-Trp-D-Phe-Gln-$NH_2$,
$H_2$-His-D-Trp-Ala-Trp-D-Phe-Lys-OH,
$H_2$-His-D-Trp-Ala-Trp-D-Phe-Arg-$NH_2$,
$H_2$-His-D-Trp-Ala-D-Phe-Gln-$NH_2$,
$H_2$-His-D-Trp-Ala-Trp-D-Phe-Glu-$NH_2$,
$H_2$-His-D-Trp-Ala-Trp-D-Phe-HomoArg-$NH_2$,
$H_2$-3-N-Me-His-D-Trp-Ala-Trp-D-Phe-Lys-$NH_2$,
$H_2$-His-D-Trp-Ala-Trp-D-Phe-Lys-$NHCH_2CH_3$,
$H_2$-His-D-Trp-Ala-Trp-D-Phe-Orn-$NH_2$,

$H_2$-His-D-Trp-Val-Trp-D-Phe-Lys-$NH_2$, bzw.
$H_2$-His-D-Trp-Ser-Trp-D-Phe-Lys-$NH_2$.

5. Kombination, enthaltend:
(a) mindestens ein wachstumsförderndes Mittel; und
(b) mindestens eines der Peptide nach einem der Ansprüche 1 bis 3 oder 4.
6. Kombination nach Anspruch 5, worin das Peptid die Formel

$H_2$-His-D-Trp-Ala-Trp-D-Phe-Lys-$NH_2$

hat und worin das wachstumsfördernde Mittel Zeranol, $C_{18}H_{26}O_5$ darstellt.

7. Peptid nach einem der vorhergehenden Ansprüche, zur Regelung der Freisetzung von Wachstumshormon aus der Hypophyse.

8. Peptid nach einem der vorhergehenden Ansprüche zur Regelung der Freisetzung von Wachstumshormon *in vivo* aus der Hypophyse.

9. Zwischenverbindung mit einer der Formeln

$(Pr_1)_q$
|
$(X'''_1)_m$
|
$(Pr_1)_r$—$(X'''_2)_a$—$B_1$—$B_2$—$B_3$—$B_4$—$B_5^-$Ⓡ (VI)
|
$(X'''_3)_b$
|
$(Pr_1)_s$—

$(Pr_1)_q$
|
$(X^{iv}_1)_m$
|
$(Pr_1)_r$—$(X^{iv}_2)_a$—$B_1$—$B_2$—$B_3$—$B_4$—$B_5$—$B_6$Ⓡ (VII)
|
$(X^{iv}_3)_b$
|
$(Pr_1)_s$

$(Pr_1)_q$
|
$(X'''_1)_m$
|
$(Pr_1)_r$—$(X'''_2)_a$—$B_1$—$B_2$—$B_3$—$B_4$—$B'_5$—Y (VIII)
|
$(X'''_3)_b$
|
$(Pr_1)_q$
|
$(X'''_1)_m$
|
$(Pr_1)_r$—$(X'''_2)_a$—$B_1$—$B_2$—$B_3$—$B_4$—$_5$—O—$Pr_2$ (IX)
$(X'''_3)_b$
|
$(Pr_1)_s$

$B_1$—$B_2$—$B_3$—$B_4$—$B'_5$—Y (X)

$(Pr_1)_q$
|
$(X^{iv}_1)_m$
|
$(Pr_1)_r$—$(X^{iv}_2)_a$—$B_1$—$B_2$—$B_3$—$B_4$—$B_5$—$B'_6$—Y (XI)
$(X^{iv}_3)_b$
|
$(Pr_1)_s$

$$\begin{array}{c} (Pr_1)_q \\ | \\ (X^{iv}{}_1)_m \\ | \\ (Pr_1)_r—(X^{iv}{}_2)_a—B_1—B_2—B_3—B_4—B_5—B_6—O—Pr_2 \\ | \\ (X^{iv}{}_3)_b \\ | \\ (Pr_1)_s \end{array} \qquad (XII)$$

bzw.

$$B_1—B_2—B_3—B_4—B_5—B'_6—Y \qquad (XIII)$$

(XIV)

worin bedeuten:

$Pr_1$ bedeutet eine α-Aminosäure-Schutzgruppe;

a, b, m, q, und s sind jeweils 0 oder 1;

$X'''_1$, $X'''_2$, $X'''_3$, $X^{iv}_2$ und $X^{iv}_3$ bedeuten N-endständige und Desamino-α-Kohlenstoff-Substitutionen und -Reste;

Desamino-α-Kohlenstoff-Substitutionen und -Reste;

$B_1$ und $B_4$ bedeuten His, Arg, Lys, α-Naphth, β-Naphth, Iql, Tyr, Trp, Phe und/oder deren Homologe und Analoge, deren in der Seitenkette geschützten Formen bzw., im Hinblick auf $B_1$, deren Desamino-Formen;

$B_2$, $B_5$ und $B'_5$ bedeuten D-His, D-Arg, D-Lys, D-α-Naphth, D-β-Naphth, D-Iql, D-Tyr, D-Trp, D-Phe und/oder deren Homologe und Analoge, deren in der Seitenkette geschützten Formen bzw., im Hinblick auf $B'_5$ deren Descarboxy-Formen;

$B_3$ bedeutet Gly, Ala, Val, Leu, Ile, Pro, Ser, Thr, Met, Asp, Glu, Asn, Gln, His, D-Ala, D-Val, D-Leu, D-Ile, D-Pro, D-Ser, D-Thr, D-Met, D-Asp, D-Glu, D-Asn, D-Gln, D-Arg, D-His und/oder deren Homologe und Analoge bzw. deren in der seitenkette geschützten Formen;

$B_6$ und $B'_6$ bedeuten Aminosäuren der L- und D-Konfiguration, deren Homologe und Analoge, deren in der Seitenkette geschützten Formen und/oder im Hinblick auf $B'_6$, deren Descarboxy-Formen;

Ⓡ bedeutet ein Harz;

Y bedeutet C-endständige und Descarboxy-α-Kohlenstoff-Substitutionen; und

$Pr_2$ bedeutet eine Carboxyl-Schutzgruppe; mit der Maßgabe, daß, wenn

(1) a = 1 und b und m = 0 und $X'''_2$ —H und/oder —$CH_3$; (2) $B_1$ und $B_4$ Tyr, Trp, Phe und/oder deren in der Seitenkette geschützten Formen; (3) $B_3$ Gly, Ala, Val, Leu, Ile, Pro, Ser, Thr, Met, Asp, Glu, Asn, Gln, His und/oder deren in der Seitenkette geschützten Formen; und, im Hinblick auf XI und XIII (4) Y —$NH_1R_2$, —OR und/oder —$CH_2OR$ bedeuten, worin jedes R, $R_1$ und $R_2$ Wasserstoff und/oder geradkettige bzw. verzweigtkettige Alkylgruppen mit 1 bis 6 Kohlenstoffatomen darstellt, mindestens eines von $B_2$, $B_5$ oder $B'_5$ kein D-Tyr, D-Trp, D-Phe bzw., im Hinblick auf $B'_5$ keine von deren Descarboxy-Formen bzw. keine von deren in der Seitenkette geschützten Formen darstellt;

wenn (1) a = 1 und b und m = 0 und $X'''_2$ —H und/oder —$CH_3$; (2) $B_2$ und $B_5$ oder $B'_5$ D-Tyr, D-Trp, D-Phe und/oder, im Hinblick auf $B'_5$, deren Descarboxy-Formen bzw. deren in der Seitenkette geschützten Formen; (3) $B_3$ Gly, Ala, Val, Leu, Ile, Pro, Ser, Thr, Met, Asp, Glu, Asn, Gln, His und/oder deren in der Seitenkette geschützten Formen und/oder, im Hinblick auf XI und XIII (4) Y—$NR_1R_2$, —OR und/oder —$CH_2OR$ bedeuten, worin jedes R, $R_1$ und $R_2$ Wasserstoff und/oder geradkettige bzw. verzweigtkettige Alkylgruppen mit 1 bis 6 Kohlenstoffatomen darstellt, mindestens eines von $B_1$ und $B_4$ kein Tyr, Trp, Phe und/oder deren in der Seitenkette geschützten Formen bedeutet;

wenn (1) a = 1 und b und m = 0 und $X^{iv}_2$ —H —$CH_3$ und/oder —$CHOCH_3$; (2) $B_1$ und $B_4$ Tyr, Trp, Phe und/oder deren in der Seitenkette geschützten Formen; (3) $B_3$ Gly, Ala, Val, Leu, Ile, Ser, Thr, Met, Asn, Gln und/oder deren in der Seitenkette geschützten Formen; (4) $B_6$ oder $B'_6$ Asn, Gln, Glu, Arg, Lys, Ser, Thr und/oder, im Hinblick auf $B'_6$, deren Descarboxy-Formen bzw. deren in der Seitenkette geschützten Formen und, im Hinblick auf XI und XIII, (5) Y —$NR_1R_2$, —OR und/oder geradkettige bzw. verzweigtkettige Alkylgruppen mit 1 bis 6 Kohlenstoffatomen darstellt; mindestens eines von $B_2$ oder $B_5$ kein D-Tyr, D-Trp, D-Phe bzw. keine von deren in der Seitenkette geschützten Formen darstellt; und wenn (1) a = 1 und b und m = 0 und $X^{iv}_2$ —H, $CH_3$ und/oder $CHOCH_3$; (2) $B_2$ und $B_5$ D-Tyr, D-Trp, D-Phe und/oder deren in der Seitenkette geschützten Formen; (3) $B_3$ Gly, Ala, Val, Leu, Ile, Ser, Thr, Met, Asn, Gln und/oder deren in der Seitenkette geschützten Formen; (4) $B_6$ oder $B'_6$ Asn, Gln, Glu, Arg, Lys, Ser, Thr und/oder im Hinblick auf $B'_6$ deren Descarboxy-Formen bzw. deren in der Seitenkette geschützten Formen; und, im Hinblick auf XI und XIII, (5) Y —$NR_1R_2$, —OR und/oder —$CH_2OR$ bedeuten, worin jedes R, $R_1$ und $R_2$ Wasserstoff und/oder —$CH_2OR$ bedeuten, worin jedes, R, $R_1$ und $R_2$ Wasserstoff und/oder geradkettige bzw. verzweigtkettige Alkylgruppen mit 1 bis 6 Kohlenstoffatomen darstellt; mindestens eines vom $B_2$ und $B_4$ kein Tyr, Trp, Phe und/oder deren in der Seitenkette geschützten Formen bedeutet.

10. Zwischenverbindung nach Anspruch 9, worin bedeuten: $B_1$ und $B_4$ bedeuten His, Tyr, Trp, Phe, deren Homologe und Analoge und, im Hinblick auf $B_1$, deren Desamino-Formen und/oder deren in der Seitenkette geschützten Formen; $B_2$, $B_5$ und $B'_5$ bedeuten D-His, D-Tyr, D-Trp, D-Phe, deren Homologe und Analoge und/oder, im Hinblick auf $B'_5$, deren Descarboxy-Formen und deren in der Seitenkette geschützten Formen;

$B_3$ bedeutet Gly, Ala, Ser, Asn, Pro, D-Ala, D-Ser, D-Asn, D-Pro, deren Homologe und Analoge und/oder deren in der Seitenkette geschützten Formen; und

$B_6$ und $B'_6$ bedeuten Arg, Lys, Orn, His, Asp, Glu, Asn, Gln, D-Arg, D-Lys, D-Orn, D-His, D-Asp, D-Glu, D-Asn, D-Gln, deren Homologe und Analoge und, im Hinblick auf $B'_6$, deren Descarboxy-Formen und/oder deren in der Seitenkette geschützten Formen.

11. Zwischenverbindung nach Anspruch 10 mit der Formel

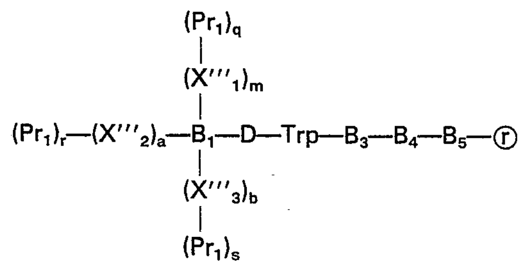

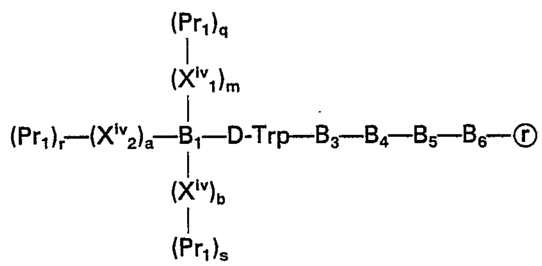

(XV)

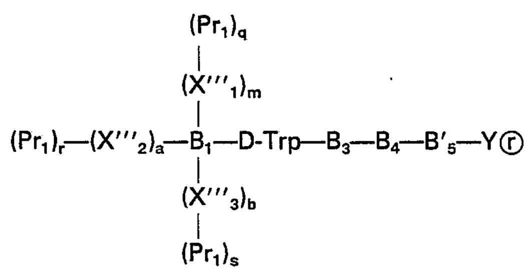

(XVI)

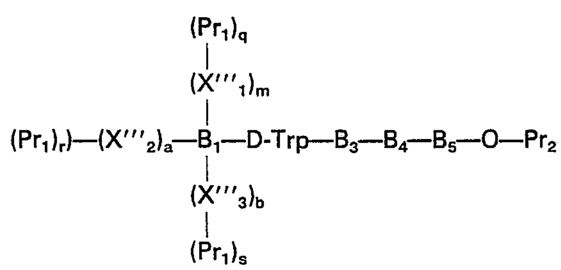

(XVII)

$B_1$—D-Trp—$B_3$—$B_4$—$B'_5$—Y (XVIII)

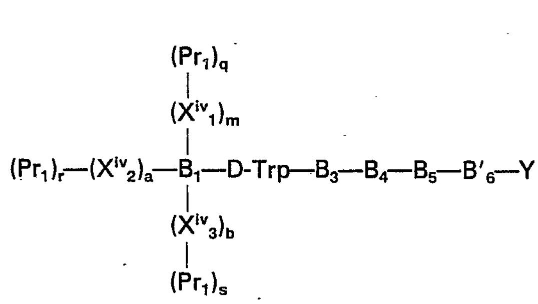

(XIX)

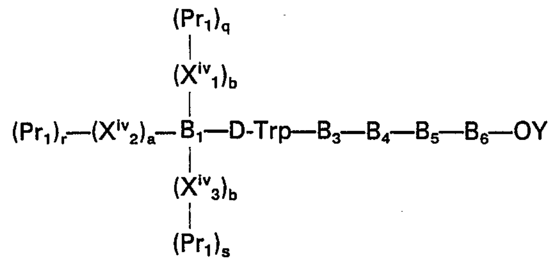 (XX)

bzw.

$$B_1—D\text{-}Trp—B_3—B_4—B_5—B'_6—Y \qquad (XXI)$$

worin bedeuten:

$B_1$ bedeutet Tyr, O-Me-Tyr, His, 3-N-Me-His, p-Cl-Phe, deren Desamino-Formen und/oder deren in der Seitenkette geschützten Formen;

$B_3$ bedeutet Ala, Ser, D-Ala und/oder deren in der Seitenkette geschützten Formen;

$B_4$ bedeutet Trp, Tyr und/oder deren in der Seitenkette geschützten Formen; und

$B_5$ und $B'_5$ bedeuten D-Phe, D-His, D-Tyr, D-p-Cl-Phe, und/oder im Hinblick auf $B'_5$, deren Descarboxy-Formen und/oder deren in der Seitenkette geschützten Formen; und

$B_6$ und $B'_6$ bedeuten Arg, HomoArg, Lys, Orn, Asp, Glu, Asn, Gln, D-Lys und/oder Descarboxyformen bzw. deren in der Seitenkette geschützten Formen.

12. Zwischenverbindung nach Anspruch 10, mit der Formel

Boc-His(Tos)-D-Trp-Ala-Trp-D-Phe-ⓡ,
Boc-Tyr(Brz)-D-Trp-Ala-Trp-D-His(Tos)-ⓡ,
Boc-His(Tos)-D-Trp-Ala-Trp-D-His(Tos)-ⓡ,
Boc-His(Tos)-D-Trp-Ala-Trp-D-Tyr(BrZ)-ⓡ,
Boc-Tyr(BrZ)-D-Trp-Ala-Trp-D-p-Cl-Phe-ⓡ,
Boc-Tyr(BrZ)-D-Trp-Ala-Trp-D-Phe-ⓡ,
Boc-p-Cl-Phe-D-Trp-Ala-Trp-D-Phe-ⓡ,
3(p-OH-phenyl)propansäure-D-Trp-Ala-Trp-D-p-Cl-Phe-ⓡ,
Boc-O-Me-Tyr(BrZ)-D-Trp-Ala-Trp-D-Phe-ⓡ,
Boc-Tyr(BrZ)-D-Trp-Ala-Trp-D-Phe-Met-ⓡ,
Boc-Tyr(BrZ)-D-Trp-Ala-Trp-D-Phe-Thr(Bzl)-ⓡ,
Boc-Tyr(BrZ)-D-Trp-Ala-Trp-D-Phe-Glu-α-benzyl ester-ⓡ,
Boc-Tyr(BrZ)-D-Trp-Ala-Trp-D-Phe-Gln-ⓡ,
Boc-Tyr(BrZ)-D-Trp-Ala-Trp-D-Phe-Asn-ⓡ,
Boc-His(Tos)-D-Trp-Ala-Trp-D-Phe-Lys(ClZ)-ⓡ,
Boc-Tyr(BrZ)-D-Trp-Ala-Trp-D-His(Tos)-Lys(ClZ)-ⓡ,
Boc-Tyr(BrZ)-D-Trp-Ala-Trp-D-Phe-Glu-γ-Bzl-ⓡ,
Boc-Tyr(BrZ)-D-Trp-Ala-Trp-D-Phe-Phe-ⓡ,
Boc-Tyr(Brz)-D-Trp-Gly-Trp-D-Phe-Gln-ONP-ⓡ,
Boc-His(Tos)-D-Trp-Ala-Trp-D-Phe-Lys(ClZ)-ⓡ',
Boc-His(Tos)-D-Trp-Ala-Trp-D-Phe-Arg(Tos)-ⓡ,
Boc-His(Tos)-D-Trp-Ala-Trp-D-Phe-Gln-ONP-ⓡ,
Boc-His(Tos)-D-Trp-Ala-Trp-D-Phe-Glu-γ-Bzl-ⓡ,
Boc-His(Tos)-D-Trp-Ala-Trp-D-Phe-HomoArg(Tos)-ⓡ,
Boc-3-N-Me-His-D-Trp-Ala-Trp-D-Phe-Lys(ClZ)-ⓡ,
Boc-His(Tos)-D-Trp-Ala-Trp-D-Phe-Lys(ClZ)-ⓡ,
Boc-His(Tos)-D-Trp-Ala-Trp-D-Phe-Orn(Z)-ⓡ,
Boc-His(Tos)-D-Trp-Val-Trp-D-Phe-Lys(ClZ)-ⓡ, bzw.
Boc-His(Tos)-D-Trp-Ser(Bzl)-Trp-D-Phe-Lys(ClZ)-ⓡ,

worin bedeuten:

Boc bedeutet t-Butyloxycarbonyl;
BrZ bedeutet o-Brombenzyloxycarbonyl;
BrZ bedeutet p-Brombenzyloxycarbonyl;
Bzl bedeutet Benzyl;
γ-Bzl bedeutet γ-Benzylester;
ClZ bedeutet o-Chlor-Benzyloxycarbonyl;
ONP bedeutet Nitrophenylester;
Tos bedeutet p-Toluolsulfonyl;
Z bedeutet Benzyloxycarbonyl;
ⓡ bedeutet p-Methylbenzhydrylaminharz; und
ⓡ' bedeutet Hydroxymethylharz.

**Revendications**

1. Peptide caractérisé en ce qu'il est choisi parmi l'ensemble répondant aux formules

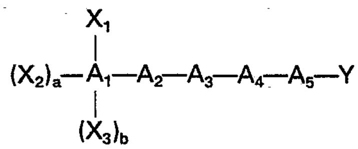

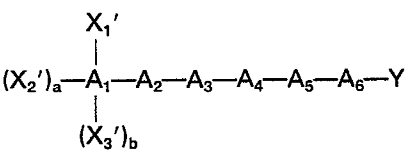

(II)

où

$X_1$, $X_2$, $X_3$, $X_1'$, $X_2'$, et $X_3'$ sont choisis parmi l'ensemble constitué par les substitutions sur l'atome d'azote terminal et l'atome de carbone en α d'un reste désamino;

a et b ont pour valeur 0 ou 1, avec la condition que a et b valent 0 quand $A_1$ est un reste désamino;

$A_1$ et $A_4$ sont choisis parmi l'ensemble constitué par les His, Arg, Lys, α-Napht, β-Napht, Iql, Tyr, Trp, Phe, leurs homologues et analogues, et, en ce qui concerne $A_1$, les formes désamino correspondantes;

$A_2$ et $A_5$ sont choisis parmi l'ensemble constitué par les D-His, D-Arg, D-Lys, D-α-Napht, D-β-Napht, D-Iql, D-Tyr, D-Trp, D-Phe, leurs homologues et analogues, et, en ce qui concerne $A_5$, les formes décarboxy correspondantes;

$A_3$ est choisi parmi l'ensemble constitué par les Gly, Ala, Val, Leu, Ile, Pro, Ser, Thr, Met, Asp, Glu, Asn, Gln, His, D-Ala, D-Val, D-Leu, D-Ile, D-Pro, D-Ser, D-Thr, D-Met, D-Asp, D-Glu, D-Asn, D-Gln, D-His, et leurs homologues et analogues;

$A_6$ est choisi parmi l'ensemble constitué par les aminoacides de configuration L- et D-, leurs homologues et analogues, et leurs formes décarboxy;

Y est choisi parmi l'ensemble constitué par les substitutions de l'atome C-terminal et de l'atome C en α d'un reste décarboxy;

et ses sels pharmaceutiquement acceptables;

avec la condition que (a) si (1) a vaut 1 et b 0, et, $X_1$ et $X_2$ sont choisis parmi les groupes —H et —$CH_3$; (2) $A_1$ et $A_4$ sont choisis parmi les groupes Tyr, Trp et Phe; (3) $A_3$ est choisi parmi les groupes Gly, Ala, Val, Leu, Ile, Pro, Ser, Thr, Met, Asp, Glu, Asn, Gln et His, et (4) Y est choisi parmi les groupes constitués par —$NR_1R_2$, —$CH_2OR$ et —OR où R, $R_1$ et $R_2$ sont choisis parmi l'atome d'hydrogène et les groupes alkyle en $C_1$—$C_6$ à chaîne linéaire ou ramifiée; alors un au moins des $A_2$ et $A_5$ est différent des groupes D-Tyr, D-Trp, D-Phe, et, en ce qui concerne $A_5$, les formes décarboxy correspondantes;

(b) si (1) a vaut 1 et b 0, et, $X_1$ et $X_2$ sont choisis parmi les groupes —H et —$CH_3$; (2) $A_2$ et $A_5$ sont choisis parmi les groupes D-Tyr, D-Trp, D-Phe, et, en ce qui concerne $A_5$, les formes décarboxy correspondantes; (3) $A_3$ est choisi parmi les groupes Gly, Ala, VDal, Leu, Ile, Pro, Ser, Thr, Met, Asp, Glu, Asn, Gln et His; et (4) Y est choisi parmi les groupes —$NR_1R_2$, —$CH_2OR$, et —OR, où R, $R_1$ et $R_2$ sont choisis parmi l'atome d'hydrogène et les groupes alkyle en $C_1$—$C_6$ à chaîne linéaire ou ramifiée; alors un au moins des $A_1$ et $A_4$ est différent des groupes Tyr, Trp et Phe;

(c) si (1) a vaut 1 et b 0, et, $X_1'$ et $X_2'$ sont choisis parmi les groupes —H, —$CH_3$ et —$CHOCH_3$; (2) $A_1$ et $A_4$ sont choisis parmi les groupes Tyr, Trp et Phe; (3) $A_3$ est choisi parmi les groupes Gly, Ala, Val, Leu, Ile, Ser, Thr, Met, Asn et Gln; (4) $A_6$ est choisi parmi les groupes Asn, Gln, Glu, Arg, Lys, Ser, Thr et les formes décarboxy correspondantes; et (5) Y est choisi parmi les groupes —$NR_1R_2$, —$CH_2OR$ et —OR où R, $R_1$ et $R_2$ sont choisis parmi l'atome d'hydrogène et les groupes alkyle en $C_1$—$C_6$ à chaîne linéaire ou ramifiée; alors un au moins des $A_2$ et $A_5$ est différent des groupes D-Tyr, D-Trp et D-Phe; et

(d) si (1) a vaut 1 et b 0, et $X_1'$ et $X_2'$ sont choisis parmi les groupes —H, —$CH_3$ et —$CHOCH_3$; (2) $A_2$ et $A_5$ sont choisis parmi l'ensemble constitué par D-Tyr, D-Trp et D-Phe; (3) $A_3$ est choisi parmi l'ensemble comprenant Gly, Ala, Val, Leu, Ile, Ser, Thr, Met, Asn et Gln; (4) $A_6$ est choisi parmi l'ensemble constitué par Asn, Gln, Glu, Arg, Lys, Ser, Thr et les formes décarboxy correspondantes; et (5) Y est choisi parmi les groupes —$NR_1R_2$, —$CH_2OR$ et —OR où R, $R_1$ et $R_2$ sont choisis parmi l'atome d'hydrogène et les groupes alkyle en $C_1$—$C_6$ à chaîne linéaire ou ramifiée; alors au moins un des $A_1$ et $A_4$ est différent des groupes Tyr, Trp et Phe.

2. Peptide suivant la revendication 1, dans lequel:

a vaut 0 ou 1 et b vaut 0;

$X_1$, $X_2$, $X_1'$ et $X_2'$ sont choisis parmi l'ensemble constitué par —R, —OR et RC(O)—, où R est choisi parmi l'atome d'hydrogène et les groupes alkyle en $C_1$—$C_6$ à chaîne linéaire ou ramifiée;

$A_1$ et $A_4$ sont choisis parmi l'ensemble constitué par les His, Tyr, Trp, Phe, leurs homologues et analogues, et, en ce qui concerne $A_1$, les formes désamine correspondantes;

$A_2$ et $A_5$ sont choisis parmi l'ensemble constitué par les D-His, D-Tyr, D-Trp, D-Phe, leurs homologues et analogues, et, en ce qui concerne $A_5$, les formes décarboxy correspondantes;

$A_3$ est choisi parmi l'ensemble constitué par les Gly, Ala, Ser, Asn, Pro, D-Ala, D-Ser, D-Asn, D-Pro et leurs homologues et analogues;

$A_6$ est choisi parmi l'ensemble constitué par les Arg, Lys, Orn, His, Asp, Glu, Asn, Gln, D-Arg, D-Lys, D-Orn, D-His, D-Asp, D-Glu, D-Asn, D-Gln, D-Arg, leurs homologues et analogues, et les formes décarboxy correspondantes;

Y est choisi parmi l'ensemble constitué par $-CH_2OH$, $-OR$ et $-NR_1R_2$ où R, $R_1$ et $R_2$ sont choisis parmi l'atome d'hydrogène et les groupes alkyle en $C_1-C_6$ à chaîne linéaire ou ramifiée; et ses sels pharmaceutiquement acceptables.

3. Peptide suivant la revendication 1, caractérisé en ce qu'il répond à la formule choisie parmi

$$\overset{H}{|}\quad (X_2)_a-A_1-\text{D-Trp}-A_3-A_4-A_5-Y \qquad \text{(IV)}$$

$$\overset{H}{|}\quad (X_2')_a-A_1-\text{D-Trp}-A_3-A_4-A_5-A_6-Y \qquad \text{(V)}$$

a vaut 0 ou 1;

$X_2$ et $X_2'$ sont choisis parmi l'ensemble constitué par RCO et R où R est choisi parmi l'atome d'hydrogène et les groupes alkyle en $C_1-C_2$;

$A_1$ est choisi parmi l'ensemble constitué par les Tyr, O-Me-Tyr, His, 3-N-Me-His, p-Cl-Phe, et les formes désamino correspondantes;

$A_3$ est choisi parmi l'ensemble constitué par les groupes Ala, Ser et D-Ala;

$A_4$ est choisi parmi l'ensemble constitué par les groupes Trp et Tyr;

$A_5$ est choisi parmi l'ensemble constitué par les groupes D-Phe, D-His, D-Tyr et D-p-Cl-Phe;

$A_6$ est choisi parmi l'ensemble constitué par les groupes Arg, HomoArg, Lys, Orn, Asp, Glu, Asn, Gln et D-Lys;

Y est choisi parmi l'ensemble constitué par les groupes $-OR$, et $-NHR$, où R est choisi parmi l'atome d'hydrogène et les groupes alkyle en $C_1-C_2$; et ses sels pharmaceutiquement acceptables.

4. Peptide suivant la revendication 1, caractérisé en ce qu'il est choisi parmi l'ensemble comprenant les

$H_2$-His-D-Trp-Ala-Trp-D-Phe-$NH_2$,
$H_2$-Tyr-D-Trp-Ala-Trp-D-His-$NH_2$
$H_2$-His-D-Trp-Ala-Trp-D-His-$NH_2$
$H_2$-His-D-Trp-Ala-Trp-D-Tyr-$NH_2$,
$H_2$-Tyr-D-Trp-Ala-Trp-D-p-Cl-Phe-$NH_2$,
$H_2$-p-Cl-Phe-D--Trp-Ala-Trp-D-Phe-$NH_2$
H-desaminoTyr-D-Trp-Ala-D-Phe-$NH_2$

$$\overset{H}{|}\quad CH_3CO\text{-Tyr-D-Trp-Ala-Trp-D-Phe-}NH_2,$$

$H_2$-O-Me-Tyr-D-Trp-Ala-Trp-D-Phe-$NH_2$,
$H_2$-Tyr-D-Trp-Ala-Trp-D-Phe-Met-$NH_2$,
$H_2$-Tyr-D-Trp-Ala-D-Phe-Thr-$NH_2$,
$H_2$-Tyr-D-Trp-Ala-Trp-D-Phe-Gln-OH,
$H_2$-Tyr-D-Trp-Ala-Trp-D-Phe-Gln-$NH_2$,
$H_2$-Tyr-D-Trp-Ala-Trp-D-Phe-Asn-$NH_2$,
$H_2$-His-D-Trp-Ala-Trp-D-Phe-Lys-$NH_2$,
$H_2$-Tyr-D-Trp-Ala-Trp-D-Phe-Lys-$NH_2$,
$H_2$-Tyr-D-Trp-Ala-Trp-D-Phe-Glu-$NH_2$,
$H_2$-Tyr-D-Trp-Ala-Trp-D-Phe-Phe-$NH_2$,
$H_2$-Tyr-D-Trp-Ala-Trp-D-Phe-Gln-$NH_2$,
$H_2$-His-D-Trp-Ala-Trp-D-Phe-Lys-OH,
$H_2$-His-D-Trp-Ala-Trp-D-Phe-Arg-$NH_2$,
$H_2$-His-D-Trp-Ala-Trp-D-Phe-Gln-$NH_2$,
$H_2$-His-D-Trp-Ala-Trp-D-Phe-Glu-$NH_2$,
$H_2$-His-D-Trp-Ala-Trp-D-Phe-HomoArg-$NH_2$,
$H_2$-3-N-Me-His-D-Trp-Ala-Trp-D-Phe-Lys-$NH_2$,
$H_2$-His-D-Trp-Ala-Trp-D-Phe-Lys-$NHCH_2CH_3$,
$H_2$-His-D-Trp-Ala-Trp-D-Phe-Orn-$NH_2$,
$H_2$-His-D-Trp-Val-Trp-D-Phe-Lys-$NH_2$, et
$H_2$-His-D-Trp-Ser-Trp-D-Phe-Lys-$NH_2$.

5. Association comprenant:
(a) au moins un agent promoteur de croissance, et
(b) au moins un peptide selon l'une quelconque des revendications 1—3 ou 4.

6. Association selon la revendication 5 dans laquelle ledit peptide a pour formule

$$H_2\text{-His-D-Trp-Ala-Trp-D-Phe-Lys-NH}_2$$

et ledit agent promoteur de croissance est le zeranol $C_{18}H_{26}O_5$.

7. Peptide suivant l'une quelconque des revendication précédentes pour utilisation dans la régulation de la libération d'hormone de croissance de la glande pituitaire.

8. Peptide suivant l'une quelconque des revendication précédentes pour utilisation dans la régulation de la libération *in vivo* d'hormone de croissance de la glande pituitaire.

9. Composé intermédiaire ayant une formule choisie parmi l'ensemble constitué par

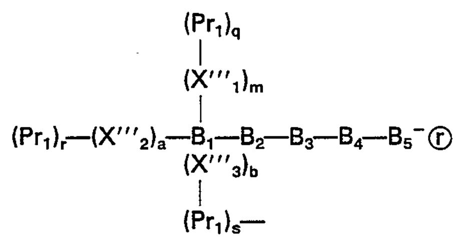

(VI)

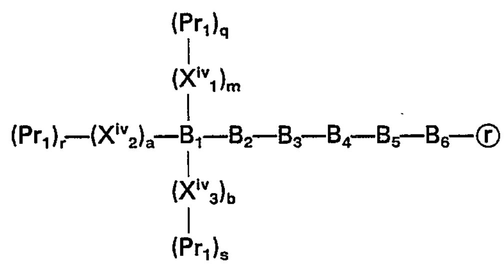

(VII)

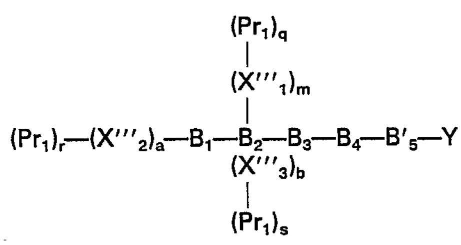

(VIII)

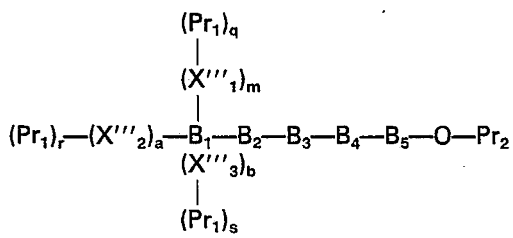

(IX)

$$B_1—B_2—B_3—B_4—B'_5—Y \qquad (X)$$

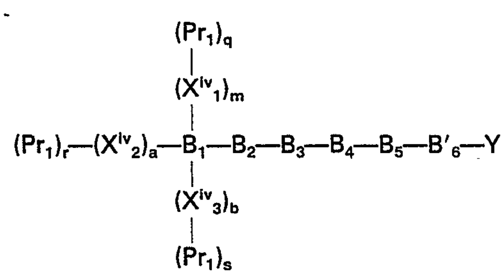

(XI)

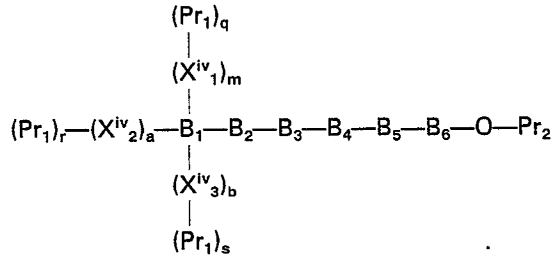

(XII)

$$B_1—B_2—B_3—B_4—B_5—B'_6—Y \quad (XIII)$$

où

$Pr_1$ est un groupe protecteur d'α-amino-acide;

a, b, m, q, r, et s valent chacun 0 ou 1;

$X'''_1$, $X'''_2$, $X'''_3$, $X^{iv}_2$ et $X^{iv}_3$ sont choisis parmi l'ensemble constitué par les substitutions et radicaux d'atome d'azote terminal et d'atome de carbone en α de groupes désamino;

$B_1$ et $B_4$ sont choisis parmi l'ensemble constitué par les groupes His, Arg, Lys, α-Napht, β-Napht, Iql, Tyr, Trp, Phe, leurs homologues et analogues, les formes protégées à chaîne latérale correspondantes, et, en ce qui concerne $B_1$, les formes désamino correspondantes;

$B_2$, $B_5$ et $B'_5$ sont choisis parmi l'ensemble constitué par les groupes D-His, D-Arg, D-Lys, D-α-Napht, D-β-Napht, D-Iql, D-Tyr, D-Trp, D-Phe, leurs homologues et analogues, les formes correspondantes à chaîne latérale protégée, et, en ce qui concerne $B'_5$, les formes décarboxy correspondantes;

$B_3$ est choisi parmi l'ensemble constitué par les groupes Gly, Ala, Val, Leu, Ile, Pro, Ser, Thr, Met, Asp, Glu, Asn, Gln, His, D-Ala, D-Val, D-Leu, D-Ile, D-Pro, D-Ser, D-Thr, D-Met, D-Asp, D-Glu, D-Asn, D-Gln, D-Arg, D-His, leurs homologues et analogues, et les formes protégées à chaîne latérale correspondantes;

$B_6$ et $B'_6$ sont choisis parmi l'ensemble constitué par les amino-acides de configurations L- et D-, leurs homologues et analogues, les formes protégées à chaîne latérale correspondantes, et, en ce qui concerne $B'_6$ les formes décarboxy correspondantes;

Ⓡ est une résine;

Y est choisi parmi l'ensemble constitué par les substitutions de carbone terminal et de carbone en α de groupe décarboxy; et

$Pr_2$ est un groupe protecteur de carboxyle;

avec la condition qui, si (1) a vaut 1 et b et m valent 0 et $X'''_2$ est choisi parmi l'ensemble constitué par —H et —$CH_3$; (2) $B_1$ et $B_4$ sont choisis parmi l'ensemble constitué par Tyr, Trp, Phe et les formes protégées à chaîne latérale correspondantes; (3) $B_3$ est choisi parmi l'ensemble constitué par les groupes Gly, Ala, Val, Leu, Ile, Pro, Ser, Thr, Met, Asp, Glu, Asn, Gln, His et les formes protégées à chaîne latérale correspondantes; et, en ce qui concerne les formules XI et XIII, (4) Y est choisi parmi l'ensemble constitué par —$NR_1R_2$, —OR et —$CH_2OR$ où R, $R_1$ et $R_2$ sont choisis parmi l'atome d-hydrogène et les groupes alkyle en $C_1$—$C_6$; alors au moins un des $B_2$, $B_5$ ou $B'_5$ est choisi de façon à être différent de l'ensemble constitué par les groupes D-Tyr, D-Trp, D-Phe, et, en ce qui concerne $B'_5$, les formes décarboxy correspondantes, et les formes protégées à chaîne latérale correspondantes;

si (1) a vaut 1 et b et m valent 0, et $X'''_2$ est choisi parmi l'ensemble constitué par —H et —$CH_3$; (2) $B_2$ et $B_5$ ou $B'_5$ sont choisis parmi l'ensemble constitué par les groupes D-Tyr, D-Trp, D-Phe, et en ce qui concerne $B'_5$, les formes décarboxy correspondantes, et les formes protégées à chaîne latérale correspondantes; (3) $B_3$ est choisi parmi l'ensemble constitué par les groupes Gly, Ala, Val, Leu, Ile, Pro, Ser, Thr, Met, Asp, Glu, Asn, Gln, His et les formes protégées à chaîne latérale correspondantes; et, en ce qui concerne les formules XI et XIII, (4) Y est choisi parmi l'ensemble constitué par les groupes —$NR_1R_2$, —OR et —$CH_2OR$, où chaque, R, $R_1$ et $R_2$ est choisi parmi l'atome d'hydrogène et les groupes alkyle en $C_1$—$C_6$ à chaîne linéaire ou ramifiée; alors au moins un des $B_1$ et $B_4$ est choisi de façon à être différent des groupes Tyr, Trp, Phe et des formes protégées à chaîne latérale correspondantes;

si (1) a vaut 1 et b et m valent 0 et $X^{iv}_2$ est choisi parmi l'ensemble constitué par les groupes —H, —$CH_3$ et —$CHOCH_3$; (2) $B_1$ et $B_4$ sont choisis parmi l'ensemble constitué par les groupes Tyr, Trp, Phe et les formes protégées à chaîne latérale correspondantes; (3) $B_3$ est choisi parmi l'ensemble constitué par les groupes Gly, Ala, Val, Leu, Ile, Ser, Thr, Met, Asn, Gln, et les formes protégées à chaîne latérale correspondantes; (4) $B_6$ ou $B'_6$ est choisi parmi l'ensemble constitué par les groupes Asn, Gln, Glu, Arg, Lys, Ser, Thr, et en ce qui concerne $B'_6$, les formes décarboxy correspondantes, et les formes protégées à chaîne latérale correspondantes; et, en ce qui concerne les formules XI et XIII, (5) Y est choisi parmi l'ensemble constitué par les groupes —$NR_1R_2$, —OR et —$CH_2OR$ où chaque, R, $R_1$ et $R_2$ est choisi parmi l'atome d'hydrogène et les groupes alkyle en $C_1$—$C_6$ à chaîne linéaire ou ramifiée; alors au moins un des $B_2$ et $B_5$ est choisi de façon à être différent des groupes D-Tyr, D-Trp, D-Phe et des formes protégées à chaîne latérale correspondantes; et

si (1) a vaut 1 et b et m valent 0 et $X^{iv}_2$ est choisi parmi l'ensemble constitué par les groupes —H, —$CH_3$ et —$CHOCH_3$; (2) $B_2$ et $B_5$ sont choisis parmi l'ensemble constitué par les groupes D-Tyr, D-Trp, D-Phe et les formes protégées à chaîne latérale correspondantes; (3) $B_3$ est choisi parmi l'ensemble constitué par les

groupes Gly, Ala, Val, Leu, Ile, Ser, Thr, Met, Asn, Gln et les formes protégées à chaîne latérale correspondantes; (4) $B_6$ ou $B'_6$ est choisi parmi l'ensemble constitué par les groupes Asn, Gln, Glu, Arg, Lys, Ser, Thr, et en ce qui concerne $B'_6$, les formes décarboxy correspondantes, et les formes protégées à chaîne latérale correspondantes; et, en ce qui concerne les formules XI et XIII, (5) Y est choisi par l'ensemble constitué par les groupes $-NR_1R_2$, $-OR$ et $-CH_2OR$ où chaque R, $R_1$ et $R_2$ est choisi parmi l'atome d'hydrogène et les groupes alkyle en $C_1-C_6$ à chaîne linéaire ou ramifiée; alors un au moins des $B_1$ et $B_4$ est choisi de façon à être différent de Tyr, Trp, Phe et des formes protégées à chaîne latérale correspondantes.

10. Composé intermédiaire suivant la revendication 9, dans lequel:

$B_1$ et $B_4$ sont choisis parmi l'ensemble constitué par les groupes His, Tyr, Trp, Phe, leurs homologues et analogues, et en ce qui concerne $B_1$, les formes désamino correspondantes, et les formes protégées à chaîne latérale correspondantes;

$B_2$, $B_5$ et $B'_5$ sont choisis parmi l'ensemble constitué par les groupes D-His, D-Tyr, D-Trp, D-Phe, leurs homologues et analogues, et en ce qui concerne $B'_5$, les formes décarboxy correspondantes, et les formes protégées, à chaîne latérale correspondantes;

$B_3$ est choisi parmi l'ensemble constitué par les groupes Gly, Ala, Ser, Asn, Pro, D-Ala, D-Ser, D-Asn, D-Pro, leurs homologues et analogues, et les formes protégées à chaîne latérale correspondantes; et

$B_6$ et $B'_6$ sont choisis parmi l'ensemble constitué par les groupes Arg, Lys, Orn, His, Asp, Glu, Asn, Gln, D-Arg, D-Lys, D-Orn, D-His, D-Asp, D-Glu, D-Asn, D-Gln, leurs homologues et analogues, et en ce qui concerne $B'_6$ les formes décarboxy correspondantes, et les formes protégées à chaîne latérale correspondantes.

11. Composé intermédiaire suivant la revendication 9 ayant une formule choisi parmi l'ensemble constitué par

$(Pr_1)_q$
|
$(X'''_1)_m$
|
$(Pr_1)_r-(X'''_2)_a-B_1-D-Trp-B_3-B_4-B_5-$Ⓡ (XIV)
|
$(X'''_3)_b$
|
$(Pr_1)_s$

$(Pr_1)_q$
|
$(X^{iv}_1)_m$
|
$(Pr_1)_r-(X^{iv}_2)_a-B_1-D\text{-}Trp\text{-}B_3-B_4-B_5-B_6-$Ⓡ (XV)
|
$(X^{iv}_3)_b$
|
$(Pr_1)_s$

$(Pr_1)_q$
|
$(X'''_1)_m$
|
$(Pr_1)_r-(X'''_2)_a-B_1-D\text{-}Trp\text{-}B_3-B_4-B'_5-Y$ (XVI)
|
$(X'''_3)_b$
|
$(Pr_1)_s$

$(Pr_1)_q$
|
$(X'''_1)_m$
|
$(Pr_1)_r)-(X'''_2)_a-B_1-D\text{-}Trp\text{-}B_3-B_4-B_5-O-Pr_2$ (XVII)
|
$(X'''_3)_b$
|
$(Pr_1)_s$

$$B_1—D\text{-}Trp\text{-}B_3—B_4—B'_5—Y \qquad (XVIII)$$

$(Pr_1)_q$
|
$(X^{iv}_1)_m$
|
$(Pr_1)_r—(X^{iv}_2)_a—B_1—D\text{-}Trp\text{-}B_3—B_4—B_5—B'_6—Y$ (XIX)
|
$(X^{iv}_3)_b$
|
$(Pr_1)_s$

$(Pr_1)_q$
|
$(X^{iv}_1)_b$
|
$(Pr_1)_r—(X^{iv}_2)_a—B_1—D\text{-}Trp\text{-}B_3—B_4—B_5—B_6—O—Y$ (XX) et
|
$(X^{iv}_3)_b$
|
$(Pr_1)_s$

$$B_1—D\text{-}Trp\text{-}B_3—B_4—B_5—B'_6—Y \qquad (XXI)$$

$B_1$ est choisi parmi l'ensemble constitué par les groupes Tyr, O-Me-Tyr, His, 3-N-Me-His, p-Cl-Phe, les formes désamino correspondantes, et les formes protégées à chaîne latérale correspondantes;

$B_3$ est choisi parmi l'ensemble constitué par les groupes Ala, Ser, D-Ala et les formes protégées à chaîne latérale correspondantes;

$B_4$ est choisi pari l'ensemble constitué par les groupes Trp, Tyr et les formes protégées à chaîne latérale correspondantes;

$B_5$ et $B'_5$ sont choisis parmi l'ensemble constitué par les groupes D-Phe, D-His, D-Tyr, D-p-Cl-Phe, et en ce qui concerne $B'_5$, les formes décarboxy correspondantes, et les formes protégées à chaîne latérale correspondantes; et

$B_6$ et $B'_6$ sont choisis parmi l'ensemble constitué par les groupes Arg, HomoArg, Lys, Orn, Asp, Glu, Asn, Gln, D-Lys, leurs formes décarboxy, et les formes protégées à chaîne latérale correspondantes.

12. Composé suivant la revendication 9 de formule choisie parmi l'ensemble constitué par
Boc-His(Tos)-D-Trp-Ala-Trp-D-Phe-ⓡ,
Boc-Tyr(BrZ)-D-Trp-Ala-Trp-D-His(Tos)-ⓡ,
Boc-His(Tos)-D-Trp-Ala-Trp-D-His(Tos)-ⓡ,
Boc-His(Tos)-D-Trp-Ala-Trp-D-Tyr(BrZ)-ⓡ,
Boc-Tyr(BrZ)-D-Trp-Ala-Trp-D-p-Cl-Phe-ⓡ,
Boc-Tyr(BrZ)-D-Trp-Ala-Trp-D-Phe-ⓡ,
Boc-p-Cl-Phe-D-Trp-Ala-Trp-D-Phe-ⓡ,
3(p-OH-phenyl)propanoic acid-D-Trp-Ala-Trp-D-p-Cl-Phe-ⓡ,
Boc-O-Me-Tyr(BrZ)-D-Trp-Ala-Trp-D-Phe-ⓡ,
Boc-Tyr(BrZ)-D-Trp-Ala-Trp-D-Phe-Met-ⓡ,
Boc-Tyr(BrZ)-D-Trp-Ala-Trp-D-Phe-Thr(Bzl)-ⓡ,
Boc-Tyr(BrZ)-D-Trp-Ala-Trp-D-Phe-Glu-α-benzyl ester-ⓡ,
Boc-Tyr(BrZ)-D-Trp-Ala-Trp-D-Phe-Gln-ⓡ,
Boc-Tyr(BrZ)-D-Trp-Ala-Trp-D-Phe-Asn-ⓡ,
Boc-His(Tos)-D-Trp-Ala-Trp-D-Phe-Lys(ClZ)-ⓡ,
Boc-Tyr(BrZ)-D-Trp-Ala-Trp-D-His(Tos)-Lys(C1Z)-ⓡ,
Boc-Tyr(BrZ)-D-Trp-Ala-Trp-D-Phe-Glu-γ-Bzl-ⓡ,
Boc-Tyr(BrZ)-D-Trp-Ala-Trp-D-Phe-Phe-ⓡ,
Boc-Tyr(BrZ)-D-Trp-Gly-Trp-D-Phe-Gln-ONP-ⓡ,
Boc-His(Tos)-D-Trp-Ala-Trp-D-Phe-Lys(ClZ)-ⓡ',
Boc-His(Tos)-D-Trp-Ala-Trp-D-Phe-Arg(Tos)-ⓡ,
Boc-His(Tos)-D-Trp-Ala-Trp-D-Phe-Gln-ONP-ⓡ,
Boc-His(Tos)-D-Trp-Ala-Trp-D-Phe-Glu-γ-Bzl-ⓡ,
Boc-His(Tos)-D-Trp-Ala-Trp-D-Phe-HomoArg(Tos)-ⓡ,
Boc-3-N-Me-His-D-Trp-Ala-Trp-D-Phe-Lys(ClZ)-ⓡ,
Boc-His(Tos)-D-Trp-Ala-Trp-D-Phe-Lys(ClZ)-ⓡ,
Boc-His(Tos)-D-Trp-Ala-Trp-D-Phe-Orn(Z)-ⓡ,
Boc-His(Tos)-D-Trp-Val-Trp-D-Phe-Lys(ClZ)-ⓡ, et
Boc-His(Tos)-D-Trp-Ser(Bzl)-Trp-D-Phe-Lys(ClZ)-ⓡ,

où

Boc est t-butyloxycarbonyle;
BrZ est o-bromobenzyloxycarbonyle;
BrZ est p-bromobenzyloxycarbonyle;
Bzl est benzyle;
γ-Bzl est un ester γ-benzylique;
ClZ est o-chloro-benzyloxycarbonyle;
ONP est un ester nitrophénylique;
Tos est p-toluènesulfonyle;
Z est benzyloxycarbonyle;
ⓡ est une résine p-méthylbenzhydrylamine; et
ⓡ' est une résine hydroxyméthylée.